# EUROPEAN PATENT SPECIFICATION

(11) **EP 1 911 843 B1**
(45) Date of publication and mention of the grant of the patent: **14.03.2012**
(21) Application number: 06756783.4
(22) Date of filing: 24.05.2006
(51) Int. Cl.: C12N 15/09, C12N 1/21, C12P 17/08, C12R 1/465

(54) **GENETICALLY MODIFIED MICROORGANISM AND PROCESS FOR PRODUCTION OF MACROLIDE COMPOUND USING THE MICROORGANISM**
GENTECHNISCH VERÄNDERTER MIKROORGANISMUS UND VERFAHREN ZUR HERSTELLUNG EINER MAKROLIDVERBINDUNG UNTER VERWENDUNG DES MIKROORGANISMUS
MICROORGANISME GÉNÉTIQUEMENT MODIFIÉ ET PROCÉDÉ POUR LA PRODUCTION D'UN COMPOSÉ MACROLIDE UTILISANT LE MICROORGANISME

(30) Priority: 26.05.2005 JP 2005154114
(43) Date of publication of application: 16.04.2008
(73) Proprietor: Eisai R&D Management Co., Ltd., Tokyo 112-8088 (JP); MicroBiopharm Japan Co., Ltd., Chuo-ku Tokyo 103-0023 (JP)
(72) Inventor: MACHIDA, Kazuhiro, 4380078 (JP); ARITOKU, Yasuhide, 4380078 (JP)
(74) Representative: HOFFMANN EITLE
(86) International application number: PCT/JP2006/310835
(87) International publication number: WO 2006/126723

(56) References cited:
- WO-A1-02/060890
- WO-A1-03/040370
- WO-A1-2004/011459
- WO-A1-2004/011661
- WO-A1-2004/050890
- WO-A1-2004/065401
- WO-A1-2005/052152
- WO-A1-2006/009276
- MACHIDA KAZUHIRO ET AL: "Increase in pladienolide D production rate using a Streptomyces strain overexpressing a cytochrome P450 gene." JOURNAL OF BIOSCIENCE AND BIOENGINEERING JUN 2008, vol. 105, no. 6, June 2008 (2008-06), pages 649-654, XP022939147 ISSN: 1347-4421
- MCALPINE J.B. ET AL.: 'Microbial genomics as a guide to drug discovery and structural elucidation: ECO-02301, a novel antifungal agent, as an example' J. NAT. PROD. vol. 68, no. 4, April 2005, pages 493 - 496, XP003002461
- SCHMID E. ET AL.: 'AUD4, a new amplifiable element from Streptomyces lividans' MICROBIOLOGY vol. 145, no. PART 12, 1999, pages 3331 - 3341, XP002979874

## Description

### Field of the invention

The present invention relates to recombinant microorganisms having the ability to produce 16-position hydroxyl macrolide compounds, and to methods for producing 16-position hydroxyl macrolide compounds using those microorganisms.

### Related art

Important bioactive substances have been discovered among the various metabolites produced by the actinomycetes. In particular, many compounds have been found having a polyketide in the basic structure (hereunder called polyketide compounds). Compounds having various biological functions are known including for example the antibacterial agents erythromycin, josamycin, tylosin, midecamycin and mycinamicin, the antifungal agents nystatin and amphotericin, the insecticidal agents milbemycin and avermectin, the immune suppressors tacrolimus and rapamycin and the anti-tumor agents daunomycin, adriamycin, aclacinomycin and the like.

The polyketide compounds include a group of macrolide compounds with excellent anti-tumor activity called pladienolides. "Pladienolide" is a general term for a group of compounds discovered in culture of the Streptomyces sp. Mer-11107 strain, and more than 50 relatives are known, beginning with 11107B (also called pladienolide B), which is represented by the formula (A) below (See WO 2002/060890).

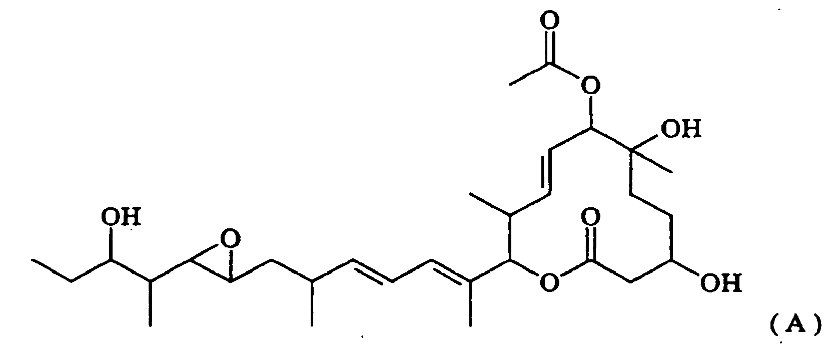

Of these pladienolides, the 16-position hydroxyl substance represented by the following formula (I) has excellent properties including strong anti-tumor activity and the like, but it has been difficult to produce it efficiently because its productivity is inferior to that of 11107B. 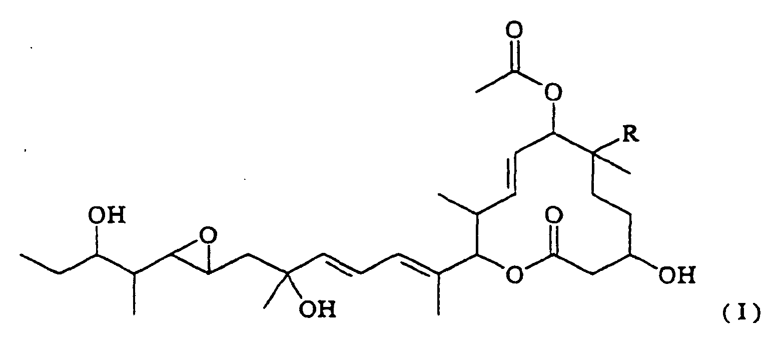 (wherein R is a hydrogen atom or hydroxyl group).

The compound represented by the formula (I) may be called ME-282 when R is a hydrogen atom and 11107D or pladienolide D when R is a hydroxyl group.

A research group including the applicants in the present application has subsequently discovered actinomycetes which hydroxylates the 16-position of 11107B but which are not pladienolide-producing organisms (see WO 2003/099813 and WO 2004/050890), have obtained and identified DNA participating in a 16-position hydroxylation reaction of 11107B (DNA encoding for 16-position hydroxylating enzyme and those ferredoxins) from these actinomycetes, and have submitted a patent application therefor (see WO-A 2005/052152 published on June 9, 2005).

We have also obtained and identified from the pladienolide-producing strain Streptomyces sp. Mer-11107 polypeptides participating in biosynthesis of 111078 and DNA encoding those polypeptides, and have submitted a patent application therefor (see WO-A 2006/009276 published. on January 26, 2006).

It has thus become possible to produce 16-position hydroxyl substances more efficiently than before by producing 11107B or the like using the Streptomyces sp. Mer 11107 strain or variants thereof and then converting the resulting 11107B into 16-position hydroxyl substances using other actinomycetes capable of the 16-position hydroxylation reaction. However, because of the two-stage reaction, the operational difficulty is doubled, and it has been therefore desired to establish a more efficient production method.

Cases are known in which a gene coding for an enzyme which modifies a polyketide compound has been introduced so as to be expressed in a strain producing the polyketide compound, resulting in the direct production of a polyketide compound modified by an introduced enzyme. For example, it has been reported that direct production of 6-hydroxy-tetracenomycin C can be achieved by introducing the hydroxylation gene of Streptomyces fradiae Tu2717 into Streptomyces glaucescens GLA.0 that produces tetracenomycin C (see J. Bacteriol. 1995, Vol. 177, 6126-6136).

Cases are also known in which a polyketide compound biosynthesis gene has been introduced so as to be expressed in a strain having an enzyme which modifies the polyketide compound, resulting in direct production of a polyketide compound modified by an enzyme originally present in the host strain. For example, it has been reported that direct production of 6-hydroxy-tetracenomycin C can be achieved by introducing the tetracenomycin C biosynthesis gene of Streptomyces glaucescens GLA.0 into Streptomyces fradiae Tu2717, which possesses an enzyme that hydroxylates the 6 position of tetracenomycin C (see J. Bacteriol. 1995, Vol. 177, 6126-6136).

In some cases, production of a modified polyketide compound can be achieved with a strain of a different species by introducing the polyketide compound biosynthesis gene and a gene encoding an enzyme which modifies the polyketide compound together into a strain of a different species. For example, it has been reported that direct production of 8,8a-dihydroxy-6-erythronolide B was achieved by introducing both a 6-erythronolide B biosynthesis gene of *Saccharopolyspora erythraea* and a P-450 gene of *Streptomyces antibioticus* ATCC11891 into *Streptomyces lividans* K4-114 as the host (see J. Antibiot. 2000, Vol. 53, 502-508).

Macrolide compounds and related polyketides or enzymes involved in the synthesis of macrolide compounds or related polyketides have also been mentioned in WO 2003 040370A1, WO 2004 011459 A1, WO 2002 060890 A1, WO2004 011661 A1 and WO 2004 065401 A1. Microbial genomics and their use in drug discovery have been described by McAlpine et al., J. Nat. Prod., 2005, 68, 493-496. Schmid et al., Microbiology, 1999, 145, 3331-3341 describe AUD4 as an amplifiable element from *Streptomyces lividans* and its potential use.

### Disclosure of the invention

It is an object of the present invention to provide recombinant microorganisms having the ability to produce the 16-position hydroxyl macrolide compound represented by the above formula (I). Another object is to provide a method of producing a 16-position hydroxyl macrolide compound using these recombinant microorganisms.

In an effort to resolve the aforementioned problems, the inventors used genetic engineering methods to prepare (breed) microorganisms having both the previously isolated and identified DNA participating in biosynthesis of 11107B and DNA participating in a 16-position hydroxylation reaction of 11107B, and they have found that a 16-position hydroxyl macrolide compound accumulated in large quantities in the resulting culture broth. Thus, they accomplished the present invention.

That is, the present invention relates to the following (1) to (13).
(1) A recombinant microorganism, which is a microorganism having ability to produce 16-position hydroxyl macrolide compounds represented by the formula (I): 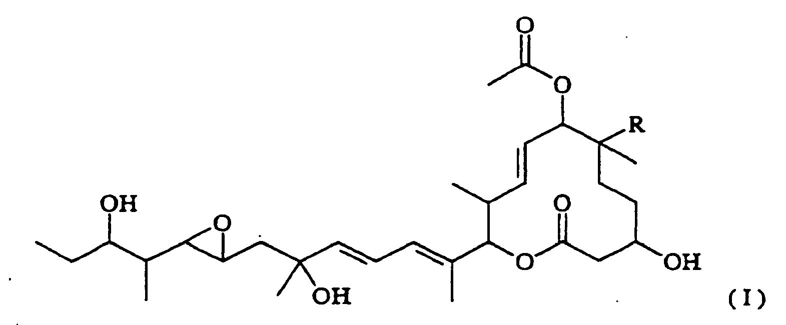 (wherein R represents a hydrogen atom or hydroxyl group), and, which comprises:
   (a) DNA encoding wholly or partially a polypeptide participating in biosynthesis of the macrolide compounds represented by the formula (II): 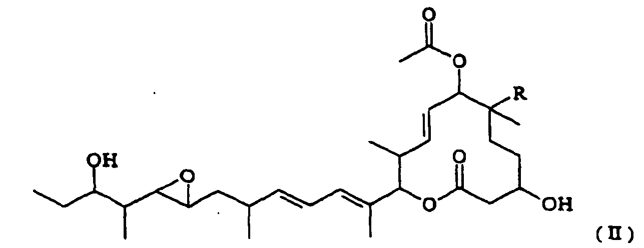 (wherein R represents a hydrogen atom or hydroxyl group), wherein the DNA described in the above (a) is DNA encoding wholly or partially a polypeptide having polyketide synthetase activity, a polypeptide having 7-position acetylase activity, a polypeptide having 18,19-position epoxidase activity and a polypeptide having transcriptional regulator factor activity,
      comprising:
      (a-1) the continuous nucleotide sequence of nucleotides 8340 to 27935 in the Sequence No. 1;
      (a-2) the continuous nucleotide sequence of nucleotides 28021 to 49098 in the Sequence No. 1;
      (a-3) the continuous nucleotide sequence of nucleotides 49134 to 60269 in the Sequence No. 1;
      (a-4) the continuous nucleotide sequence of nucleotides 60269 to 65692 in the Sequence No. 1;
      (a-5) the continuous nucleotide sequence of nucleotides 68160 to 66970 in the Sequence No. 1;
      (a-6) the continuous nucleotide sequence of nucleotides 69568 to 68270 in the Sequence No. 1; and
      (a-7) the continuous nucleotide sequence of nucleotides 72725 to 70020 in the Sequence No. 1; and
   (b) DNA encoding wholly or partially a polypeptide having 16-position hydroxylating enzyme activity with respect to the macrolide compounds represented by the above formula (II), wherein the DNA is selected from the group consisting of:
      (b-1) the continuous nucleotide sequence of nucleotides 1322 to 2548 in the Sequence No. 2;
      (b-2) the continuous nucleotide sequence of nucleotides 420 to 1604 in the Sequence No. 3; and
      (b-3) the continuous nucleotide sequence of nucleotides 172 to 1383 in the Sequence No. 4, or a variant thereof.
(2) The recombinant microorganism described in (1), wherein the DNA described under (a) above further includes DNA encoding wholly or partially a polypeptide having 6-position hydroxylating enzyme activity.
(3) The recombinant microorganism described in (2), wherein the DNA encoding wholly or partially a polypeptide having 6-position hydroxylating enzyme activity is DNA comprising the continuous nucleotide sequence of nucleotides 65707 to 66903 in the Sequence No. 1.
(4) The recombinant microorganism described in any one of (1) to (3), wherein the DNA represented by (b) above is a DNA comprising the continuous nucleotide sequence of nucleotides 1322 to 2548 in the Sequence No. 2 which has 1 or 2 or more modified sites selected from the group consisting of 1) to 6) below:
   1) a modified site wherein the sequence cgc of nucleotides 1592 to 1594 is modified to a codon encoding an amino acid other than arginine;
   2) a modified site wherein the sequence atg of nucleotides 1655 to 1657 is modified to a codon encoding an amino acid other than methionine;
   3) a modified site wherein the sequence cgc of nucleotides 1904 to 1906 is modified to a codon encoding an amino acid other than arginine;
   4) a modified site wherein the sequence cgc of nucleotides 2027 to 2029 is modified to a codon encoding an amino acid other than arginine;
   5) a modified site wherein the sequence atc of nucleotides 2054 to 2056 is modified to a codon encoding an amino acid other than isoleucine; and
   6) a modified site wherein the sequence ctg of nucleotides 2528 to 2530 is modified to a codon encoding an amino acid other than leucine.
(5) The recombinant microorganism described in any one of (1) to (3), wherein the DNA represented by (b) above is a DNA comprising the continuous nucleotide sequence of nucleotides 1322 to 2548 in the sequence No. 2 which has 1 or 2 or more modified sites selected from the group consisting of 1) to 6) below:
   1) a modified site wherein the sequence cgc of nucleotides 1592 to 1594 is modified to a codon encoding cysteine, methionine or proline;
   2) a modified site wherein the sequence atg of nucleotides 1655 to 1657 is modified to a codon encoding threonine or serine;
   3) a modified site wherein the sequence cgc of nucleotides 1904 to 1906 is modified to a codon encoding leucine, isoleucine, proline, tyrosine or phenylalanine;
   4) a modified site wherein the sequence cgc of nucleotides 2027 to 2029 is modified to a codon encoding leucine, isoleucine or proline;
   5) a modified site wherein the sequence atc of nucleotides 2054 to 2056 is modified to a codon encoding phenylalanine or valine; and
   6) a modified site wherein the sequence ctg of nucleotides 2528 to 2530 is modified to a codon encoding methionine, cysteine or isoleucine.
(6) The recombinant microorganism described in any one of (1) to (3), wherein the DNA represented by (b) above is a DNA comprising the continuous nucleotide sequence of nucleotides 1322 to 2548 in the sequence No. 2 which has 1 or 2 or more modified sites selected from the group consisting of 1) to 6) below:
   1) a modified site wherein the sequence cgc of nucleotides 1592 to 1594 is modified to a codon encoding cysteine;
   2) a modified site wherein the sequence atg of nucleotides 1655 to 1657 is modified to a codon encoding threonine;
   3) a modified site wherein the sequence cgc of nucleotides 1904 to 1906 is modified to a codon encoding leucine or tyrosine;
   4) a modified site wherein the sequence cgc of nucleotides 2027 to 2029 is modified to a codon encoding leucine;
   5) a modified site wherein the sequence atc of nucleotides 2054 to 2056 is modified to a codon encoding phenylalanine; and
   6) a modified site wherein the sequence ctg of nucleotides 2528 to 2530 is modified to a codon encoding methionine.
(7) The recombinant microorganism described in (1), comprising a host having the DNA described under (a) above into which has been incorporated the DNA described under (b) above derived from a microorganism of a different species.
(8) The recombinant microorganism described in (1), comprising a host having the DNA described under (b) above into which has been incorporated the DNA described under (a) above derived from a different species.
(9) The recombinant microorganism described in (1), comprising a host having none of the DNA described under (a) and (b) above into which has been incorporated the DNA described under both (a) and (b) above derived from different species.
(10) The recombinant microorganism described in any of (1) to (9), wherein the recombinant microorganism having the ability to produce the 16-position hydroxyl macrolide compound represented by the formula (I) is a strain belonging to Streptomyces genus.
(11) A method for producing 16-position hydroxyl macrolide compounds represented by the formula (I) or pharmacologically acceptable salts thereof, or hydrates of them, wherein the recombinant microorganism described in any of (1) to (10) is cultured in nutritious medium, and the 16-position hydroxyl macrolide compounds represented by the formula (I) are collected from the culture broth thereof.
(12) The method described in (11), wherein a cyclodextrin is present in the culture broth.
(13) The method described in (12), wherein the Cyclodextrin is a cyclodextrin selected from the group consisting of β-cyclodextrin, γ-cyclodextrin, partially methylated β-cyclodextrin, dimethyl-β-cyclodextrin, trimethyl-β-cyclodextrin, glycosyl-β-cyclodextrin and hydroxypropyl-β-cyclodextrin.

The terms in the present specifications are defined as follows.

A "recombinant microorganism" is a microorganism (bacteria, actinomycete, yeast, mould or the like) comprising a gene derived from another organism introduced into a specific microorganism by genetic recombination, and the genetic introduction techniques used therefor are not limited to genetic recombination using plasmids and other vectors but include techniques of homologous recombination and the like.

"Variant of DNA" means:
(1) DNA that hybridizes under stringent conditions with the original DNA;
(2) DNA with a nucleotide sequence having 70% or greater homology with the nucleotide sequence of the original DNA;
(3) DNA having a nucleotide sequence complementary to the nucleotide sequence of the original DNA; or
(4) DNA which does not hybridize under stringent conditions with the original DNA due to degeneracy of a gene codon, but which has a nucleotide sequence coding for the same amino acid sequence as the amino acid sequence coded for by the DNA defined in any of (1) to (3).

"DNA that hybridizes under stringent conditions" means for example DNA obtained by colony hybridization, plaque hybridization, Southern hybridization or the like using the original DNA as the probe, and specifically may be DNA which can be identified using a filter having DNA derived from a colony or plaque fixed thereon by first hybridizing at 65°C in the presence of 0.7 to 1.0 M sodium chloride, and then washing the filter at 65°C using an 0.1 to 2x concentration SSC solution (an SSC solution with a 1x concentration comprising 150 mM sodium chloride and 15 mM sodium citrate).

A "relative" is a compound that has the same framework characterizing the chemical structure but that differs in terms of the form of modification or the form of the side chain.

"16-position hydroxylating enzyme activity" means the activity of converting the 16-position hydrogen atom of macrolide compound (II) into a hydroxyl group.

"6-position hydroxylating enzyme activity" means the activity of converting the 6-position hydrogen atom of the macrolide compound (sometimes called ME-265) represented by the following formula (B) into a hydroxyl group.

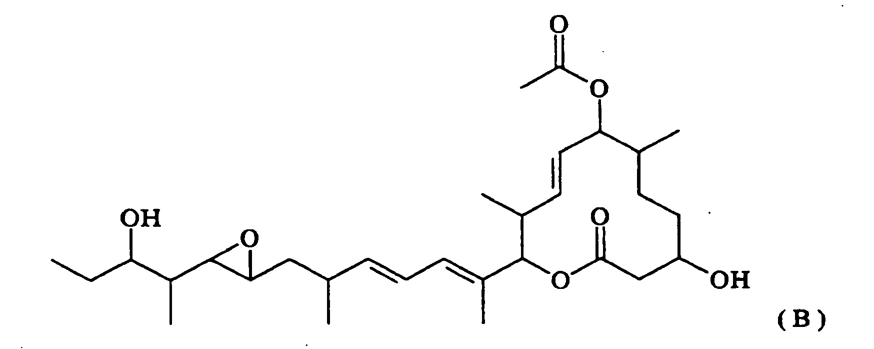

"Polyketide synthetase activity" means the activity of synthesizing the macrolide compound represented by the following formula (E):

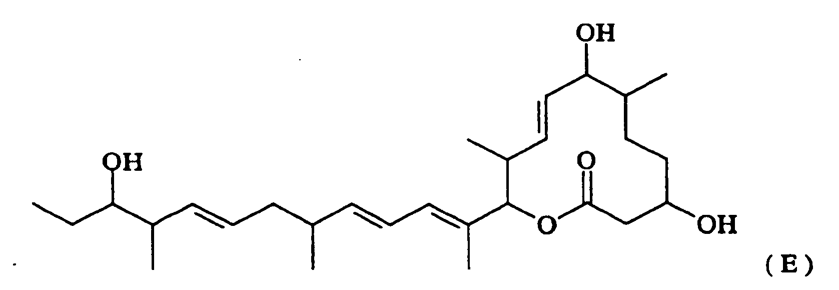

"7-position acetylase activity" means the activity of converting the 7-position hydroxyl group in the macrolide compound represented by the following formula (C) into an acetyl group. 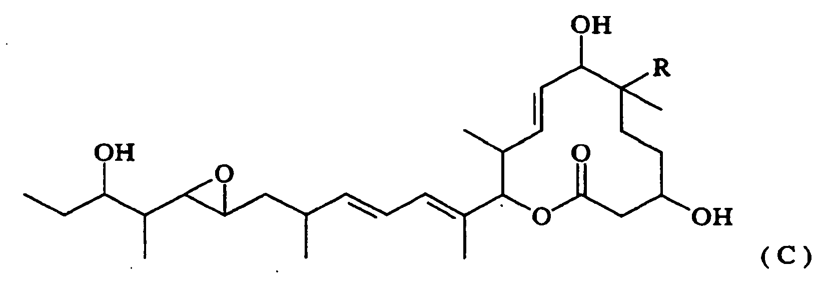 (wherein R represents a hydrogen atom or hydroxyl group).

"18,19-position epoxidase activity" means the activity of converting an epoxy group for the 18,19-position double bond in the macrolide compound represented by the following formula (D): 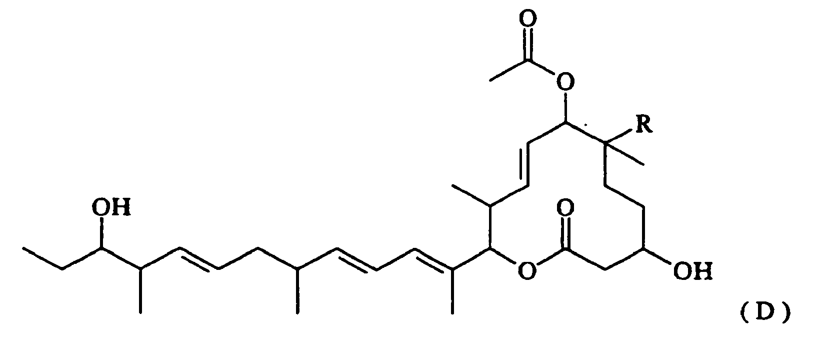 (wherein R represents a hydrogen atom or hydroxyl group).

"Transcription regulator activity" means the activity of regulating the transcription of DNA encoding a polypeptide participating in biosynthesis of macrolide compound (II).

According to the present invention, a recombinant microorganism can be obtained having DNA encoding a polypeptide participating in biosynthesis of macrolide compound (II) and having DNA encoding a polypeptide having 16-position hydroxylating enzyme activity with respect to macrolide compound (II), and capable of direct fermentative production of 16-position hydroxyl macrolide compound (I). 16-position hydroxyl macrolide compound (I) can be produced efficiently by culturing that recombinant microorganism and collecting the compound from the culture broth.

Particularly, 16-position hydroxyl macrolide compound (I) can be produced more efficiently and selectively, by applying DNA encoding a polypeptide which has 16-position hydroxylating enzyme activity, in which polypeptide the amino acid sequence has been partially modified, to the above-mentioned method.

### Brief description of the drawings

Fig. 1 shows the biosynthesis pathway of pladienolides in Mer-11107.
Fig. 2 shows the correspondence between cosmids and each of ORFs of DNA participating in biosynthesis of pladienolides in Mer-11107.
Fig. 3 shows the structure of plasmid pKU253.
Fig. 4 shows the structure of plasmid pUC19aph::oriT::intphiC31.

### Detailed Description of the Invention

Embodiments of the present invention are explained in detail below.

DNA encoding a polypeptide participating in biosynthesis of macrolide compound (II) in the present invention can be obtained by a method known in the field (such as the colony hybridization method described in Molecular Cloning, 2^{nd} Edition) from the cultured mycelia of a microorganism capable of producing that macrolide compound. This microorganism may be any capable of producing the macrolide compound, regardless of species or strain, but strains belonging to the Streptomyces genus of the actinomycetes are preferred. One example is the Streptomyces sp. Mer-11107 strain, which has been isolated from soil. Mer-11107 was deposited as FERM P-18144 at the National Institute of Bioscience and Human-Technology Agency of Industrial Science and Technology (1-3, Higashi 1-chome Tsukuba-shi, Ibaraki-ken 305-8566 Japan) as of December 19, 2000, and then transferred to International Deposit FERM BP-7812 at International Patent Organism Depositary (IPOD) National Institute of Advanced Industrial Science and Technology (Tsukuba Central 6, 1-1, Higashi 1-Chome, Tsukuba-shi, Ibaraki-ken 305-8566 Japan) as of November 27, 2001.

The operations for obtaining the target DNA are outlined below using the Mer-11107 strain as an example. Recombinant DNA obtained by connecting genomic DNA of Mer-11107 that has been partially digested with a suitable restriction enzyme together with a cosmid vector capable of replication in *E*. *coli* that has been digested with a suitable restriction enzyme is incorporated into *E*. *coli* to obtain transduced strains. The multiple transduced strains obtained above are then screened using a fragment of a known hydroxylating enzyme (cytochrome P450 enzyme) gene as the probe, and the transduced strains that hybridize to the probe are selected. DNA that hybridizes to the hydroxylating enzyme gene present in the selected cosmids is then obtained and sequenced. This is then introduced into *E*. *coli,* and once it has been confirmed that the transformed *E*. *coli* has 6-position hydroxylating enzyme activity, DNA coding for this 6-position hydroxylating enzyme can be used as the probe in Southern hybridization to select, cosmids comprising a macrolide compound biosynthesis gene cluster neighboring DNA coding for the 6-position hydroxylating enzyme from the multiple positive clones (cosmids) obtained above, and the target DNA is obtained by organizing these cosimids. Details of all these operations are described in Reference Example 1 and also in WO-A 2006/009276, specifications submitted by the applicants in this case, and these can be used for reference.

A nucleotide sequence obtained in this way (amino acid sequence in the coding sequence) comprising the region surrounding of DNA coding for a polypeptide participating in biosynthesis of macrolide compound (II) is represented by the Sequence No. 1 in the sequence listing.

The DNA represented by the Sequence No. 1 includes 8 open reading frames (ORFs): pldA I (nucleotides 8340 to 27935), pldA II (nucleotides 28021 to 49098), pldA III (nucleotides 49134 to 60269), pldA IV (nucleotides 60269 to 65692), p1dB (nucleotides 65707 to 66903), p1dc (nucleotides 68160 to 66970), pldD (nucleotides 69568 to 68270) and pldR (nucleotides 72725 to 70020).

Of these DNAs, p1dA I, p1dA II, p1dA III and p1dA IV have several transcription reading frames each including one or more repeating units called modules in the same way as other known polyketide biosynthesis genes. As explained below, each module codes for all or some domains selected from the acyl carrier protein (ACP), β-ketoacyl ACP synthetase (KS), acyl transferase (AT) domains, which participate in condensation reactions in polyketide synthesis, and the ketoacyl reductase (KR), dehydrogenase (DH) and enoyl reductase (ER) domains, which participate in β-position carbonyl group modification reactions, and the final module includes the thioesterase (TE) domain, which removes the polyketide chain from the polyketide synthetase.

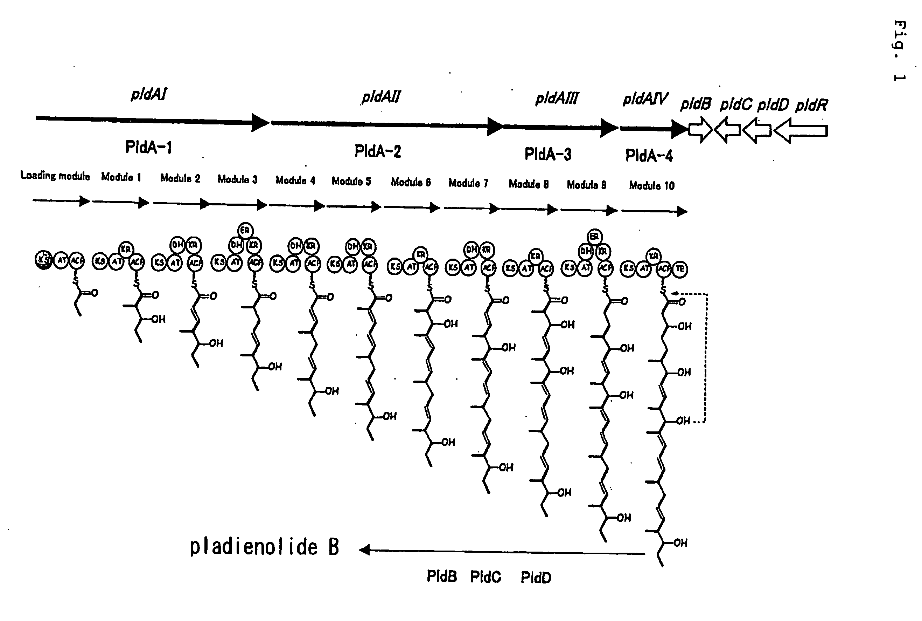

Fig. 1 shows the biosynthesis pathway of a macrolide compound in the Mer-11107 strain. Unlike the other modules the loading module has the central cysteine replaced by glutamine, indicating that pldA I participates in the initial reaction. Module 10 includes a thioesterase (TE) domain, indicating that pldA IV participates in the final reaction of synthesizing the basic polyketide skeleton. Once the basic skeleton of the polyketide has been formed in this way, it is modified by the 18,19-position epoxidase coded for by pldD, the 7-position acetylase encoded by pldc and the 6-position hydroxylase encoded by pldB to biosynthesize macrolide compound (II). Macrolide compound (II) in which R is a hydrogen atom can be produced by either deleting pldB or modifying it so that it does not express its function. PldR, which has high homology with the aveR gene encoding a transcription regulator factor in avermectin biosynthesis, encodes a DNA transcription regulator factor participating in biosynthesis of macrolide compounds.

In the present invention, DNA encoding a polypeptide having 16-position hydroxylating enzyme activity with respect to macrolide compound (II) can be obtained by methods well known in the field (such as the colony hybridization method described in Molecular Cloning, 2^{nd} Edition) from cultured mycelia of a microorganism having 16-position hydroxylation ability with respect to the macrolide compound. Any microorganism having the ability to convert the 16-position hydrogen atom of the macrolide compound to a hydroxyl group can be used regardless of species or strain, but desirable examples include strains belonging to the actinomycetes. Examples of these include the Streptomyces sp. A-1544 strain and the Streptomyces sp. Mer-11107 strain and the Streptomyces sp. A-1560 strain. The A-1544 strain was deposited as FERM P-18943 at International Patent Organism Depositary National Institute of Advanced Industrial Science and Technology (Tsukuba Central 6, 1-1, Higashi 1-Chome, Tsukuba-shi, Ibaraki-ken 305-8566 Japan) as of July 23, 2002, and then transferred to International Deposit FERM BP-8446 as of July 30, 2003, at International Patent Organism Depositary (IPOD) National Institute of Advanced Industrial Science and Technology (Tsukuba Central 6, 1-1, Higashi 1-Chome, Tsukuba-shi, Ibaraki-ken 305-8566 Japan). The A-1560 strain was deposited as FERM P-19585 at International Patent Organism Depositary National Institute of Advanced Industrial Science and Technology (Tsukuba Central 6, 1-1, Higashi 1-Chome, Tsukuba-shi, Ibaraki-ken 305-8566 Japan) as of November 13, 2003 and then transferred to International Deposit FERM BP-10102 as of August 19, 2004, at International Patent Organism Depositary (IPOD) National Institute of Advanced Industrial Science and Technology (Tsukuba Central 6, 1-1, Higashi 1-Chome, Tsukuba-shi, Ibaraki-ken 305-8566 Japan).

The operations for obtaining the target DNA are outlined below using the A-1544 strain as an example. Genomic DNA of A-1544 is first prepared and subjected to PCR using primers prepared based on the sequence information for a known hydroxylating enzyme gene (cytochrome P450 enzyme) to obtain a specifically amplified DNA fragment which is then sequenced. The nucleotide sequence of the neighboring region upstream and downstream from the cloned DNA fragment is then amplified by an inverse PCR (Cell Technology Vol. 14, pp. 591-593, 1995) based on the resulting sequence and cloned, and the sequence is analyzed. Details of all these operations are described in Reference Example 2 (A-1544 strain), Reference Example 3 (Mer-11107 strain) and Reference Example 4 (A-1560 strain), and also in WO-A 2005/052152 published on June 9, 2005, and these can be used for reference.

The DNA (including neighboring region) thus obtained from A-1544 strain which encodes a polypeptide having 16-position hydroxylating enzyme activity with respect to macrolide compound (II) is represented by the Sequence No. 2 in the sequence listing. The DNA represented by the Sequence No. 2 includes two open reading frames (ORFs), psmA (nucleotides 1322 to 2548) and psmB (nucleotides 2564 to 2761), which encode 16-position hydroxylating enzyme and ferredoxin, respectively.

Similarly, DNA (including neighboring region) encoding a polypeptide having 16-position hydroxylating enzyme activity with respect to macrolide compound (II) obtained from Mer-11107 is represented by the Sequence No. 3 in the sequence listing. This DNA represented by the Sequence No. 3 includes two open reading frames (ORFs), bpmA (nucleotides 420 to 1604) and bpmB (nucleotides 1643 to 1834), which encode 16-position hydroxylatinge enzyme and ferredoxin, respectively.

DNA (including neighboring region) encoding a polypeptide having 16-position hydroxylating enzyme activity with respect to macrolide compound (II) obtained from A-1560 is represented by the Sequence No. 4 in the sequence listing. This DNA represented by the Sequence No. 4 includes two open reading frames (ORFs), tpmA (nucleotides 172 to 1383) and tpmB (nucleotides 1399 to 1593), which encode 16-position hydroxylating enzyme and ferredoxin, respectively.

Further, variants of the above-mentioned various DNA which encodes a polypeptide having 16-position hydroxylating enzyme activity can be obtained by the following procedures. First, the site-directed modification is conducted, based on the original DNA sequence information, and then the resulting site-directed variant of DNA is introduced into the host. Thus, the objective variants can be selected and obtained by using the highly selective and active 16-position hydroxylating enzyme of the variant of DNA of the resulting recombinant microorganism as the index. The site-directed modification, for example, can be conducted by using, as a template, a plasmid cloning the original DNA and using the primer constructed based on the sequence information thereof, using a commercially available kit such as QuickChange^{™} Site-Directed Mutagenesis Kit (Stratagene Co.).

Among these variants, as those having high 16-position hydroxylating enzyme activity and further high selectivity, for example, in the case of psmA encoding 16-position hydroxylating enzyme and being obtained from A-1544 strain, the variant of DNA having the continuous nucleotide sequence of nucleotides 1322 to 2548 in the Sequence No. 2 and 1 or 2 or more modified sites selected from the group consisting of the following 1) to 6) may be included:
1) a modified site wherein the sequence cgc of nucleotides 1592 to 1594 is modified to a codon encoding an amino acid, preferably cysteine, other than arginine;
2) a modified site wherein the sequence atg of nucleotides 1655 to 1657 is modified to a codon encoding an amino acid, preferably threonine, other than methionine;
3) a modified site wherein the sequence cgc of nucleotides 1904 to 1906 is modified to a codon encoding an amino acid, preferably leucine or tyrosine, other than arginine;
4) a modified site wherein the sequence cgc of nucleotides 2027 to 2029 is modified to a codon encoding an amino acid, preferably leucine, other than arginine;
5) a modified site wherein the sequence atc of nucleotides 2054 to 2056 is modified to a codon encoding an amino acid, preferably phenylalanine, other than isoleucine; and
6) a modified site wherein the sequence ctg of nucleotides 2528 to 2530 is modified to a codon encoding an amino acid, preferably methionine, other than leucine.

The recombinant microorganism of the present invention is a recombinant microorganism having the aforementioned "DNA encoding a polypeptide participating in biosynthesis of macrolide compound (II)" and "DNA encoding a polypeptide having 16-position hydroxylating enzyme activity with respect to macrolide compound (II)," and can be prepared by the methods shown under A) to C) below.
A) "DNA encoding a polypeptide having 16-position hydroxylating enzyme activity with respect to macrolide compound (II)" is incorporated into a host that originally has "DNA encoding a polypeptide participating in biosynthesis of macrolide compound (II)".
B) "DNA encoding a polypeptide participating in biosynthesis of macrolide compound (III)" is incorporated into a host that originally has "DNA encoding a polypeptide having 16-position hydroxylase activity with respect to macrolide compound (II)".
C) "DNA encoding a polypeptide participating in biosynthesis of macrolide compound (II)" and "DNA encoding a polypeptide having 16-position hydroxylating enzyme activity with respect to macrolide compound (II)" are both incorporated into a host that originally possesses neither DNA.

The host for the recombinant "DNA encoding a polypeptide participating in biosynthesis of macrolide compound II" and "DNA encoding a polypeptide having 16-position hydroxylating activity with respect to macrolide compound II" may be any microorganism capable of incorporating the target DNA and producing the target 16-position hydroxyl macrolide compound, but an example of a preferred microorganism is a strain belonging to the Streptomyces genus of actinomycetes. For example, Streptomyces sp. Mer-11107 or the like can be used in method A) above, while Streptomyces sp. A-1544, Streptomyces sp. Mer-11107 or Streptomyces sp. A-1560 can be used in method B) above. In method C), a strain of Escherichia coli or the like can be used.

There are no particular limits on the method of incorporating the target DNA and causing it to be expressed in a host, but for example the methods described in Molecular Cloning, 2^{nd} Edition or Current Protocols in Molecular Biology can be used. There are no particular limits on the host and plasmid vector system as long as the target DNA can be stably maintained and expressed in the host. The plasmid may include self-replicating sequences, promoter sequences, terminator sequences, drug-resistance genes and the like in addition to the target DNA, and the plasmid may be either a self-replicating plasmid or an integrative plasmid having a sequence homologous to a specific region in the genome of the anticipated host. The target DNA may be incorporated into a site either on the plasmid or on the genome of the host microorganism.

If the host is an actinomycete or related strain, the self-replicating vector pIJ6021 (Gene, 166, 133-137 (1995)) or the genome integration vector KC515 (The Bacteria Vol. 9, Antibiotic-producing Streptomyces (ed. Queener, S.E. and Day, L.E.), pp. 119-158, Academic Press, Orlando, Fla) can be used.

Examples of self-replicating sequences include rep pIJ101 (1988, J. Bacteriol., 170, 4634-9651), rep SCP2 (1981, J. Gen. Microbiol., 126, 427-442), pUC19 (Gene, 33 (1), 103-119 (1985)) and the like, while examples of promoter sequences include ermEp (Mol. Microbiol., 1994, Nov; 14(3): 533-45), SnpAp (J. Bacteriol. 1992, May; 174(9): 2797-2808) and the like, examples of terminator sequences include ter(mmr) (Gene, 1987; 58(2-3): 229-41), fd terminator (Nucleic Acids Res., 5(12), 4495-4503 (1978) and the like, and examples of drug-resistance genes include hyg (Nucleic Acids Res., 4(14), 1565-1581 (1986)), tsr (Mol. Gen. Genet., 199(1), 26-36 (1985)) and the like. The microorganism of the present invention can be easily prepared by a person skilled in the art following the descriptions of the present specification.

A recombinant microorganism prepared in this way can be cultured, and productivity of 16-position hydroxyl macrolide compound (I) can be evaluated and selected for to obtain a strain with a high level of productivity of the macrolide compound. That is, a recombinant organism having the ability to produce 50 mg or more of the macrolide compound per 1 liter of culture broth can be obtained at a high rate of about 40%, while one having the ability to produce 100 mg or more per 1 liter of culture broth can be obtained at a high rate of about 30%.

### (Method of evaluating productivity)

The recombinant microorganism is inoculated into a 250 mL Erlenmyer flask containing 30 mL of medium, (soluble starch 5%, glucose 1%, Pharmamedia 3%, CaCO₃ 0.1%, pH 7.5), cultured with rotary shaking (220 rpm) for 4 days at 25°C and extracted by addition of 270 mL acetonitrile, and the resulting extract is analyzed by HPLC under the following measurement conditions to assay the concentration of accumulated 16-position hydroxyl macrolide compound (I).

### (HPLC measurement conditions)

Device: Shimadzu HPLC 10Avp
Column: Develosil ODS UG-3 (ϕ 4.6 mm x 50 mm 3 µm)
Mobile phase: 45%-55% methanol (0 to 5 min), 55% methanol (5 to 13 min), 55%-70% methanol (13 to 21 min), 70% methanol (21 to 25 min)
Flow rate: 1.2 mL/min
Detection: UV 240 nm
Injection volume: 5 µL
Column temperature: 40°C

The recombinant microorganism of the present invention can be inoculated into a nutrient medium and culture aerobically, and 16-position hydroxyl macrolide compound (I) or a pharmacologically acceptable salt thereof, or a hydrate of them can be obtained from the resulting culture broth.

In principle the recombinant microorganism can be cultured by ordinary culture methods for microorganisms, but culture under aerobic conditions such as culture with aeration and agitaion or shaking culture in the case of a liquid culture is normally preferred. As the medium used for cultivation, any medium may be used insofar as it contains nutrients which these microorganisms can utilize, and various synthetic medium, semi-synthetic medium and organic medium may be all utilized. As the medium composition, various carbon sources such as glucose, sucrose, fructose, glycerin, dextrin, starch, molasses and soybean oil may be used either singly or in combinations. As the nitrogen source, there can be used a single or a combination of organic nitrogen sources such as Pharmamedia, peptone, meat extract, soybean meal, casein, amino acid, yeast extract or urea, and inorganic nitrogen sources such as sodium nitrate or ammonium sulfate. Additionally, for example, there can be added and used salts such as sodium chloride, potassium chloride, calcium carbonate, magnesium sulfate, sodium phosphate, potassium phosphate or cobalt chloride; heavy metal salts; vitamins such as vitamin B or biotin, if necessary. Further, when foaming is remarkable during cultivation, various antifoaming agents can be appropriately added in the medium. Chemicals necessary for maintaining drug-resistant genes may also be added as necessary. When the antifoaming agent is added, it is required to set at a concentration for not adversely affecting the production of an objective substance.

The concentration of the accumulated 16-position hydroxyl macrolide compound can be increased by addition of cyclodextrins in the culture broth when producing 16-position hydroxyl macrolide compound (I) by cultivation of the recombinant microorganism of the present invention. Examples of cyclodextrins to be used in the present invention include β-cyclodextrin, γ-cyclodextrin, partially methylated β-cyclodextrin, dimethyl-β-cyclodextrin, trimethyl-β-cyclodextrin, glycosyl-β-cyclodextrin and hydroxypropyl-β-cyclodextrin. Each of them may be used singly or in a combination with another.

The amount of cyclodextrin added to the culture broth is preferably 0.5% to 5%. Addition is preferably at the beginning of cultivation or during cultivation.

The culture conditions can be selected appropriately within the range at which the microorganism grows well and can produce 16-position hydroxyl macrolide compound (I). For example, the pH of the medium is kept at 5 to 9 of near normally the neutral range. The culture temperature is normally 20 to 40°C or preferably 23 to 35°C. Cultivation is 2 to 12 days or normally 3 to 10 days. Of course, the various culture conditions described above can be changed and optimal conditions selected appropriately depending on the type of microorganism used, the external conditions and the like. Common methods used for isolation and purification of microbial metabolites from culture broth can be used to isolate and purify the 16-position hydroxyl macrolide compound (I) which accumulates in the culture broth. Possible methods include all known methods such as organic solvent extraction with methanol, ethanol, butanol, ethyl acetate, chloroform, acetone, toluene or the like, various forms of ion-exchange chromatography, gel filtration chromatography using Sephadex LH-20 or the like, adsorption chromatography using activated charcoal, silica gel, adsorbent resin (Diaion HP20) or the like, adsorption-desorption by thin layer chromatography and high-performance liquid chromatography using a reverse-phase column and the like, and are not limited to the methods indicated herein. These methods may be used singly or in combinations of two or more in an optional order or repeatedly, which makes it possible to isolate and purify the target 16-position hydroxyl macrolide compound (I).

### Examples

The present invention is explained in detail below using examples, but the present invention is not limited by these examples. In the explanations below, concentrations of medium components are expressed as weight percentages unless otherwise specified.

### Reference Example 1: Obtaining DNA encoding polypeptide involved in biosynthesis of macrolide compound (II)

### (1) Cultivation of Mer-11107 and isolation of genomic DNA

Hyphae of Streptomyces sp. Mer-11107 were inoculated into 25 mL of Tryptic Soy Broth, and cultured with shaking at 28°C for 3 days. Genomic DNA was prepared from the resulting culture broth according to the methods described under "Isolation genomic DNA" (pp. 162-170) in D.A. Hopwood et al's Practical Streptomyces Genetics (The John Innes Foundation, Norwich, England, 2000).

### (2) Preparation of mer-11107 genomic library

160 µL of sterile purified water, 200 µL of Mer-11107 genome DNA solution (1 mg/mL), 40 µL of 10x concentration M buffer solution (100 mM Tris-HCl (pH 7.5), 100 mM MgCl₂, 10 mM dithiothreitol, 500 mM NaCl) and 1 µL of restriction enzyme Sau3AI (1 unit/µL) were mixed and incubated at 37°C for 3 minutes. 50 µL was then taken out and extracted with 50 µL of phenol-chloroform mixture (phenol:chloroform:isoamyl alcohol=25:24:1, volume ratio), the aqueous layer was collected and extracted again with 50 µL of chloroform , and the aqueous layer was again collected. 5 µL of 3 M sodium acetate (pH 6.0) and 150 µL of ethanol were added to the liquid, which was then left at -80°C for 30 minutes and centrifuged to collect the precipitated DNA. After being washed in 70% ethanol, this DNA was dissolved in 90 µL of sterile purified water, and incubated at 37°C for 3 hours after addition of 10 µL of 10 times concentration BAP buffer solution (500 mM Tris-HCl (pH 9.0), 10 mM MgCl₂) and 5 µL of bacterial alkaline phosphatase (0.5 unit/µL, Takara Bio Inc.). The reaction liquid was extracted with 100 µL of phenol-chloroform mixture (phenol:chloroform:isoamyl alcohol=25:24:1, volume ratio), the aqueous layer was collected and extracted again with 100 µL of chloroform, and the aqueous layer was again collected. 10 µL of 3 M sodium acetate (pH 6.0) and 300 µL of ethanol were added to this liquid, which was then left at -80°C for 30 minutes and centrifuged to collect the precipitated DNA. After being washed in 70% ethanol, this DNA was dissolved in 20 µL of TE buffer solution (10 mM Tris-HCl (pH 8.0), 1 mM EDTA).

Meanwhile, 10 µg of SuperCos cosmid vector (Stratagene Co.) was digested with restriction enzyme XbaI in accordance with the Stratagene manual, the DNA terminals were de-phosphorylated with calf intestinal alkaline phosphatase (Takara Bio Inc.), and after being digested with restriction enzyme BamHI and purified, this was dissolved in 10 µL of TE bluffer solution. 2.5 µL of the Sau3A1 partial digest solution of Mer-11107 DNA described above was added to 1µL of this cosmid DNA solution, and 1.5 µL of sterile purified water, 5 µL of DNA Ligation Kit (Takara Bio Inc.) Solution II and 10 µL of Solution I were added in that order and incubated at 23°C for 10 minutes. 4 µL of the reaction liquid was packaged into a lambda-phage using Gigapack III XL Kit (Stratagene Co.). When the resulting packaged liquid (total 500 µL) was subjected to a transduction test, colony formation ability was tested at 380 cfu (colony forming units)/µL.

### (3) Preparation of various probes

### (3-1) Preparation of probes comprising keto synthetase coding regions

The following two primers, KS-3F and KS-4R, were synthesized based on sequences that are normally conserved in the keto synthesis domains of polyketide synthetases (see the Sequence Nos. 5 and 6).
KS-3F: 5'-GACCGCGGCTGGGACGTGGAGGG-3'
KS-4R: 5'-GTGCCCGATGTTGGACTTCAACGA-3'

These primers were used to carry out PCR under the following conditions.

### (PCR reaction liquid composition)

| | |
|---|---|
| Sterile purified water | 31 µL |
| 2x concentration GC buffer | 50 µL |
| dNTP mixed solution (2.5 mM each dATP, dGTP, dTTP and dCTP) | 16 µL |
| KS-3F (100 pmol/µL) | 0.5 µL |
| KS-4R (100 pmol/µL) | 0.5 µL |
| Mer-11107 total DNA (100 ng/µL) | 1 µL |
| LA Taq polymerase (5u/µL, Takara Bio Inc.) | 1 µL |

### (Reaction temperature conditions)

95°C 3 minutes
(98°C 20 sec, 63°C 30 sec, 68°C 2 min) 30 cycles
72°C 5 minutes

The 930 bp DNA fragments amplified as a result of this reaction were electrophoresed on 0.8% agarose gel, and the isolated 930 bp DNA fragments were excised and collected and purified using SUPREC-01 (Takara Bio Inc.). Using 10 ng of the resulting DNA fragments as the template, 930 bp DNA fragments comprising the keto synthetase coding region were amplified again under the same PCR conditions as above except that the number of reaction cycles was changed to 20. These DNA fragments were concentrated and purified using SUPREC-02 (Takara Bio Inc.), and 50µL of the resulting TE solution was taken as the probe solution.

### (3-2) Preparation of probe comprising cytochrome P450 gene region

Two known cytochrome P450 genes were amplified from actinomycetes for purposes of preparing a cytochrome P450 gene probe. That is, the two primers CB-1F and CB-2R comprising the sequences showed below (see the Sequence Nos.7 and 8) were synthesized for purposes of amplifying the ORF-A gene derived from *Streptomyces thermotolerans* ATCC11416 (Biosci. Biotechnol. Biochem. 59: 582-588, 1995).
CB-1F: 5'-ATGACAGCTTTGAATCTGATGGATCCC-3'
CB-2R: 5'-TCAGAGACGGACCGGCAGACTCTTCAGACG-3'

Meanwhile, the two primers PKC-1F and PKC-2R comprising the sequences shown below (see the Sequence Nos. 9 and 10) were synthesized for purposes of amplifying the pik-C gene derived from *Streptomyces venezuelae* ATCC15439 (Chem. Biol. 5: 661-667, 1998).
PKC-1F: 5'-GTGCGCCGTACCCAGCAGGGAACGACC-3'
PKC-2R: 5'-TCACGCGCTCTCCGCCCGCCCCCTGCC-3'

These primers were used to carry out PCR under the following conditions.

### (PCR reaction liquid composition)

| | |
|---|---|
| Sterile purified water | 31 µL |
| 2x concentration GC buffer | 50 µL |
| dNTP mixed solution (2.5 mM each dATP, dGTP, dTTP and dCTP) | 16 µL |
| primer-F (100 pmol/µL) | 0.5 µL |
| primer-R (100 pmol/µL) | 0.5 µL |
| ATCC11416 or ATCC15439 genome DNA (100 ng/µL) | 1 µL |
| LA Taq polymerase (5u/µL), Takara Bio Inc.) | 1 µL |

### (Reaction temperature conditions)

95°C, 3 minutes
(98°C 20 sec, 63°C 30 sec, 68°C 2 min) 30 cycles
72°C, 5 minutes

The two 1.2 kb DNA fragments amplified as a result of this reaction were purified by QIAGEN PCR Purification Kit (QIAGEN Co.), and a mixed solution comprising 10 ng/µL of each DNA fragment was prepared and used as the probe.

### (4) Screening using probe comprising keto synthetase coding region

An *E*. *coli* XL-1Blue MR host (Stratagene Co.) was transduced with the Mer-11107 genome DNA library packaged solution prepared in the above (2) in accordance with the Stratagene manual. After transduction the bacterial suspension was dispensed and spread onto ten LB-50 µq/mL ampicillin-1.5% agar medium plates (inner diameter 90 mm, height 15 mm), and cultured for 18 hours at 37°C. The colonies growing on each plate were transferred to HybondoN+ filters (Amersham Biosciences), alkali and neutral treated under the conditions described in the manual for the HybondoN+ filters, and dried for 2 hours at 80°C to fix DNA derived from the colonies onto the filters.

The genome DNA library was screened by colony hybridization using an AlkPhos Direct System (Amersham Biosciences) with 100 ng of the 930 bp DNA fragment comprising the keto synthetase region prepared under (3-1) above as the probe. Hybridization was performed for 2 hours at 65°C at a salt concentration of 0.5 M NaCl. The conditions for hybridization and detection were those described in the manual attached to the AlkPhos Direct System. Of the roughly 7,600 colonies tested, 59 colonies which hybridized strongly with the alkali phosphatase-labeled probe were isolated. Cosmids were extracted and purified from *E*. *coli* clones derived from these colonies.

### (5) Selection and verification of cosmid clones having pladienolide biosynthesis gene region using probe comprising cytochrome P450 gene region

Two µL of each of the cosmid DNA solutions obtained under the above (4) was spotted onto a HybondoN+ filter, alkali and neutral treated under the conditions described in the attached manual, and dried for 2 hours at 80°C to fix the DNA on the filter. Hybridization was performed with these filters under the same conditions as in the above (4) using the cytochrome P450 gene fragment described under the above (3) as the probe. One cosmid that hybridized strongly with the probe was selected as a result and named pKS58.

The pKS58 DNA was partially digested with Sau3AI restriction enzyme, ligated with the BamHI-CIAP treated phage vector Zap Express (Stratagene Co.), and packaged into a lambda phage using a Gigapack III XL Kit (Stratagene Co.). *E. coli* XL1-Blue MRF' was infected with this phage solution, and made to form a plaque. Plaque hybridization was performed using the cytochrome P450 gene probe prepared in the above (3-2) to subclone an approximately 2 kb lengh

DNA fragment containing cytochrome P450 gene.

This cytochrome P450 gene DNA fragment was sequenced, and two primers PDL58-1F and PDL58-2R (see the Sequence Nos. 11 and 12) having the sequences shown below were synthesized from the N- and C-terminals, which are considered to be the cytochrome P450 coding regions.
PDL58-1F: 5'-GCCCCGCATATGGATCTGGAAACCCAACTTCTC-3'
PDL58-2R: 5'-GCACTAGTCAGCCGCGCTCGACGAGGAGGTG-3'

These primers were used to carry out PCR under the following conditions.

### (PCR reaction liquid composition)

| | |
|---|---|
| Sterile purified water | 31 µL |
| 2 x concentration GC buffer | 50 µL |
| dNTP mixed solution (2.5 mM each dATP, dGTP, dTTP and dCTP) | 16 µL |
| PDL58-1F (100 pmol/µL) | 0.5 µL |
| PDL58-2R (100 pmol/µL) | 0.5 µL |
| pKS58DNA (10 ng/µL) | 1 µL |
| LA Taq polymerase (5u/µL, Takara Bio Inc.) | 1 µL |

### (Reaction temperature conditions)

95°C, 3 minutes
(98°C 20 sec, 63°C 30 sec, 68°C 2 min) 20 cycles
72°C, 5 minutes

The 1.2 kb DNA fragment amplified as a result of this reaction was purified with QIAGEN PCR Purification Kit (QIAGEN Co.), and digested with NdeI and SpeI restriction enzymes. After the reaction the DNA was electrophoresed on 0.8% agarose gel, the isolated 1.2 kb DNA fragment was excised and DNA was collected and purified using QIAGEN GelExtraction Kit (QIAGEN Co.). This DNA fragment was inserted into the NdeI and SpeI sites of the cytochrome P450 gene expression plasmid pT7NS-camAB (see Reference Example 5 and WO 03/087381) to construct pPDL96.

*E. coli* BL21 (DE3) was transformed using this plasmid and cultured in M9CG medium (1.28% Na₂HPO₄·7H₂O, 0.3% KH₂PO₄, 0.05% NaCl, 0.1% NH₄Cl, 1% casamino acid, 0.4% glucose, 1 mM MgCl₂, 100 µM CaCl₂, 50 µg/mL ampicillin) to a density of 0.8 OD₆₀₀ (optical density at 600 nm). 5-Aminolevulinic acid was added to 80 µg/mL and IPTG to 0.1 mM, and cultivation was continued at 22°C for 25 hours to induce the cytochrome P450 protein. After induction, the mycelia were collected and suspended in 5 mL of CV buffer solution (50 mM NaPO₄ (pH 7.3), 1 mM EDTA, 10% glycerol, 1 mM glucose). 1 mL of this suspension was taken in a test tube and 5 µL of a DMSO solution (50 mg/mL) of ME-265 (the 6-position deoxide of pladienolide B) was added and incubated at 28°C for 15 hours. 1 mL of acetonitrile was added and mixed with this reaction solution, which was then centrifuged and supernatant analyzed by HPLC under the following conditions to confirm conversion to pladienolide B. These results lead the pladienolide biosynthesis gene region is involved in pKS58.

### (HPLC analysis conditions)

Device: Shimadzu HPLC 10Avp
Column: Develosil ODS UG-3 (ϕ 4.6 mm x 50 mm 3 µm)
Mobile phase: 45% to 55% methanol (0 to 5 min)
   55% methanol (5 to 13 min)
   55% to 70% methanol (13 to 21 min)
   45% methanol (21 to 25 min)
Flow rate: 1.2 mL/min
Detection: UV 240 nm
Injection volume: 5 µL
Column temperature: 40°C
Analysis time: 25 minutes
Retention time: ME-265: 20 min, Pladienolide B: 13 min

### (6) Selection of cosmid comprising biosynthesis gene cluster neighboring cytochrome P450 gene

A cosmid comprising the biosynthesis gene cluster neighboring the cytochrome P450 gene obtained in the above (5) was selected from the 59 cosmid DNA samples obtained in the above (4).

The 59 cosmid DNA samples were digested with restriction enzymes EcoRI and BamHI, and the DNA obtained in each case was electrophoresed on agarose gel and subjected to Southern hybridization using as probes the KS domain DNA (aveA2 KS6 domain) and the AT domain DNA (aveA1 AT2 domain) of the avermectin aglycone biosynthesis gene (see Proc. Natl. Acad. Sci. USA 96 (1999) 9509-9514; JP-A 2000-245457; or WO 00/50605) and the cytochrome P450 gene obtained in the above (5).

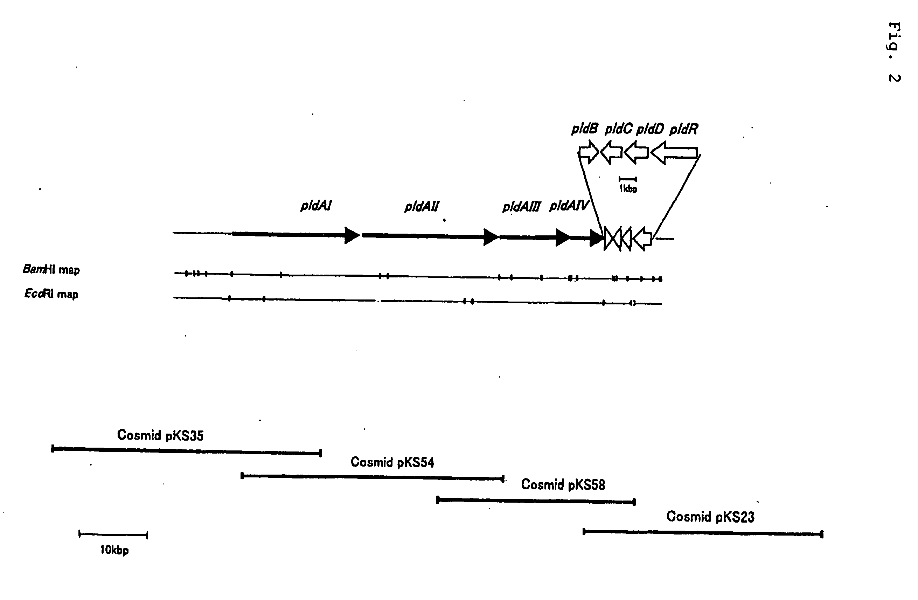

Those cosmids having DNA fragments that hybridized at the same length were grouped on the basis of the electrophoresis patterns of DNA digested with restriction enzymes EcoRI and BamHI, and the hybridization band patterns using the various probes. Of these, all but one of the cosmids exhibiting similar patterns were deleted, and the remaining cosmids were organized according to partially matching band patterns. Beginning with the pKS58 cosmid comprising the cytochrome P450 gene obtained in (5), pKS54 was selected as a cosmid neighboring the side comprising the polyketide synthetase gene from the cytochrome P450 gene side, and pKS35 was selected as a cosmid neighboring pKS54. pKS23 was also selected as a cosmid neighboring the cosmid pKS58 from cytochrome P450 gene side to the side not comprising the polyketide synthetase gene. As a result, as shown in Figure 2, pKS23, pKS58, pKS54 and pKS35 were selected as cosmid clones encompassing the pladienolide biosynthesis gene cluster.

### (7) Determining nucleotide sequence of pladienolide biosynthesis gene cluster

The nucleotide sequence of a DNA group coding for the pladienolide biosynthesis gene was determined. Each cosmid, selected in the above (6), was first isolated by the cesium chloride method, and then sheared to about 1 kb using HydroShear (Genomic Solutions K.K.) and subcloned using a BKL Kit (Takara Bio Inc.).

The resulting subclones were subjected to a cycle sequence reaction (Amersham Biosciences Co.) using fluorescent-labeled primers, and the nucleotide sequences of the respective fragments were determined (MegaBACE 1000: Amersham Biosciences Co.). Thus, a roughly 75 kb nucleotide sequence comprising DNA associated with pladienolide biosynthesis was determined (see the Sequence No. 1).

### (8) Preparation of pladienolide 6-position hydroxylase gene (pldB)-deficient strain.

It has been shown that a pladienolide is biosynthesized by the biosynthesis pathway shown in Fig. 1 from the roughly 75 kb nucleotide sequence comprising DNA participating in pladienolide biosynthesis which was sequenced in the above (7) (see the Sequence No. 1). A pldB-deficient strain was thus prepared by the following methods with the idea that a strain producing only ME-265 (the 6-position deoxide of pladienolide B) could be obtained by destroying only the cytochrome P450 gene pldB.

The four primers pldB-L-Bg12F, pldB-L-Hind3R, pldB-R-Hind3F and pldB-R-Bg12R (see the sequence nos. 13, 14, 15 and 16) having the sequences shown below were synthesized based on the nucleotide sequence of the sequence no. 1. pldB-L-Bg12F: 5'-GGGAGATCTAGAGGCCGGTTACCTCTACGAGTA-3' pldB-L-Hind3R: 5'-GGGAAGCTTGCGATGAGCTGTGCCAGATAG-3' pldB-R-Hind3F: 5'-GGGAAGCTTGAACTGGCGCGACAGTGTCTT-3' pldB-R-Bg12R: 5'-GGGAGATCTGCAGCGGATCGTCTTCGAGACCCTT-3'

PCR was performed under the following conditions using these primers.

### (PCR reaction liquid composition)

| | |
|---|---|
| Sterile purified water | 30 µL |
| 2 x concentration GC buffer | 50 µL |
| dNTP mixed solution (2.5 mM each dATP, dGTP, dTTP and dCTP) | 16 µL |
| pldB-L-Bgl2F or pldB-R-Hind3F (50 pmol/µL) | 1 µL |
| pldB-L-Hind3R or pldB-R-Bgl2R (50 pmol/µL) | 1 µL |
| Mer-11107 total DNA (100 ng/µL) | 1 µL |
| LA Taq polymerase (5 u/µL, Takara Bio Inc.) | 1 µL |

### (Reaction temperature conditions

95°C, 3 minutes
(98°C 20 sec, 63°C 30 sec, 68°C 2 min) 30 cycles
72°C, 5 minutes

As a result, a 1.57 kb DNA fragment (DNA fragment L1) comprising nucleotides 64756 to 66302 in the Sequence No. 1 was amplified by the reaction using pldB-L-Bgl2F and pldB-L-Hind3R, while a 1.54 kb DNA fragment (DNA fragment R1) comprising nucleotides 66849 to 68368 in the Sequence No. 1 was amplified from the reaction using pldB-R-Hind3F and pldB-R-Bgl2R. DNA fragments L1 and R1 were purified with a QIAGEN PCR purification Kit (QIAGEN Co.), and digested with restriction enzymes BglII and HindIII.

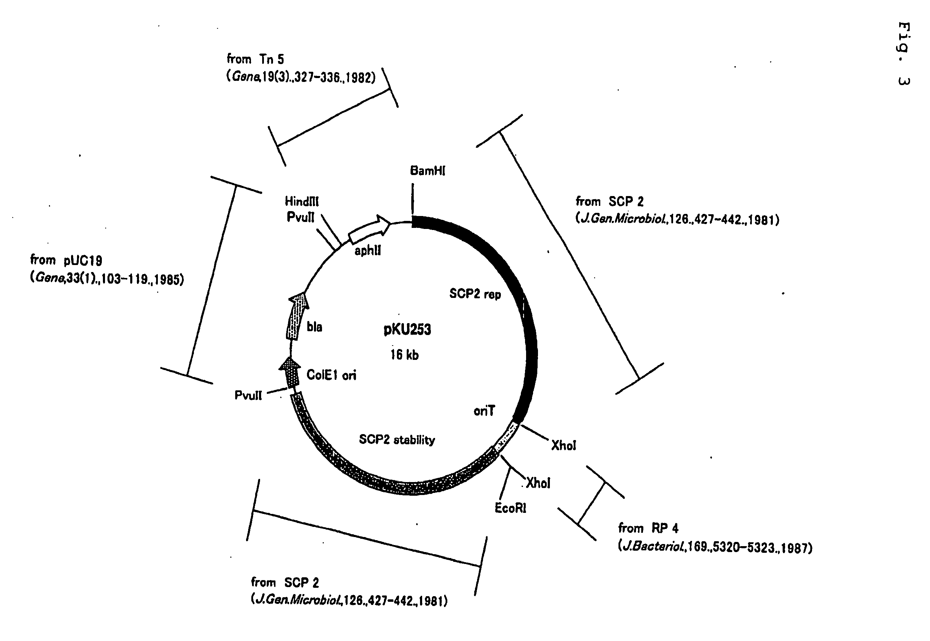

The DNA fragments L1 and R1 which had been digested with restriction enzymes BglII and HindIII, a 2.3 kb hygromycin B resistance gene (derived from pHP45omegahyg: Gene 190, 315-317, 1997, sometimes abbreviated hereunder as "hyg") which had been digested with restriction enzyme HindIII and the shuttle vector pKU253 (see Fig. 3) which had been digested with restriction enzyme BamHI were all four connected to DNA ligation kit Ver. 2.1 (Takara Bio Inc.). A roughly 5.4 kb DNA fragment having the hygromycin B resistance gene inserted between DNA fragments L1 and R1 was thus inserted into pKU253 to construct a roughly 21.4 kb plasmid called pKU253-L1-hyg-R1.

The resulting pKU253-L1-hyg-R1 was transformed into conjugated *E*. *coli* S17-1 by electroporation to obtain S17-1/pKU253-L1-hyg-R1. The resulting S17-1/pKU253-L1-hyg-R1 was inoculated into 10 mL of LB medium (1% bacto tryptone, 0.5% yeast extract, 0.5% NaCl) comprising 25 µg/mL of kanamycin and 100 µg/mL of hygromycin B and shaking cultured at 30°C for 2 hours, and the mycelia were collected, washed twice with 10 mL of LB medium and suspended in 5 mL of LB medium. This was the donor suspension.

While the donor suspension was being prepared, Mer-11107 was inoculated into 10 mL of TSB medium (Trypto-Soya broth: Nissui Seiyaku) and shaking cultured at 30°C for 5 hours, and the mycelia were collected, washed twice with 10 mL of sterile water and suspended in 1 mL of sterile water. This was the recipient suspension. 500 µL of the S17-1/pKU253-L1-hyg-R1 donor suspension was mixed with 10 µL of the Mer-11107 recipient suspension, and applied to Actino Medium No. 4 agar medium. After incubated at 30°C for 18 hours, this was covered with 2.5 mL of SNA (0.8% nutrient medium: Difco, 0.4% agar) comprising 2 mg/mL ribostamycin, and incubated at 30°C for 7 days to obtain a ribostamycin-resistant pKU253-L1-hyg-R1 transformant strain.

The resulting pKU253-L1-hyg-R1 transformant strain was inoculated intp 10 mL of TSB medium containing no ribostamycin, and shaking cultured at 30°C for 24 hours. Mycelia were collected from the pKU253-L1-hyg-R1 transformant culture broth, washed twice with 10 mL of sterilized water and suspended in 10 mL of sterilized water. After being diluted appropriately, the suspension was applied to YMS agar medium (0.4% yeast extract, 1% wheat germ extract, 0.4% soluble starch, 2% agar, 10 mM calcium chloride) comprising 200 µg/mL hygromycin B, and incubated at 30°C for 4 days. Single colonies growing on the YMS agar medium comprising hygromycin B were transplanted to YMS agar medium comprising 200 µg/mL of hygromycin B and YMS agar medium comprising 200 µg/mL of ribostamycin, and incubated at 30°C for 2 days. After incubated, a hygromycin B -resistant, ribostamycin-sensitive strain was selected. The resulting strain, called Mer-11107 pldB::hyg, was a-pldb-deficient strain lacking 546 bp (nucleotides 66303 to 66848 in the Sequence No. 1) of the pldB gene from the genome, with the hygromycin B resistance gene inserted in its place.

### (9) Pladienolide production test of pladienolide 6-position hydroxylase gene (pldB)-deficient strain

200 µl-of frozen seed of the Mer-11107 pldB::hyg strain obtained in the above (8) was inoculated into 20 mL of seed medium (soluble starch 2%, soy bean powder (ESUSAN-MEAT manufactured by Ajinomoto Co. Ltd.) 2%, yeast extract 0.5%, K₂HPO₄ 0-1%, MgSO₄·7H₂O 0.25%, CaCO₃ 0.3%, pH not adjusted) and incubated at 25°C for 2 days. 300 µL of the resulting seed culture broth was inoculated into 30 mL of seed culture medium (5% soluble starch, 1% glucose, 3% Pharmamedia, 2% β-cyclodextrin, 0.1% CaCO₃, pH 7.5) and cultured at 25°C for 4 and 5 days. After the completion of the cultivation, 20 mL of the resulting culture liquid was extracted by adding an equal amount of acetonitrile thereto. Part of this extract was taken and diluted with 5 times the amount of acetonitrile, and levels of pladienolide B and ME-265 were measured by HPLC under the following conditions. The measurement results are shown in Table 1.

### (HPLC analysis conditions)

Device: Shimadzu HPLC 10Avp
Column: Develosil ODS UG-3 (ϕ 4.6 mm x 50 mm 3 µm)
Mobile phase: 45% through 55% methanol (0 to 5 min)
   55% methanol (5 to 13 min)
   55% to 70% methanol (13 to 17 min)
   70% methanol (17 to 35 min)
   45% methanol (35 to 40 min)
Flow rate: 1.2 mL/minute
Detection: UV 240 nm
Injection volume: 10 µL
Column temperature: 40°C
Analysis time: 35 minutes
Retention time: ME-265: 22 min, Pladienolide B: 16 min

**Table 1**

| Mer-11107 pldB::hyg strain | ME-265 (mg/L) | Pladienolide B (mg/L) |
|---|---|---|
| cultured for 4 days (96 hours) | 1247.7 | 0.0 |
| cultured for 5 days (120 hours) | 1316.6 | 0.0 |

### Reference Example 2: DNA-1 encoding polypeptide having the 16-position hydroxylating enzyme activity on the macrolide compound (II)

### (1) Preparation of genomic DNA of Streptomyces sp. A-1544 strain

The A-1544 strain was inoculated into a medium containing 1% of glucose, 0.4% of malt extract and 1% of yeast extract and incubated at 28°C for 3 days. The obtained culture broth was centrifuged at 3000 rpm for 10 minutes to collect the mycelia. A genomic DNA was prepared using Blood & Cell Culture Kit (QIAGEN Co.) from the mycelia.

### (2) Cloning of a partial sequence of a DNA encoding a protein having the activity in hydroxylating the 16-position of the macrolide compound 11107B

The following mix primers (5Dm-3F and 5Dm-3R) were designed and produced on reference to the amino acid sequence assumed to be that of the cytochrome P450 (CYP105D5) of Streptmyces coelicolor A3 (2) (see the Sequence Nos. 17 and 18).
5Dm-3F: 5'-TTCGCSCTSCCSGTCCCSTCSATGGTSAT-3'
5Dm-3R: 5'-GTTGATSAYSGASGTSGAGAA-3'

In order to promote reactivity taking the fluctuation of a codon into account, mixed bases S(=C+G) and Y(=C+T) were used.

Next, these two types of primers (5Dm-3F and 5Dm-3R) and the A-1544 strain genomic DNA as a template obtained in the above (1), were used to run a PCR reaction. The PCR reaction was accomplished by repeating a three-stage reaction including denaturing carried out at 98°C for 20 seconds, annealing carried out at 50°C for 2 minutes and elongation carried out at 68°C for 30 seconds 35 times by using Takara LA Taq (Takara Bio Inc.) and a PCR amplifier (T Gradient, Biometra Co.). As a result, a DNA fragment (hereinafter referred to as a DNA fragment-A1) having a size of about 500 bp was amplified. It is highly possible that this DNA fragment-A1 is a part of the DNA encoding a protein having hydroxylating activity. The DNA fragment-A1 amplified by a PCR reaction was recovered, from the reaction solution by SUPREC PCR (Takara Bio Inc.).

In order to obtain the DNA fragment-A1 in an amount enough to analyze the nucleotide sequence of the obtained DNA fragment-A1, the DNA fragment was combined with a plasmid vector pT7Blue T (Novagen Co.) by using DNA Ligation kit ver.2 (Takara Bio Inc.) to transform E.coli JM109 strain. Thereafter, the transformed E. coli was selected using a L-broth agar media (1.0% bacto tryptone, 0.5% yeast extract, 0.5% NaCl, 1.5% agar) containing ampicillin (50 µg/mL), X-gal (5-bromo-9-chloro-3-indolyl-β-D-galactoside; 40 µg/mL) and IPTG (isopropyl-β-D-thiogalactopyranoside; 100 µM). The colony of the transformed E. coli thus isolated was cultured in a L-broth liquid medium (1% bacto tryptone, 0.5% yeast extract, 0.5% NaCl) containing ampicillin (50 µg/mL). A plasmid DNA was separated from the mycelia of the proliferated transformed E. coli and purified by using a plasmid purifying kit (QIA filter Plasmid Midi Kit, QIAGEN Co.), to obtain enough amount of the DNA fragment-A1.

### (3) Analysis of the nucleotide sequence of the cloned DNA fragment-A1

The nucleotide sequence of the DNA fragment-A1 obtained in the above (2) was analyzed using a DNA nucleotide sequence analyzer (PE Biosystems 377XL) according to a dye terminator cycle sequence method. As the result of the nucleotide sequence analysis, it was clarified that the DNA fragment-A1 amplified by a PCR reaction had an exact size of 528 bp though it had been found to have a size of about 500 bp by the above measurement using electrophoresis (see the nucleotide sequence 1775 to nucleotide sequence 2302 of the Sequence No. 2). Sinces DNA sequences corresponding to the two types of primers used in the above PCR reaction were found at both ends of the above cloned 528 bp DNA sequence, it was clarified that the DNA fragment-A1 was singularly amplified by these two types of primers (5Dm-3F and 5Dm-3R) in the above PCR reaction.

### (4) Analysis of the neighboring region of the DNA fragment-A1

As mentioned above, the partial sequence of the DNA encoding a protein having the hydroxylating activity derived from the A-1544 strain was determined. Therefore, the amplification, cloning and sequence analysis of the nucleotide sequence in the neighboring region extending to the upstream side and downstream side of the cloned fragment were accomplished by an inverse PCR method (Cell Technology vol. 14, p.591-593, 1995). Specifically, the A-1544 strain genomic DNA (see the above (1)) was digested by respective restriction enzymes PstI and SalI in a H buffer solution (50 mM Tris-HCl, pH 7.5, 10 mM MgCl₂, 10 mM dithiothreitol and 100 mM NaCl). The obtained each DNA fragment cleaved by the restriction enzymes was self-circularized by using DNA Ligation Kit ver.2 (Takara Bio Inc.).

Meanwhile, the following primers (6PIN-2F and 6PIN-2R) were designed and produced based on the nucleotide sequence of DNA fragment-Al (see the Sequence Nos. 19 and 20).
6PIN-2F: 5'-GCTGCGCCTGGCCCTGGAGGACATCGAGAT-3'
6PIN-2R: 5'-CTGTTCCTCGAAGAACTCGTGGTCGGCGTA-3'

Next, these two primers (6PIN-2F and 6PIN-2R) and the above self- circularized A-1544 strain genomic DNA as a template, were used to run a PCR reaction. In the PCR reaction, the cycle of a two-stage reaction including denaturing carried out at 98°C for 20 seconds and annealing and elongation carried out at 68°C for 5 minutes was repeated 35 times by using Takara LA Taq (Takara Bio Inc.) and a PCR amplifier (T Gradient, Biometra Co.).

As a result, a DNA fragment (DNA fragment-B1) about 3.5 kbp in size and a DNA fragment (DNA fragment-C1) about 2.8 kbp in size were amplified. It was highly possible that these DNA fragments were a DNA encoding a protein having hydroxylating activity and a DNA having a DNA sequence including the upstream and downstream regions of the former DNA.

The DNA fragment-B1 and the DNA fragment-C1 were recovered from the PCR amplified reaction solution by SUPREC PCR (Takara Bio Inc.). Next, as to the obtained DNA fragment-B1 and DNA fragment-C1, in order to obtain each DNA fragment in an amount enough to analyze the nucleotide sequence of the obtained DNA fragment, a plasmid vector pT7Blue T (Novagen Co.), DNA Ligation kit ver.2 (Takara Bio Inc.), E. coli JM109 strain and a plasmid purifying kit (QIA filter Plasmid Midi kit, QIAGEN Co.) were used in the same manner as the above (2), to obtain enough amount of each DNA fragment.

### (5) Analysis of each nucleotide sequence of the DNA fragment-B1 (about 3.5 kbp in size) and the DNA fragment-C1 (about 2.8 kbp in size)

Each nucleotide sequence of the DNA fragment-B1 and DNA fragment-C1 obtained in the above (4) was analyzed using a DNA nucleotide sequence analyzer (PE Biosystems 377XL) according to a dye terminator cycle sequence method. The nucleotide sequence was thus analyzed to obtain the information of the nucleotide sequence of 3793 bp shown in the Sequence No. 2 from each sequence of the DNA fragment-B1 and DNA fragment-C1.

An open reading frame (ORF) in this 3793 bp was retrieved, to find that the two proteins were coded. Each amino acid sequence of these proteins was retrieved by the BLAST search, and as a result, an ORF (hereinafter referred to as PsmA) coding a protein consisting of 409 amino acids having high homology to cytochrome P450 existed in the base 1322 to base 2548 of the Sequence No. 2. The psmA had the highest homology (homology: 72.6%) to the amino acid sequence assumed to be that of cytochrome P450 (CYP105D5) of the Streptmyces coelicolor A3 (2) and to the amino acid sequence assumed to be that of cytochrome P450 (CYP105D4) of the Streptmyces lividans, and also had a relatively high homology (homology: 69.4%) to cytochrome P450 soy (Soy C)of Streptmyces griseus. It was considered from this fact that the psmA was highly possibly a gene coding hydroxylating enzyme of the cytochrome P-450 type.

Also, an ORF (hereinafter referred to as psmB) encoding a protein having a high homology to ferredoxin of a 2Fe-2S type existed just downstream (the base 2564 to base 2761 of the Sequence No. 2) of the psmA. The protein encoded by the psmB consists of 66 amino acids, and had the highest homology (83.3%) to the amino acid sequence assumed to be that of ferredoxin just downstream of the amino acid sequence assumed to be that of cytochrome P450 (CYP105D5) of the Streptmyces coelicolor A3(2) and a relatively higher homology (homology: 57.6%) to ferredoxin soy (soyB) of Streptmyces griseus. Therefore, it was considered that the psmB serves to transfer electrons and codes ferredoxin participating in hydroxylation together with the psmA.

### (6) Construction of a plasmid pTC-DM

pT7NS-CamAB (see Reference Example 5 and WO 03/087381) was digested by respective restriction enzymes NdeI and SpeI in a H buffer solution (50 mM Tris-HCl, pH 7.5, 10 mM MgCl₂, 10 mM dithiothreitol and 100 mM NaCl) to obtain a plasmid digested products. Meanwhile, a primer DM-NdeF (5'-GCCCCCATATGACGGAACTGACGGACATCA-3': see the Sequence No. 33) obtained by adding a NdeI site to the 5'-terminal and a primer DM-SpeR (5'-GGGCCACTAGTCAGCCGGCCGGTTCGGTCA-3': see the Sequence No. 34) obtained by adding a SpeI site to the 5'-terminal were designed and produced on reference to the nucleotide sequence of the Sequence No. 2. Next, these two types of primers (DM-NdeF and DM-SpeR) and the A-1544 strain genomic DNA obtained in the above (1) as a template, were used to run a PCR reaction. The PCR reaction was accomplished by repeating a two-stage reaction including denaturing carried out at 98°C for 20 seconds and annealing and elongation carried out at 68°C for 2 minutes 30 times by using Takara LA Taq (TAKARA HOLDINGS INC.) and a PCR amplifier (T Gradient, Biometra Co.). From the PCR amplification reaction solution, a DNA fragment having a size of about 1.5 kbp and containing the psmA and the psmB was recovered by SUPREC PCR (TAKARA HOLDINGS INC.). The resulting DNA fragment was digested with restriction enzymes NdeI and SpeI, and the digest and the aforementioned pT7NS-camAB plasmid digest were connected using DNA Ligation Kit Ver. 2 (Takara Bio Inc.). Thus, a roughly 9.5 kbp plasmid (sometimes called plasmid pTC-DM) was constructed comprising the pT7NS-camAB plasmid connected to a DNA fragment containing both the psmA and the psmB.

### Reference Example 3: DNA-2 encoding polypeptide having the 16-position hydroxylating enzyme activity on the macrolide compound (II)

### (1) Preparation of genomic DNA of Streptomyces sp. Mer-11107 strain

The Mer-11107 strain was inoculated into a medium containing 1% of glucose, 0.4% of malt extract and 1% of yeast extract and incubated at 28°C for 3 days. The obtained culture broth was centrifuged at 3000 rpm for 10 minutes to collect the mycelia. A genomic DNA was prepared using Blood & Cell Culture Kit (QIAGEN Co.) from the mycelia.

### (2) Cloning of a partial sequence of a DNA encoding a protein having the activity in hydroxylating the 16-position of the macrolide compound 11107B

The following mix primers (5Dm-3F and 5D-1R) were designed and produced on reference to the amino acid sequence assumed to be that of the cytochrome P450 (CYP105D5) of Streptmyces coelicolor A3(2) (see the Sequence Nos. 17 and 21).
5Dm-3F: 5'-TTCGCSCTSCCSGTCCCSTCSATGGTSAT-3'
5D-1R: 5'-AGGTGCCCAGCGAGATCATGTT-3'

In order to promote reactivity taking the fluctuation of a codon into account, mixed bases S(=C+G) and Y(=C+T) were used.

Next, these two primers (5Dm-3F and 5D-1R) and the Mer-11107 genomic DNA obtained in the above (1) as the template were used to run a PCR reaction. The PCR reaction was accomplished by repeating a three-stage reaction including denaturing carried out at 98°C for 20 seconds, annealing carried out at 50°C for 2 minutes and elongation carried out at 68°C for 30 seconds 35 times by using Takara LA Taq (Takara Bio Inc.) and a PCR amplifier (T Gradient, Biometra Co.). A roughly 300 bp DNA fragment (hereunder called DNA fragment A2) was amplified as a result. It is highly possible that this DNA fragment A2 is a part of the DNA encoding a protein having hydroxylation activity. The DNA fragment-A2 amplified by the PCR reaction was recovered from the reaction solution by SUPREC PCR (Takara Bio Inc.).

Next, in order to obtain the DNA fragment-A2 in an amount enough to analyze the nucleotide sequence of the obtained DNA fragment-A2, the DNA fragment-A2 was combined with a plasmid vector pT7Blue T (Novagen Co.) using DNA Ligation Kit Ver. 2 (Takara Bio Inc.), to transform E. coli JM109 strain. The transformed E. coli was subsequently selected using a L-broth agar media (1.0% bacto tryptone, 0.5% yeast extract, 0.5% NaCl, 1.5% agar) containing ampicillin (50 µg/mL), X-gal (5-bromo-4-chloro-3-indolyl-β-D-galactoside; 40 µg/mL) and IPTG (isopropyl-β-D-thiogalactopyranoside; 100 µM). The transformed E. *coli* colonies isolated in this way were cultured in a L-broth liquid medium (1% bacto tryptone, 0.5% yeast extract, 0.5% NaCl) containing ampicillin (50 µg/mL). A plasmid DNA was isolated and purified from the mycelia of the proliferated transformed E. coli using a plasmid purifying kit (QIA filter Plasmid Midi Kit, QIAGEN Co.) to obtain enough amount of DNA fragment-A2.

### (3) Analysis of the nucleotide sequence of the cloned DNA fragment-A2

The nucleotide sequence of the DNA fragment-A2 obtained in the above (2) was analyzed using a DNA nucleotide sequence analyzer (PE Biosystems 377XL) according to a dye terminator cycle sequence method. While the DNA fragment-A2 amplified by a PCR reaction was measured at about 300 bp by electrophoresis, it was clarified by the nucleotide sequence analysis to be exactly 325 bp in length (see the nucleotide sequence 837 to nucleotide sequence 1161 of the Sequence No. 3). Since DNA sequences corresponding to the two types of primers used in the aforementioned PCR reaction were found at both ends of the above cloned 325 bp DNA sequence, it was clarified that the DNA fragment-A2 had been specifically amplified by these two types of primers (5Dm-3F and 5D-1R) in the above-mentioned PCR reaction.

### (4) Analysis of the neighboring region of the DNA fragment-A2

Once the partial sequence of the DNA encoding a protein having the hydroxylating activity derived from the Mer-11107 strain had been determined as described above, the nucleotide sequence of the neighboring region extending the upstream side and downstream side of the cloned fragment was amplified, cloned and sequenced by an inverse PCR method (Cell Technology Vol. 14, pp. 591-593, 1995). Specifically, the Mer-11107 strain genomic DNA (see the above (1)) was digested with a restriction enzyme BamHI in a K buffer solution (50 mM Tris-HCl, pH 8.5, 10 mM MgCl₂, 1 mM dithiothreitol, 100 mM KCl) and with a restriction enzyme SalI in a H buffer solution (50 mM Tris-HCl, pH 7.5, 10 mM MgCl₂, 1 mM dithiothreitol, 100 mM NaCl). The obtained each DNA fragment cleaved by the restriction enzymes was self-circularized by using DNA Ligation Kit Ver. 2 (Takara Bio Inc.).

Meanwhile, the following primers (7PIN-2F and 6PIN-2R) were designed and prepared from the nucleotide sequence of the DNA fragment-A2. (see the Sequence Nos. 22 and 20)
7PIN-2F: 5'-CCATGATCCTGCTGGTGGCCGGCCATGAGA-3'
6PIN-2R: 5'-CTGTTCCTCGAAGAACTCGTGGTCGGCGTA-3'

Next, these two types of primers (7PIN-2F and 6PIN-2R) and the above self-circularized Mer-11107 strain genomic DNA as a template, were used to run a PCR reaction. In the PCR reaction, the cycle of a two-stage reaction including denaturing carried out at 98°C for 20 seconds and annealing and elongation carried out at 68°C for 5 minutes was repeated 35 times by using Takara LA Taq (Takara Bio Inc.) and a PCR amplifier (T Gradient, Biometra Co.).

As a result, a DNA fragment (DNA fragment-B2) about 1.3kbp in size and a DNA fragment (DNA fragment-C2) about 1.4kbp in size were amplified. It is highly possible that these DNA fragments were respectively a DNA encoding a protein having hydroxylating activity and a DNA having a DNA sequence including those in the upstream and downstream regions of the former DNA.

The DNA fragment-B2 and DNA fragment-C2 were recovered from the PCR amplified reaction solution by SUPREC PCR (Takara Bio Inc.). Next, as to the obtained DNA fragment-B2 and DNA fragment-C2, in order to obtain each DNA fragment in an amount enough to analyze the nucleotide sequence of the obtained DNA fragment, a plasmid vector pT7Blue T (Novagen Co.), DNA Ligation kit ver.2 (TAKARA HOLDINGS INC.), E. coli JM109 strain and a plasmid purifying kit (QIA filter Plasmid Midi Kit, QIAGEN Co.) were used in the same manner as the above (2), to obtain enough amount of each DNA fragment.

### (5) Analysis of each nucleotide sequence of the DNA fragment-B2 (about 1.3 kbp in size) and the DNA fragment-C2 (about 1.4 kbp in size)

Each nucleotide sequence of the DNA fragment-B2 and DNA fragment-C2 obtained in the above (4) was analyzed using a DNA nucleotide sequence analyzer (PE Biosystems 377XL) according to a dye terminator cycle sequence method.The nucleotide sequences were thus analyzed to obtain the information of the nucleotide sequence of 2329 bp shown in the Sequence No. 3 from each sequence of the DNA fragment-B2 and DNA fragment-C2.

An open reading frame (ORF) in this 2329 bp was retrieved, to find that the two kinds of protein were coded. Each amino acid sequence of these proteins was retrieved by the BLAST search, and as a result, an ORF (hereinafter referred to as bpmA) encoding a protein consisting of 395 amino acids having high homology to cytochrome P450 existed in the base 420 to base 1604 of the Sequence No. 3. The bpmA had the highest homology (homology: 67.4%) to the amino acid sequence of the psmA isolated from the A-1544 strain and also had a relatively high homology (homology: 64.8%) to cytochrome P450 soy (Soy C)of Streptmyces griseus. It was considered from this fact that the bpmA highly possibly encoded hydroxylating enzyme of the cytochrome P-450 type.

Also, an ORF (hereinafter referred to as bpmB) encoding a protein having a high homology to ferredoxin of a 2Fe-2S type that existed just downstream (the base 1643 to base 1834 of the Sequence No. 3) of the bpmA. The protein encoded by the bpmB consisted of 64 amino acids, and had the highest homology (81.0%) to the amino acid sequence of the psmB isolated from the A-1544 strain and had a relatively higher homology (homology: 76.2%) to the amino acid sequence assumed to be that of ferredoxin just downstream of the amino acid sequence assumed to be cytochrome P450 (CYP105D5) of Streptmyces coelicolor A3(2). Therefore, it was considered that the bpmB served to transfer electrons and participated in hydroxylation together with the bpmA.

### Reference Example 4: DNA-3 encoding polypeptide having the 16-position hydroxylating enzyme activity on the macrolide compound (II)

### (1) Preparation of genomic DNA of A-1560 strain

The A-1560 strain was inoculated into medium containing 1% glucose, 0.4% wheat germ extract and 1% yeast extract, and incubated at 28°C for 3 days. The obtained culture broth was centrifuged at 3000 rpm for 10 minutes to collect the mycelia. A genomic DNA of A-1560 strain was prepared from the mycelia using Blood & Cell Culture Kit (QIAGEN Co.).

### (2) Cloning of a partial sequence of a DNA encoding a protein having the activity in hydroxylating the 16-position of the macrolide compound 11107B

The following mixed primers (5Dm-3F and 5Dm-2R) were designed and produced on reference to the amino acid sequence assumed to be that of the cytochrome P450 (CYP105D5) of Streptomyces coelicolor A3(2) (see the Sequence Nos. 17 and 23).
5Dm-3F: 5'-TTCGCSCTSCCSGTCCCSTCSATGGTSAT-3'
5Dm-2R: 5'-CTGGATSGTGTCSCCSGGYTT-3'

In order to promote reactivity taking the fluctuation of a codon into account, mixed bases S (=C+G) and Y (=C+T) were used.

Next, these two types of primers (5Dm-3F and 5Dm-2R) and the A-1560 genomic DNA obtained in the above (1) as the template, were used to run a PCR reaction. The PCR reaction was accomplished by repeating a three-stage reaction including denaturing carried out at 98°C for 20 seconds, annealing carried out at 50°C for 2 minutes and elongation carried out at 68°C for 30 seconds 35 times by using Takara LA Taq (Takara Bio Inc.) and a PCR amplifier (T Gradient, Biometra Co.). A roughly 750 bp DNA fragment (hereunder called DNA fragment-A3) was amplified as a result. It is highly possible that this DNA fragment-A3 is a part of DNA encoding a protein having hydroxylating activity. The DNA fragment-A3 amplified by a PCR reaction was recovered from the reaction solution by SUPREC PCR (Takara Bio Inc.).

Next, in order to obtain the DNA fragment-A3 in an amount enough to analyze the nucleotide sequence of the obtained DNA fragment-A3, DNA fragment-A3 was combined with a plasmid vector pT7Blue T (Novagen Co.) by using DNA Ligation Kit Ver. 2 (Takara Bio Inc.) to transform a colibacillus JM109 strain. (Stratagene Co.). Thereafter, the transformed colibacillus was selected using a L-broth agar media (1.0% bacto tryptone, 0.5% yeast extract, 0.5% NaCl, 1.5% agar) containing ampicillin (50 µg/mL), X-gal (5-bromo-4-chloro-3-indolyl-β-D-galactoside; 40 µg/mL) and IPTG (isopropyl-β-D-thiogalactopyranoside; 100 µM). The colony of the transformed colibacillus isolated in this way were cultured in a L-broth liquid medium (1% bacto tryptone, 0.5% yeast extract, 0.5% NaCl) containing ampicillin (50 µg/mL). A plasmid DNA was isolated and purified from the mycelia of the proliferated transformed colibacillus by using a plasmid purifying kit (QIA filter Plasmid Midi Kit, QIAGEN Co.), to obtain enough amount of DNA fragment-A3.

### (3) Analysis of the nucleotide sequence of cloned DNA fragment-A3

The nucleotide sequence of the DNA fragment-A3 obtained in the above (2) was analyzed using a DNA nucleotide sequence analyzer (PE Biosystems 377XL) according to a dye terminator cycle sequence method. While the DNA fragment-A3 amplified by a PCR reaction was measured at about 750 bp by electrophoresis, it was clarified by the nucleotide sequence analysis to be exactly 741 bp in length (see the nucleotide sequence 616 to nucleotide sequence 1356 of the Sequence No. 4). Since DNA sequences corresponding to the two types of primers used in the aforementioned PCR reaction were found at both ends of the above cloned 741 bp DNA sequence, it was clarified that the DNA fragment-A3 had been specifically amplified by these two types of primers (5Dm-3F and 5Dm-2R) in the aforementioned PCR reaction.

### (4) Analysis of the neighboring region of the DNA fragment-A3

Once the partial sequence of the DNA encoding a protein having hydroxylating activity derived from the A-1560 strain had been determined as described above, the nucleotide sequence of the neighboring region extending the upstream side and downstream side of the cloned fragment was amplified, cloned and sequenced by an inverse PCR method (Cell Technology Vol. 14, pp. 591-593, 1995). Specifically, the A-1560 genomic DNA (see the above (1)) was digested with a restriction enzyme BamHI in K buffer solution (50 mM Tris-HCl, pH 8.5, 10 mM MgCl₂, 1 mM dithiothreitol and 100 mM KCl), with a restriction enzyme KpnI in a L buffer solution (10 mM Tris-HCl, pH 7.5, 10 mM MgCl₂ and 1 mM dithiothreitol) and with a restriction enzyme SalI in a H buffer solution (50 mM Tris-HCl, pH 7.5, 10 mM MgCl₂, 1 mM dithiothreitol and 100 mM NaCl). The obtained each DNA fragment cleaved by the restriction enzymes was self-circularized by using DNA Ligation Kit Ver. 2 (Takara Bio Inc.).

Meanwhile, the following primers (5PIN-2F and 6PIN-2R) were designed and prepared from the nucleotide sequence of the DNA fragment-A3 (see the Sequence Nos. 24 and 20).
5PIN-2F: 5'-CGGAATCCACCAGTGCCTCGGCCAGAACCT-3'
6PIN-2R: 5'-CTGTTCCTCGAAGAACTCGTGGTCGGCGTA-3'

Next, these two types of primers (5PIN-2F and 6PIN-2R) and the above self-circularized A-1560 strain genomic DNA as a template, were used to run a PCR reaction. In the PCR reaction, the cycle of a two-stage reaction including denaturing carried out at 98°C for 20 seconds and annealing and elongation carried out at 68°C for 5 minutes was repeated 35 times by using Takara LA Taq (Takara Bio Inc.) and a PCR amplifier (T Gradient, Biometra Co.).

As a result, a DNA fragment (DNA fragment-B3) about 4.5 kbp in size, a DNA fragment (DNA fragment-C3) about 3.0 kbp in size and a DNA fragment (DNA fragment-D3) about 1.7 kbp in size were amplified. It was highly possible that these DNA fragments were a DNA encoding a protein having hydroxylating activity and a DNA having a DNA sequence including those in the upstream and downstream regions of the former DNA.

The DNA fragment-B3, the DNA fragment-C3 and the DNA fragment-D3 were recovered from the PCR amplified reaction solution by SUPREC PCR (Takara Bio Inc.). Next, as to the obtained DNA fragment-B3, DNA fragment-C3 and DNA fragment-D3, in order to obtain each DNA fragment in an amount enough to analyze the nucleotide sequence of the obtained DNA fragment, a plasmid vector pT7Blue T (Novagen Co.), DNA Ligation kit ver. 2 (Takara Bio Inc.), E. coli JM109 strain and a plasmid purifying kit (QIA filter Plasmid Midi Kit, QIAGEN Co.) were used in the same manner as the above (2), to obtain enough amount of each DNA fragment.

### (5) Analysis of each nucleotide sequence of the DNA fragment-B3 (about 4.5 kbp in size), the DNA fragment-C3 (about 3.0 kbp in size) and the DNA fragment-D3 (about 1.7 kbp in size)

Each nucleotide sequence of the DNA fragment-B3, DNA fragment-C3 and DNA fragment-D3 obtained in the above (4) was analyzed using a DNA nucleotide sequence analyzer (PE Biosystems 377XL) according to a dye terminator cycle sequence method. The nucleotide sequence was thus analyzed to obtain the information of the nucleotide sequence of 1860 bp shown in the Sequence No. 4 from each sequence of the DNA fragment-B3, DNA fragment-C3 and DNA fragment-D3.

An open reading frame (ORF) in this 1860 bp was retrieved, to find that the two kinds of protein were encoded. Each amino acid sequence of these proteins was retrieved by the BLAST search, and as a result, an ORF (hereinafter referred to as tpmA) encoding a protein consisting of 404 amino acids having high homology to cytochrome P450 existed in the base 172 to base 1383 of the Sequence No. 4. The tpmA had the highest homology (homology: 77.4%) to the amino acid sequence assumed to be that of cytochrome P450 (CYP105D5) of Streptmyces coelicolor A3(2) and also a high homology (homology: 76.6%) to the amino acid sequence of the psmA isolated from the A-1544 strain. It was considered from this fact that the tpmA was highly possibly a gene encoding hydroxylating enzyme of the cytochrome P-450 type.

Also, an ORF (hereinafter referred to as tpmB) encoding a protein having a high homology to ferredoxin of a 2Fe-2S type existed just downstream (the base 1399 to base 1593 of the Sequence No. 4) of the tpmA. The protein encoded by the tpmB consisted of 65 amino acids, and had the highest homology (87.3%) to the amino acid sequence of the psmB isolated from the A-1544 strain and also a high homology (homology: 82.5%) to the amino acid sequence assumed to be that of ferredoxin just downstream of the amino acid sequence assumed to be cytochrome P450 (CYP105D5) of Streptmyces coelicolor A3 (2). Therefore, it was considered that the tpmB served to transfer electrons and coded ferredoxin participating in hydroxylation together with the tpmA.

### Reference Example 5: Construction of plasmid pT7NS-camAB

The primers PRR-1F (see the Sequence No. 29) and PRR-2R (see the Sequence No. 30) with the sequences shown below were used to carry out PCR under the following conditions. with *Pseudomonas putida* ATCC 17453 genome DNA as the template.
PRR-1F: 5'-GCCCCCCATATGAACGCAAACGACAACGTGGTCATC-3'
PRR-2R: 5'-GCGGATCCTCAGGCACTACTCAGTTCAGCTTTGGC-3'

### (Reaction liquid composition)

| | |
|---|---|
| Sterile purified water: | 15 µL |
| 2x concentration GC buffer 1 (TAKARA HOLDINGS INC.) | 25 µL |
| dNTP mixed solution (2.5 mM each of dATP, dGTP, dTTP and dCTP) | 8 µL |
| PRR-1F primer (100 pmol/µL) | 0.5 µL |
| PRR-2R primer (100 pmol/µL) | 0.5 µL |
| *Pseudomonas putida* ATCC 17453 genome DNA (10 ng/µL) | 0.5 µL |
| LA Taq (5 units/pL, TAKARA HOLDINGS INC.) | 0.5 µL |

### (Temperature conditions)

95°C, 3 minutes
(98°C 20 sec, 63°C 30 sec, 68°C 2 min) 30 cycles
72°C, 5 minutes

The resulting 1.5 kb amplified fragment (camAB fragment) comprising the putidaredoxin reductase gene (camA) and the putidaredoxin gene just downstream from that gene (camB) was digested with restriction enzymes Nde I and Bam HI, and electrophoresed on 0.8% agarose gel. After electrophoresis, the camAB fragment was collected and purified using SUPREC-01 (TAKARA HOLDINGS INC.) from a gel section comprising that fragment which had been excised from the gel. The fragment was connected by T4 DNA ligase to the Nde I site and Bam HI site of *E. coli* plasmid vector pET11a (Stratagene Co.), and then transformed into *E. coli* DH5α to construct plasmid pT7-camAB. Next, a linker 1 molecule obtained by annealing the two synthetic oligo DNAs SP-1 (see the Sequence No. 31) and SP-2 (see the Sequence No. 32) with the sequences shown below was connected to the Nde I site of the plasmid by T4 DNA ligase and transformed into *E*. *coli* DH5α to construct plasmid pT7NS-camAB.
SP-1: 5'-TATGCGTCACTAGTCGGGAGTGCGTTA-3'
SP-2: 5'-TATAACGCACTCCCGACTAGTGACGCA-3'

### Example 1: Preparation of plasmid pPapsmAB by connection of pldA promoter and psmAB

The pldA promoter region of the pladienolide B biosynthesis gene was connected to psmAB in order to express psmAB in conjunction with the pladienolide B biosynthesis gene. The two primers pdlApro-SpeNdeF (SpeI and NdeI sites added to 5'-terminal: see the Sequence No. 25) and pldApro-NdeR (NdeI site added to 5'-terminal: see the Sequence No. 26) comprising the sequences shown below were synthesized based on the nucleotide sequence information of the Sequence No. 1 for purposes of amplifying the pldA promoter region.
pldApro-SpeNdeF: 5'-GGGCATATGACTAGTAGCCGTGTCCTGCCCGCC-3'
pldApro-NdeR: 5'-GGGCATATGTTCGGACGTGAATTCATTCG-3'

PCR was carried out under the following conditions using these primers.

### (PCR reaction liquid composition: using Toyo Boseki KOD-plus-)

| | |
|---|---|
| Sterile purified water: | 71 µL |
| 10 x concentration PCR buffer: | 10 µL |
| dNTP mixed solution (2 mM each dATP, dGTP, dCTP and dTTP): | 10 µL |
| MgSO₄ (25 mM) | 4 µL |
| KOD+ | 2 µL |
| pldApro-SpeNdeF (50 pmol/µL) | 1 µL |
| pldApro-NdeR (50 pmol/µL) | 1 µL |
| pKS35 (Referential Example 1 (6), 10 ng/µL) | 1 µL |

### (Reaction conditions: using Biometra T GRADIENT)

95°C, minutes
(98°C 20 sec, 63°C 1 min, 68°C 1 min) 25 cycles
72°C, 5 minutes

The 360 bp DNA fragment amplified as a result of this reaction was collected with a QIAquick PCR Purification Kit (QIAGEN Co.). The resulting 360 bp DNA fragment was digested with restriction enzyme NdeI. The plasmid pTC-DM (Reference Example 2(6)) was similarly digested with restriction enzyme NdeI. The resulting 360 bp DNA fragment digest and plasmid digest were connected using DNA Ligation Kit Ver. 2.1 (Takara Bio Inc.). A roughly 9.9 kbp plasmid pPapsmAB comprising the pldA promoter connected to psmAB was constructed in this way.

### Example 2: Preparation of plasmid for recombination

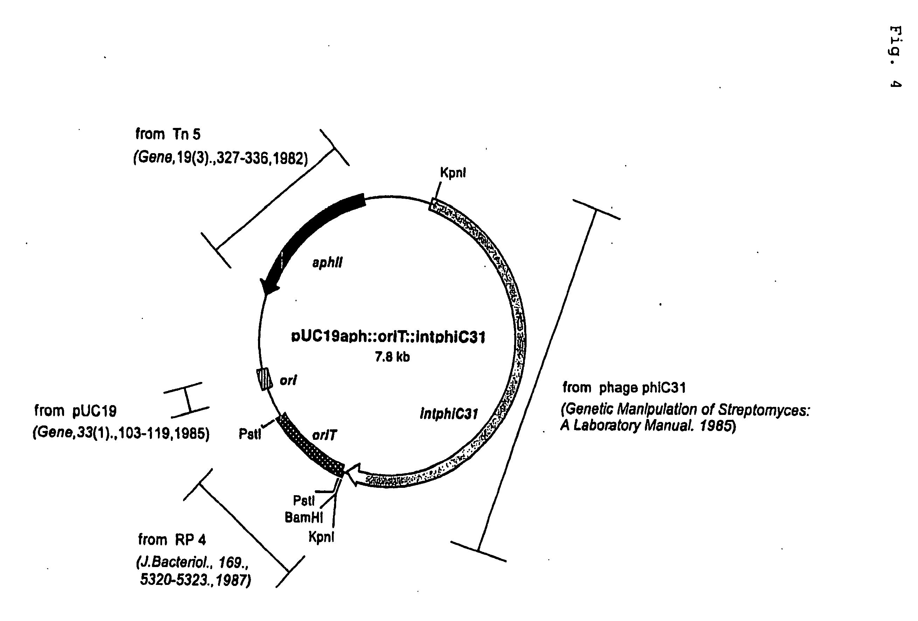

The pPapsmaAB plasmid prepared in Example 1 was digested with restriction enzyme SpeI, and blunt-ended using BKL Kit (Takara Bio Inc.). The resulting DNA was electrophoresed (Mupid-ex: Advance Co.) on 0.8% Molecular Biology Agarose^{™} (BIO-RAD), and the isolated 1.8 kbp DNA fragment comprising the pldA promoter and psmAB (sometimes called PapsmAB hereunder) was excised and collected and purified using a QIAquick Gel Extraction Kit (QIAGEN Co.). The recombination vector pUC19aph::oriT::intphiC31 shown in Fig. 4 was also digested with restriction enzyme BamHI, and blunt-ended with BKL Kit (Takara Bio Inc.). The resulting vector and PapsmAB were connected using DNA Ligation Kit Ver. 2.1 (Takara Bio Inc.). Thus, a recombination plasmid pAOC::PapsmAB was constructed having PapsmAB inserted into the recombination vector pUC19aph::oriT::intphiC31.

### Example 3: Introduction of recombination plasmid pAOC::PapsmAB into Mer-11107

The resulting pAOC::PapsmAB was transformed into conjugated *E. coli* S17-1 (ATCC47055) by electroporation to obtain S17-1/pAOC::PapsmAB. The resulting S17-1/pAOC::PapsmAB was, inoculated into 10 mL of LB medium (1% bacto tryptone, 0.5% yeast extract, 0.5% NaCl) containing 25 µg/ml kanamycin and shaking cultured at 30°C for 2 hours, and the mycelia were collected, washed twice with 10 mL of LB medium and suspended in 5 mL of LB medium. This was used as the donor suspension. While the donor suspension was being prepared, Mer-11107 was inoculated into 10 mL of TSB medium (Trypto-Soya broth: Nissui Seiyaku) and shaking incubated at 30°C for 5 hours, and the mycelia were collected, washed twice with 10 mL of sterilized water and suspended in 1 mL of sterilized water. This was used as the recipient suspension. 500 µL of the S17-1/pAOC::PapsmAB donor suspension was mixed with 10 µL of the resulting Mer-11107 recipient suspension, and applied to Actino Medium No. 4 agar medium (Nissui Pharmaceutical). After incubated at 30°C for 18 hours, this was covered with 2.5 mL of SNA (0.8% nutrient medium: Difco, 0.4% agar) containing 2 mg/mL ribostamycin, and incubated at 30°C for 7 days to obtain a the ribostamycin-resistant pAOC::PapsmAB-transformed strain Mer-11107::pAOC::PapsmAB. Example 4: Pladienolide production test of Mer-11107::pAOC::PapsmAB strain

The Mer-11107::pAOC::PapsmAB obtained in Example 3 and the parent Mer-11107 strain as a control were tested for productivity of pladienolide B and its 16-position hydroxide pladienolide D.

300 µL each of frozen seed of Mer-11107::pAOC::PapsmAB and Mer-11107 was inoculated into 60 mL of seed medium (soluble starch 2%, soy bean meal (ESUSAN-MEAT manufactured by Ajinomoto Co. Ltd.) 2%, yeast extract 0.3%, K₂HPO₄ 0.1%, MgSO₄·7H₂O 0.25%, CaCO₃ 0.3%, pH not adjusted) and incubated at 25°C for 3 days. 600 µL of the resulting seed culture broth was inoculated into 60 mL of main culture medium (soluble starch 5%, glucose 1%, Pharmamedia 3%, adekanol LG-126 0.05%, CaCO₃ 0.1%, pH 7.5), and cultured at 25°C for 3 days, 4 days, 5 days and 6 days. After the completion of the cultivation, the resulting culture broth was extracted by addition of 4 times the amount of acetonitrile. The amounts of pladienolide B and its 16-position hydroxide pladienolide D in the resulting extract were measured by HPLC. The measurement results are shown in Table 2. The HPLC measurement conditions were as follows.
Device: Shimadzu HPLC 1OAvp
Column: Develosil ODS UG-3 (ϕ 4.6 mm × 50 mm 3 µm) Mobile phase: 45% to 55% methanol (0 to 5 min)
   55% methanol (5 to 13 min)
   55% to 70% methanol (13 to 21 min)
   70% methanol (21 to 25 min)
Flow rate: 1.2 mL/min
Detection: UV 240 nm
Injection volume: 5 µL
Column temperature: 40°C
Analysis time: 25 min
Retention time: Pladienolide B 12.4 min
   Pladienolide D 4.3 min

**Table 2**

| | Mer-11107::pAOC::PapsmAB strain | | Mer-11107 strain | |
|---|---|---|---|---|
| | Pladienolide B (mg/L) | Pladienolide D (mg/L) | Pladienolide B (mg/L) | Pladienolide D (mg/L) |
| cultured for 3 days (72 hours) | 36.2 | 148.4 | 180.5 | 0.0 |
| cultured for 4 days (96 hours) | 93.2 | 289.3 | 338.1 | 0.0 |
| cultured for 5 days (120 hours) | 122.3 | 420.6 | 402.2 | 0.0 |
| cultured for 6 days (144 hours) | 118.3 | 535.8 | 372.2 | 0.0 |

While almost no production of pladienolide D was seen with the parent strain Mer-11107, pladienolide D was produced by the Mer-11107::pAOC::PapsmAB strain having the introduced pAOC::PapsmAB. This shows that direct production of pladienolide D can be achieved by introducing the pladienolide B 16-position hydroxylase gene psmAB into the pladienolide B-producing strain Mer-11107.

### Example 5: Effects of β-CD addition on pladienolide D production by Mer-11107::pAOC::PapsmAB strain

The effects of β-cyclodextrins (β-CD) addition on productivity of pladienolide B and its 16-position hydroxide pladienolide D by the Mer-11107::pAOC::PapsmAB strain obtained in Example 3 were investigated. 300 µL of frozen Mer-11107::pAOC::PapsmAB seed was inoculated into 30 mL of seed culture medium (soluble starch 2%, soy bean meal (ESUSAN-MEAT manufactured by Ajinomoto Co. Ltd.) 2%, yeast extract 0.3%, K₂HPO₄ 0.1%, MgSO₄·7H₂O 0.25%, CaCO₃ 0.3%, pH not adjusted), and incubated at 25°C for 3 days. 300 µL of the resulting seed culture broth was inoculated into 30 mL of main culture medium to which 2% β-CD was added (soluble starch 5%, glucose 1%, Pharmamedia 3%, adekanol LG-126 0.05%, β-CD 2.0%, CaCO₃ 0.1%, pH 7.5) or 30 mL of main culture medium without β-CD added (soluble starch 5%, glucose 1%, Pharmamedia 3%, adekanol LG-126 0.05%, CaCO₃ 0.1%, pH 7.5), and incubated for 3 days, 4 days, 5 days and 6 days at 25°C. After the completion of the cultivation, the resulting culture liquid was extracted by addition of 4 times the amount of acetonitrile. The amounts of pladienolide B and its 16-position hydroxide pladienolide D in the resulting extract were measured by HPLC. The measurement results are shown in Table 3. The HPLC measurement conditions were as follows.
Device: Shimadzu HPLC 10Avp
Column: Develosil ODS UG-3 (ϕ 9.6 mm × 50 mm 3 µm)
Mobile phase: 45% to 55% methanol (0 to 5 min)
   55% methanol (5 to 13 min)
   55% to 70% methanol (13 to 21 min)
   70% methanol (21 to 25 min)
Flow rate: 1.2 mL/min
Detection: UV 240 nm
Injection volume: 5 µL
Column temperature: 40°C
Analysis time: 25 min
Retention time: Pladienolide B 12.4 min
   Pladienolide D 4.3 min

**Table 3**

| | 2% β-CD added | | No β-CD added | |
|---|---|---|---|---|
| | Pladienolide B (mg/L) | Pladienolide D (mg/L) | Pladienolide B (mg/L) | Pladienolide D (mg/L) |
| cultured for 3 days (72 hours) | 199.4 | 202.0 | 36.2 | 148.4 |
| cultured for 4 days (96 hours) | 362.8 | 350.5 | 93.2 | 289.3 |
| cultured for 5 days (120 hours) | 484.3 | 580.1 | 122.3 | 420.6 |
| cultured for 6 days (144 hours) | 433.1 | 774.1 | 118.3 | 535.8 |

As a result, addition of 2% β-CD resulted in a roughly 1.4 times increase in pladienolide D accumulation, showing that addition of β-CD has an effect on direct production of pladienolide D.

### Example 6: Pladienolide production by Mer-11107::pAOC::PapsmAB strain (2)

300 µL of frozen seed of the Mer-11107::pAOC::PapsmAB strain obtained in Example 3 was inoculated into 30 mL of seed culture medium (soluble starch 2%, soy bean meal (ESUSAN-MEAT manufactured by Ajinomoto Co. Ltd.) 2%, yeast extract 0.3%, K₂HPO₄ 0.1%, MgSO₄·7H₂O 0.25%, CaCO₃ 0.3%, pH not adjusted) and incubated at 25°C for 3 days. 300 µL of the resulting seed culture broth was inoculated into 30 mL of main culture medium (soluble starch 5%, glucose 1%, Pharmamedia 3%, soy bean powder (J-Oil Mills: Honen SoyPro) 1%, adekanol LG-126 0.05%, β-CD 2.0%, CaCO₃ 0.1%, pH 7.5), and cultured for 3 days, 4 days, 5 days, 6 days and 7 days at 25°C.

After the completion of the cultivation, the resulting culture liquid was extracted by addition of 4 times the amount of acetonitrile. The amounts of pladienolide B and its 16-position hydroxide pladienolide D in the resulting extract were measured by HPLC. The measurement results are shown in Table 4. The HPLC measurement conditions were as follows.
Device: Shimadzu HPLC 10Avp
Column: Develosil ODS UG-3 (ϕ 4.6 mm × 50 mm 3 µm)
Mobile phase: 45% to 55% methanol (0 to 5 min)
   55% methanol (5 to 13 min)
   55% to 70% methanol (13 to 21 min)
   70% methanol (21 to 25 min)
Flow rate: 1.2 mL/min
Detection: UV 240 nm
Injection volume: 5 µL
Column temperature: 40°C
Analysis time: 25 minutes
Retention time: Pladienolide B 12.4 minutes
   Pladienolide D 4.3 minutes

**Table 4**

| | Pladienolide B (mg/L) | Pladienolide D (mg/L) |
|---|---|---|
| cultured for 3 days (72 hours) | 378.3 | 230.6 |
| cultured for 4 days (96 hours) | 601.4 | 567.9 |
| cultured for 5 days (120 hours) | 480.4 | 874.2 |
| cultured for 6 days (144 hours) | 387.4 | 985.4 |
| cultured for 7 days (168 hours) | 439.1 | 1215.9 |

50 mL of extract obtained by adding an equal amount of acetonitrile to the aforementioned culture broth was filtered, and the mycelia were washed with 30 mL of water. 70 mL of water was added to the filtrate, which was then extracted once with 100 mL and twice with 50 mL of ethyl acetate. The ethyl acetate layers were combined, washed twice with 50 mL of brine and dried over anhydrous sodium sulfate. After removing the solvent, the resulting residue was purified by thin layer chromatography (MERCK Silicagel 60 F254 0.5 mm developing solution; toluene:acetone=1:1) to obtain 12.8 mg of pladienolide B and 23.1 mg of pladienolide D. The ¹H-NMR analysis results for pladienolide B and pladienolide D were as follows.
Pladienolide B;
¹H-NMR spectrum (CD₃OD, 500 MHz): δ ppm (integration, multiplicity, coupling constant J (Hz)):
0.93 (3H, d, J=7.0Hz), 0.99 (3H, d, J=6.8Hz), 0.98 (3H, t, J=8.0Hz), 1.12 (3H, d, J=6.8Hz), 1.23 (3H,s), 1.25 (1H, m), 1.42 (2H, m), 1.53-1.70 (6H,m), 1.79 (3H, d, J=1.0Hz), 2.10 (3H,s), 2.52 (1H, m), 2.56 (2H,m), 2.60 (1H, m), 2.70 (1H, dd, J=2.4, 8.3Hz), 2.76 (1H, dt, J=2.4, 5.7Hz), 3.56 (1H, dt, J=8.3, 4.4Hz), 3.82 (1H; m), 5.08 (2H,d,J=9.8Hz), 5.60 (1H, dd, J=9.8, 15.2Hz), 5.70 (1H, dd, J=8.3, 15.2Hz), 5.74 (1H, dd, J=9.8, 15.2Hz), 6.13 (1H,d,J=9.8Hz), 6.36 (1H, dd, J=9.8, 15.2Hz)
Pladienolide D;
¹H-NMR spectrum (CD₃OD, 500 MHz): δ ppm (integration, multiplicity, coupling constant J (Hz)):
0.93 (3H,d,J=7.0Hz), 0.95 (3H,d,J=6.8Hz), 0.98 (3H, t, J=8.0Hz), 1.23 (3H,s), 1.30 (1H, m), 1.36-1.66 (9H,m), 1.70 (1H, dd, J=6. 4, 14.2Hz), 1.82 (3H,d,J=1.0Hz), 1.90 (1H, dd, J=6.4, 14.2Hz), 2.10 (3H,s), 2.52 (2H,m), 2.62 (1H, m), 2.72 (1H, dd, J=2.4, 8.3Hz), 2.94 (1H, dt, J=2.4, 5.7Hz), 3.55 (1H, dt, J=8.3, 4.4Hz), 3.82 (1H, m), 5.10 (1H,d,J=9.8Hz), 5.11 (1H,d,J=10.8Hz), 5.60 (1H, dd, J=9.8, 15.2Hz), 5.74 (1H, dd, J=8.3, 15.2Hz), 5.92 (1H,d,J=15.2Hz), 6.18 (1H,d,J=10.8Hz), 6.57 (1H,dd,J=10.8, 15.2Hz)

### Example 7: Introduction of recombination plasmid pAOC::PapsmAB into Mer-11107 pldB::hyg strain

The S17-1/pAOC::PapsmAB strain obtained in Example 3 was inoculated into 10 mL of LB medium (1% bacto tryptone, 0.5% yeast extract, 0.5% NaCl) comprising 25 µg/mL kanamycin and shaking cultured for 2 hours at 30°C, and the mycelia were collected, washed twice with 10 mL of LB medium and suspended in 5 mL of LB medium. This was the donor suspension.

While the donor suspension was being prepared, the Mer-11107 pldB::hyg strain prepared in Reference Example 1(8) was inoculated into 10 mL of TSB medium (Trypto-Soya broth: Nissui Pharmaceutical) and shaking cultured at 30°C for 5 hours, and the mycelia were collected, washed twice with 10 mL of sterilized water and suspended in 1 mL of sterilized water. This was the recipient suspension. 500 µL of the S17-1/pAOC::PapsmAB donor suspension was mixed with 10 µL of the resulting Mer-11107 pldB::hyg recipient suspension, and applied to Actino Medium No. 4 agar medium (Nippon Seiyaku). After incubated at 30°C for 18 hours, this was covered with 2.5 mL of SNA (0.8% nutrient medium: Difco, 0.4% agar) containing 2 mg/mL ribostamycin and incubated at 30°C for 7 days to obtain a ribostamycin-resistant pAOC::PapsmAB-transformed Mer-11107 pldB::hyg::pAOC::PapsmAB strain.

### Example 8: Pladienolide production test of Mer-11107 pldB::hyg::pAOC::PapsmAB strain

The Mer-11107 pldB::hyg::pAOC::PapsmAB strain obtained in Example 7 and the parent Mer-11107 pldB::hyg strain as a control were tested for productivity of ME-265 and its 16-position hydroxide ME-282. 300 µL each of frozen seed of Mer-11107 pldB::hyg::pAOC::PapsmAB and Mer-11107 pldB::hyg was inoculated into 30 mL of seed medium (soluble starch 2%, soy bean meal (ESUSAN-MEAT manufactured by Ajinomoto Co. Ltd.) 2%, yeast extract 0.3%, K₂HPO₄ 0.1%, MgSO₄·7H₂O 0.25%, CaCO₃ 0.3%, pH not adjusted) and incubated at 25°C for 3 days. 300 µL of the resulting seed culture broth was inoculated into 30 mL of main culture medium with 2% β-CD added thereto (soluble starch 5%, glucose 1%, Pharmamedia 3%, adekanol LG-126 0.05%, β-CD 2.0%, CaCO₃ 0.1%, pH 7.5), and incubated at 25°C for 3 days, 4 days, 5 days and 6 days. After the completion of the cultivation, the culture broth was extracted by addition of 4 times the amount of acetonitrile. The amounts of ME-265 and its 16-position hydroxide ME-282 in the resulting extract were measured by HPLC. The measurements results are shown in Table 5. The HPLC measurement conditions were as follows.
Device: Shimadzu HPLC 10Avp
Column: Develosil ODS UG-3 (ϕ 4.6 mm × 50 mm 3 µm)
Mobile phase: 45% to 55% methanol (0 to 5 min)
   55% methanol (5 to 13 min)
   55% to 70% methanol (13 to 17 min)
   70% methanol (1 to 35 min)
Flow rate: 1.2 mL/min
Detection: UV 240 nm
Injection volume: 5 µL
Column temperature: 40°C
Analysis time: 35 minutes
Retention time: ME-265 21.0 min
   ME-282 15.6 min

**Table 5**

| | Mer-11107 pldB::hyg::pAOC::PapsmAB strain | | Mer-11107pldB::hyg strain | |
|---|---|---|---|---|
| | ME-265 (mg/L) | ME-282 (mg/L) | ME-265 (mg/L) | ME-282 (mg/L) |
| cultured for 3 days (72 hours) | 47.8 | 153.8 | 485.7 | 0.0 |
| cultured for 4 days (96 hours) | 63.5 | 222.1 | 907.0 | 0.0 |
| cultured for 5 days (120 hours) | 70.5 | 280.5 | 1534.4 | 0.0 |
| cultured for 6 days (144 hours) | 71.8 | 309.6 | 2031.3 | 0.0 |

While almost no production of ME-282 was seen with the parent strain Mer-11107 pldB::hyg, ME-282 was produced by the Mer-11107 pldB::hyg::pAOC::PapsmAB strain into which pAOC::PapsmAB was introduced. This shows that direct production of M-282 can be achieved by introducing the ME-265 16-position hydroxylating enzyme gene psmAB into the ME-265 producing strain Mer-11107 pldB::hyg.

A filtration aid was added to about 90 mL of extract obtained by addition of an equal amount of acetonitrile to the aforementioned Mer-11107 pldB::hyg::pAOC::PapsmAB culture broth, which was then mixed and filtered through 60 ϕ Kiriyama funnel (Kiriyama filter paper No. 4) to isolate the mycelia. The mycelia were washed with 50 mL of water to obtain a mycelia isolation filtrate. The mycelia isolation filtrate was extracted twice with 100 mL of ethyl acetate. After drying over anhydrous sodium sulfate, the solvent was removed to obtain the residue. This was purified by thin layer chromatography (MERCK Silicagel 60 F254 0.5 mm developing solution; toluene:acetone=1:1) to obtain 0.5 mg of ME-265 and 12.5 mg of ME-282. The ¹H-NMR analysis results for the ME-265 and ME-282 were as follows. ME-265;
¹H-NMR spectrum (CD₃OD, 500 MHz) : δ ppm (integration, multiplicity, coupling constant J (Hz)):
0.87 (3H, d, J=7.0Hz), 0.90 (3H, d, J=7.0Hz), 0.94 (3H, d, J=7.3Hz), 0.97 (3H, d, J=7.0Hz), 1.08 (3H, d, J=7.0Hz), 1.17-1.21 (1H, m), 1.24-1.36 (2H, m), 1.42-1.52 (3H, m), 1.61-1.66 (3H, m), 1.74 (3H, d, J=1.1Hz), 1.89-1.96 (1H, m), 2.00 (3H, s), 2.41-2.97 (1H, m), 2.43 (1H, dd, J=5.5, 13.9Hz), 2.51-2.58 (1H, m), 2.56 (1H, dd, J=3.7, 13.9Hz), 2.65 (1H, dd, J=2.2, 8.1Hz), 2.72 (1H, dt, J=2.2, 5.9Hz), 3.51 (1H, dt, J=4.4, 8.4Hz), 3.75-3.80 (1H, m), 4.91 (1H, dd, J=8.8, 10.6Hz), 5.00 (1H, d, J=10.6Hz), 5.42 (1H, dd, J=9.2, 15.0Hz), 5.49 (1H, dd, J=9.2,15.0Hz), 5.65 (1H, dd, J=8.4, 15.0Hz), 6.08 (1H, d, J=10.6Hz), 6.32 (1H, dd, J=10.6, 15.0Hz)
ME-282;
¹H-NMR spectrum (CD₃OD 500 MHz): δ ppm (integration, multiplicity, coupling constant J (Hz)):
0.87 (3H, d, J=7.0Hz), 0.90 (3H, d, J=7.0Hz), 0.94 (3H, t, J=7.3Hz), 0.97 (3H, d, J=6.6Hz), 1.21-1.26 (1H, m), 1.29-1.37 (3H,m), 1.39 (3H, s), 1.44-1.52 (2H, m), 1.60-1.69 (1H,m), 1.65 (1H, dd, J=6.2, 13.9Hz), 1.77 (3H, d, J=1.1Hz), 1.86 (1H, dd, J=5.9, 13.9Hz), 1.89-1.94 (1H, m), 2.00(3H,s), 2.43 (1H, dd, J=5.5, 13.9Hz), 2.50-2.60 (1H, m), 2.56 (1H, dd, J=3.3,13.9Hz), 2.66 (1H, dd, J=2.2,7.7Hz), 2.89 (1H, dt, J=2.2,6.2Hz), 3.52 (1H, dt, J=9.8,8.4Hz), 3.75-3.80 (1H, m), 4.90 (1H, overlapped with D₂O), 5.01 (1H, d, J=10.6Hz), 5.42 (1H, dd, J=9.2, 15.0Hz), 6.13 (1H, d, J=10.6Hz), 6.52 (1H, dd, J=11.0, 15.0Hz)

### Example 9: Preparation of plasmid pPepsmAB comprising ermE promoter connected to psmAB

The ermE promoter region was ligated to psmAB in order to cause expression of psmAB using the ermE promoter. The two primers eDM-SphF (SphI site added to 5'-terminal: see Sequence No. 27) and eDM-SphR (SphI site added to 5'-terminal: see Sequence No. 28) comprising the sequences shown below were synthesized based on the nucleotide sequence information of Sequence No. 2 for purposes of amplifying psmAB.
eDM-SphF: 5'-TCCCCGCATGCCGGAACTGACGGACATCAC-3'
eDM-SphR: 5'-CCCGGGCATGCAGACGACGACGTAGAGGAA-3'

These primers were used to carry out PCR under the following conditions.

### (PCR reaction liquid composition: using Takara LA Taq (Takara Bio Inc.))

| | |
|---|---|
| Sterile purified water: | 17 µL |
| 2x concentration GC buffer II | 25 µL |
| dNTP mixed solution (2 mM each dATP, dGTP, dCTP and dTTP): | 5 µL |
| LA Taq | 0.5 µL |
| eDM-SphF (50 pmol/µL) | 0.5 µL |
| eDM-SphR (50 pmol/µL) | 0.5 µL |
| *Streptomyces esp.* A-1544 genome DNA | 1 µL |

### (Reference Example 2(1), 10 ng/µL)

### (Reaction conditions: using T GRADIENT (Biometra))

95°C, 3 minutes
(98°C 20 sec, 68°C 4 min) 30 cycles
68°C, 5 minutes

The 1570 bp DNA fragment comprising psmAB that was amplified as a result of this reaction was collected with a QIAquick PCR Purification Kit (QIAGEN Co.). The resulting 1570 bp DNA fragment was digested with restriction enzyme SphI. The plasmid pSK117 *(*J. Bacteriol. Vol. 2002 184, 6417-6423) was similar digested with restriction enzyme SphI. The resulting 1570 bp DNA fragment digest comprising psmAB and the plasmid digest were connected using DNA Ligation Kit Ver. 2.1 (Takara Bio Inc.). A plasmid pPepsmAB comprising the ermE promoter connected to psmAB was constructed in this way.

### Example 10: Preparation of recombination plasmid containing psmAB connected to ermE promoter

The plasmid pPepsmAB prepared in Example 9 was digested with restriction enzymes KpnI and BglII, and blunt-ended using BKL Kit (Takara Bio Inc.). The resulting DNA was electrophoresed (Mupid-ex: Advance) on 0.8% Molecular Biology Agarose^{™} (BIO-RAD), and the isolated 2060 bp DNA fragment (hereunder called PepsmAB) comprising the ermE promoter and psmAB was excised and collected and purified by QIAquick Gel Extraction Kit (QIAGEN Co.). The recombination vector pUC19aph::oriT::intphiC31 shown in Fig. 4 was also digested with restriction enzyme BamHI, and blunt-ended with BKL Kit (Takara Bio Inc.). The resulting vector was connected to PepsmAB using DNA Ligation Kit Ver. 2.1 (Takara Bio Inc.). Thus, the recombination plasmid pAOC::PepsmAB having PepsmAB inserted into the recombination vector pUC19aph::oriT::intphaiC31 was constructed.

### Example 11: Preparation of recombination plasmid containing natural psmAB

A primer DM-BglF (5'-CGCATAGATCTTCACCCGAGCGGGTGATCA-3': see the Sequence No. 35) having a BglII site added to the 5'-end and a primer DM-BglR (5'-TCCCGAGATCTTGAAGGTCCGCGTCACCGT-3': see the Sequence No. 36) having a BglII site added to the 5'-end were designed and prepared with reference to the nucleotide sequence of the Sequence No. 2. Next, these two primers (DM-BglF and DM-BglR) and the A-1544 genomic DNA obtained in Reference Example 2(1) as the template were used to carry out PCR reaction. The PCR reaction was conducted by repeating a three-stage reaction including denaturing carried out at 98°C for 20 seconds, annealing carried out at 63°C for 30 seconds and elongation carried out at 68°C for 4 minutes 30 times by using Takara LA Taq (Takara Bio Inc.) and a PCR amplifier (T Gradient, Biometra Co.). A roughly 3.5 kbp DNA fragment (hereunder called PopsmAB) comprising natural psmA and psmB was collected from the PCR amplification reaction liquid by agarose gel electrophoresis and SUPREC 01 (Takara Bio Inc.). The resulting DNA fragment was digested with restriction enzyme BglII. The pUC19aph::oriT::intphiC31 recombination vector shown in Fig. 4 was also digested with restriction enzyme BamHI. The resulting vector and PopsmAB were connected using DNA Ligation Kit Ver. 2.1 (Takara Bio Inc.). A recombination plasmid pAOC::PopsmAB comprising PopsmAB inserted into the recombination vector pUC19aph::oriT::intphiC31 was thus constructed.

### Example 12: Introduction of recombination plasmids pUC19aph::oriT::intphiC31, pAOC::PepsmAB and pAOC::PopsmAB into Mer-11107.

The recombination vector pUC19aph::oriT::intphiC31 and the pAOC::PepsmAB and pAOC::PopsmAB obtained in Examples 10 and 11 were each transformed into conjugated E. coli S17-1 (ATCC47055) by electroporation to obtain S17-1/pUC19aph::oriT::intphiC31, S17-1/pAOC::PepsmAB and S17-1/pAOC::PoepsmAB strains. The resulting three strains were inoculated into 10 mL of LB medium (1% bacto tryptone, 0.5% yeast extract, 0.5% NaCl) comprising 25 µg/mL kanamycin and shaking cultured at 30°C for 2 hours, after which the mycelia were collected, washed twice with 10 mL of LB medium and suspended in 5 mL of LB medium. These were the donor suspensions.

While the donor suspension was being prepared, Mer-1107 was inoculated into 10 mL of TSB medium (Trypto-Soya broth: Nissui Seiyaku) and shaking cultured at 30°C for 5 hours, after which the mycelia were collected, washed twice with 10 mL of sterile water and suspended in 1 mL of sterile water. This was the recipient suspension. 500 µL of each of the donor suspensions of the obtained three strains above was mixed with 10 µL of the Mer-11107 recipient suspension, and applied to Actino Medium No. 4 agar medium. After incubated at 30°C for 18 hours, these were covered with 2.5 mL of SNA (0.8% nutrient medium: Difco, 0.4% agar) comprising 2 mg/mL ribostamycin, and incubated at 30°C for 7 days to obtain ribostamycin-resistant pUC19aph::oriT::intphiC31-transformed Mer-11107::pAOC, pAOC::PepsmAB-transformed Mer-11107::pAOC::PepsmAB and pAOC::PopsmAB-transformed Mer-11107::pAOC::PopsmAB strains.

### Example 13: Pladienolide production test of Mer-11107::pAOC strain, Mer-11107::pAOC::PepsmAB strain and Mer-11107::pAOC::PopsmAB strain

The Mer-11107::pAOC strain, Mer-11107::pAOC::PepsmAB strain and Mer-11107::pAOC::PopsmAB strain obtain in Example 12 were tested for productivity of pladienolide B and its 16-position hydroxylate pladienolide D. 300 µL each of frozen seed of Mer-11107::pAOC, Mer-11107::pAOC::PepsmAB and Mer-11107::pAOC::PopsmAB was inoculated into 20 mL of seed medium (soluble starch 2%, soy bean meal (ESUSAN-MEAT manufactured by Ajinomoto Co. Ltd.) 2%, yeast extract 0.3%, K₂HPO₄ 0.1%, MgSO₄·7H₂O 0.25%, CaCO₃ 0.3%, pH not adjusted) and incubated at 25°C for 2 days. 300 µL of the resulting seed culture broth was inoculated into 30 mL of main culture medium (soluble starch 5%, glucose 1%, Pharmamedia 3%, CaCO₃ 0.1%, pH 7.5) and incubated at 25°C for 4 days. After the completion of the cultivation, the culture broth was extracted by addition of 9 times the amount of acetonitrile. The amounts of pladienolide B and its 16-position hydroxide pladienolide D in the resulting extract were measured by HPLC. The measurement results are shown in Table 6. The HPLC measurement conditions were as follows.
Device: Shimadzu HPLC 10Avp
Column: Develosil ODS UG-3 (ϕ 4.6 mm × 50 mm 3 µm)
Mobile phase: 45% to 55% methanol (0 to 5 min)
   55% methanol (5 to 13 min)
   55% to 70% methanol (13 to 21 min)
   70% methanol (21 to 25 min)
Flow rate: 1.2 mL/min
Detection: UV 240 nm
Injection volume: 5 µL
Column temperature: 40°C
Analysis time: 25 min
Retention time: Pladienolide B 12.4 min
   Pladienolide D 4.3 min

**Table 6**

| | Mer-11107::pAOC C strain | Mer-11107::pAOC: :PepsmAB strain | Mer-11107::pAOC: :PopsmAB strain |
|---|---|---|---|
| Pladienolide B (mg/L) | 243.0 | 309.0 | 241.3 |
| Pladienolide D (mg/L) | 0.0 | 54.8 | 18.8 |

While almost no production of pladienolide D was seen with Mer-11107::pAOC in which only the vector was introduced, pladienolide D was produced by the Mer-11107::pAOC::PepsmAB and Mer-11107::pAOC::PopsmAB strains having introduced pAOC::PepsmAB and pAOC::PopsmAB. This shows that direct production of pladienolide D can be achieved by introducing the pladienolide B 16-position hydroxylase gene psmAB into the pladienolide B producing strain Mer-11107.

### Example 14: Preparation of pPampsmAB comprising variant psmA

pPampasmAB-CLM, pPampsmAB-CLLM, pPampsmAB-CLFM and pPampsmAB-TY having mutations introduced using QuickChange™ Site-Directed Mutagenesis Kit with the pPapsmAB prepared in Example 1 as the template were prepared in order to add site-directed mutations to psmA.

Primers R91C-F and R91C-R (see the Sequence Nos. 37 and 38) having the sequences shown below were first synthesized:
R91C-F: 5'-TTCGCGGCCGTCTGCGACCGGCGGGTG-3'
R9AC-R: 5'-CACCCGCCGGTCGCAGACGGCCGCGAA-3'
and a mutation was introduced using QuickChange™ Site-Directed Mutagenesis Kit with pPapsmAB as the template, replacing the #91 arginine of PsmA with cysteine to prepare pPampsmAB-C.

Next, primers R195L-F and R195L-R (see the Sequence Nos. 39 and 40) having the sequences shown below were synthesized:
R195L-F: 5'-TCGCAAGGGGCGCTCGAGCGGCTCGAG-3'
R195L-R: 5'-CTCGAGCCGCTCGAGCGCCCCTTCCGA-3'
and a mutation was introduced using QuickChange™ Site-Directed Mutagenesis Kit with pPapsmAB-C as the template, replacing the #195 arginine of PsmA with leucine to prepare pPampsmAB-CL.

Next, primers L403M-F and L403M-R (see the Sequence Nos. 41 and 42) having the sequences shown below were synthesized:
L403M-F: 5'-ACGATCCAGGGGATGATGGAACTCCCCGTGA-3'
L403M-R: 5'-TCACGGGGAGTTCCATCATCCCCTGGATCG-3'
and a mutation was introduced using QuickChange™ Site-Directed Mutagenesis Kit with pPapsmAB-CL as the template, replacing the #403 leucine of PsmA with methionine to prepare pPampsmAB-CLM.

Next, primers R236L-F and R236L-R (see the Sequence Nos. 43 and 44) having the sequences shown below were synthesized:
R236L-F: 5'-AGCTGGACCGACTCGACGTGGTGGCGCTGG-3'
R236L-R: 5'-AGCGCCACCACGTCGAGTCGGTCCAGCTC-3'
and a mutation was introduced using QuickChange™ Site-Directed Mutagenesis Kit with pPapsmAB-CLM as the template, replacing the #236 arginine of PsmA with leucine to prepare pPampsmAB-CLLM.

Next, primers I249F-F and I249F-R (see the Sequence Nos. 45 and 46) having the sequences shown below were synthesized:
I244F-F: 5'-TGGCGCTGGCCGTCTTCCTGCTCGTGG-3'
I249F-R: 5'-CCACGAGCAGGAAGACGGCCAGCGCCACCA-3'
and a mutation was introduced using QuickChange™ Site-Directed Mutagenesis Kit with pPapsmAB-CLM as the template, replacing the #244 isoleucine of PsmA with phenylalanine to prepare pPampsmAB-CLFM.

Next, primers M112T-F and M112T-R (see the Sequence Nos. 47 and 48) having the sequences shown below were synthesized:
M112T-F: 5'-CAGCGGCGGATGACGATCCCGTCGTTC-3'
M112T-R: 5'-GAACGACGGGATCGTCATCCGCCGCTG-3'
and a mutation was introduced using QuickChange™ Site-Directed Mutagenesis Kit with pPapsmAB as the template, replacing the #112 methionine of PsmA with threonine to prepare pPampsmAB-T.

Finally, primers R195Y-F and R195Y-R (see the Sequence Nos. 49 and 50) having the sequences shown below were synthesized:
R195Y-F: 5'-AGGGGCGCGCGAGTACCTCGAGGAGTACCT-3'
R195Y-R: 5'-GGTACTCCTCGAGGTACTCGCGCGCCCCTT-3'
and a mutation was introduced using QuickChange™ Site-Directed Mutagenesis Kit with pPapsmAB-T as the template, replacing the #195 arginine of PsmA with tyrosine to prepare pPampsmAB-TY.

### Example 15: Preparation of introduction plasmid by double homologous recombination

In the belief that psmAB or variants thereof could be introduced by double homologous recombination using a nucleotide sequence comprising DNA participating in pladienolide biosynthesis that had already been determined in Reference Example 1 (7) determining the nucleotide sequence of the pladienolide biosynthesis gene cluster, along with neighboring DNA thereof, an introduction plasmid was prepared by the following methods.

The Four primers, pldout-L-Bgl2F, pldout-L-Sph1R, pldout-R-SphlF and pldout-R-Bgl2R (see the Sequence Nos. 51, 52, 53 and 54) having the sequences shown below, were synthesized based on the nucleotide sequence downstream from DNA participating in pladienolide biosynthesis in order to amplify a sequence for purposes of double homologous recombination:
pldout-L-Bgl2F: 5'-GGGAGATCTGCAGGTCATCGGGGGGAAGAACCACA-3'
pldout-L-Sph1R: 5'-TCTCCAGCCGCATGCGGTCCCGGGTCACCGT-3'
pldout-R-SphlF: 5'-CCCGCATGCGGCTGGAGATCATCCAGAGCGCA-3'
pldout-R-Bgl2R: 5'-GGGAGATCTAGAACATGCCGGGCCAGAGGCTGAC-3'

These primers were used to run a PCR under the following conditions.

### (PCR reaction liquid composition)

| | |
|---|---|
| Sterile purified water | 30 µL |
| 2x concentration GC buffer II | 50 µL |
| dNTP mixed solution (2.5 mM each dATP, dGTP, dTTP and dCTP) | 16 µL |
| pldout-L-Bgl2F or pldout-R-Sph1F (50 pmol/µL) | 1 µL |
| pldout-L-Sph1R or pldout-R-Bg12R (50 pmol/µL) | 1 µL |
| Mer-11107 total DNA (100 ng/µL) | 1 µL |
| LA Taq polymerase (5u/µL, TAKARA HOLDINGS INC.) 1 | µL |

### (Reaction temperature conditions)

| | |
|---|---|
| 95°C, 3 minutes | 30 cycles |
| (98°C 20 sec, 65°C 30 sec, 68°C 3 min) | |
| 72°C, 5 minutes | |

A 2.08 kb DNA fragment (DNA fragment LZ) was amplified from the reaction using pldout-L-Bgl2F and pldout-L-Sph1R, while a 2.73 kb DNA fragment (DNA fragment RX) was amplified from the reaction using pldout-R-Sph1F and pldout-R-Bgl2R. DNA fragments LZ and RX were purified with QIAGEN PCR Purification Kit (QIAGEN Co.), and digested with restriction enzymes BglII and SphI.

Next, the two primers hyg-SpeSphF and hyg2-SphR (see the Sequence Nos. 55 and 56) having the sequences shown below were synthesized based on the nucleotide sequences for purposes of amplifying the hygromycin resistance gene: hyg-SpeSphF: 5'-GGGGCATGCGGACTAGTACACCGTCGCCTCGGT-3' hyg2-SphR: 5'-GCCGCATGCGTCAGGCGCCGGGGGCGGTGT-3'

These primer were used to run a PCR under the following conditions.

### (PCR reaction liquid composition)

| | |
|---|---|
| Sterile purified water | 30 µL |
| 2x concentration GC buffer II | 50 µL |
| dNTP mixed solution (2.5 mM each dATP, dGTP, dTTP and dCTP) | 16 µL |
| hyg-SpeSphF (50 pmol/µL) | 1 µL |
| hyg2-SphR (50 pmol/pL) | 1 µL |
| *Streptomyces hygroscopicus* JCM 4772strain | 1 µL |
| total DNA (100 ng/µL) | |
| LA Taq polymerase (5u/µL, TAKARA HOLDINGS INC.) | 1 µL |

### (Reaction temperature conditions)

| | |
|---|---|
| 95°C, 6 minutes | 30 cycles |
| (98°C 20 sec, 63°C 30 sec, 68°C 2 min) | |
| 72°C, 5 minutes | |

The 1.21 kb DNA fragment (DNA fragment hyg2) amplified as a result of this reaction was purified with QIAGEN PCR Purification Kit (QIAGEN Co.) and digested with restriction enzyme SphI.

The DNA fragments LZ and RX digested with restriction enzymes BglII and SphI, the DNA fragment hyg2 digested with restriction enzyme SphI and shuttle vector pKU253 (see Fig. 3) digested with restriction enzyme BamHI were all 4 connected together with DNA Ligation Kit Ver. 2.1 (Takara Bio Inc.). Thus, a roughly 22 kb plasmid pKU253-LZ-hyg2-RX for double homologous recombination was constructed comprising pKU253 having inserted therein a roughly 6.0 kb DNA fragment comprising DNA fragment hyg2 inserted between DNA fragments LZ and RX.

### Example 16: Insertion of psmAB or variants thereof into introduction plasmid pKU253-LZ-hyg2-RX by double homologous recombination

The pPapsmAB constructed in Example 1 and the pPampsmAB-CLM, pPampsmAB-CLLM, pPampsmAB-CLFM and pPampsmAB-TY constructed in Example 14 were digested with restriction enzyme SpeI. Each kind of DNA digested with restriction enzyme SpeI was electrophoresed (Mupid-ex: Advance) on 0.8% Certified^{™} Molecular Biology Agarose (BIO-RAD), and the isolated 1.8 kbp DNA fragment comprising the pldA promoter and psmAB or variants thereof (hereunder sometimes called PapsmAB, PampsmAB-CLM, PampsmAB-CLLM, PapmsmAB-CLFM or PampsmAB-TY) was excised and collected and purified with QIAquick Gel Extraction Kit (QIAGEN Co.). The resulting DNA fragments (PapsmAB, PampsmAB-CLM, PampsmAB-CLLM, PapmsmAB-CLFM and PampsmAB-TY) were connected using DNA Ligation Kit Ver. 2.1 (Takara Bio Inc.) to the pKU253-LZ-hyg2-RX prepared in Example 15, which had been digested with restriction enzyme SpeI. The respective DNA fragments (PapsmAB, PampsmAB-CLM, PampsmAB-CLLM, PapmsmAB-CLFM and PampsmAB-TY) were thus inserted upstream from the hyg2 of pKU253-LZ-hyg2-RX to construct the roughly 24 kb plasmids pKU253-LZ-hyg2-PapsmAB-RX, pKU253-LZ-hyg2-PampsmAB-CLM-RX, pKU253-LZ-hyg2-PampsmAB-CLLM-RX, pKU253-LZ-hyg2-PampsmAB-CLFM-RX and pKU253-LZ-hyg2-PampsmAB-TY-RX for purposes of introducing psmAB or variants thereof by double homologous recombination.

### Example 17: Introduction of psmAB or variants thereof into Mer-11107 by double homologous recombination

The resulting pKU253-LZ-hyg2-PapsmAB-RX, pKU253-LZ-hyg2-PampsmAB-CLM-RX, pKU253-LZ-hyg2-PampsmAB-CLLM-RX, pKU253-LZ-hyg2-PampsmAB-CLFM-RX and pKU253-LZ-hyg2-PampsmAB-TY-RX were each transformed by electroporation into conjugated E. *coli* S17-1 to obtain S17-1/pKU253-LZ-hyg2-PapsmAB-RX, S17-1/pKU253-LZ-hyg2-PampsmAB-CLM-RX, S17-1/pKU253-LZ-hyg2-PampsmAB-CLLM-RX, S17-1/pKU253-LZ-hyg2-PampsmAB-CLFM-RX and S17-1/pKU253-LZ-hyg2-PampsmAB-TY-RX. The resulting S17-1/pKU253-LZ-hyg2-PapsmAB-RX, S17-1/pKU253-LZ-hyg2-PampsmAB-CLM-RX, S17-1/pKU253-LZ-hyg2-PampsmAB-CLLM-RX, S17-1/pKU253-LZ-hyg2-PampsmAB-CLFM-RX and S17-1/pKU253-LZ-hyg2-PampsmAB-TY-RX were inoculated into LB medium (1% bacto tryptone, 0.5% yeast extract, 0.5% NaCl) comprising 25 µg/mL of kanamycin and 100 µg/mL of hygromycin B and shaking cultured at 30°C for 2 hours, and the mycelia were collected, washed twice with 10 mL of LB medium and suspended in 5 mL of LB medium. These were the donor suspensions.

While the donor suspensions were being prepared, Mer-11107 was inoculated into 10 mL of TSB medium (Trypto-Soya broth: Nissui Seiyaku) and shaking cultured at 30°C for 5 hours, and the mycelia were collected, washed twice with 10 mL of sterilized water and suspended in 1 mL of sterilized water. This was the recipient suspension. 500 mL each of the S17-1/pKU253-LZ-hyg2-PapsmAB-RX, S17-1/pKU253-LZ-hyg2-PampsmAB-CLM-RX, S17-1/pKU253-LZ-hyg2-PampsmAB-CLLM-RX, S17-1/pKU253-LZ-hyg2-PampsmAB-CLFM-RX and S17-1/pKU253-LZ-hyg2-PampsmAB-TY-RX donor suspensions was mixed with 10 µL of the Mer-11107 recipient suspension, and applied to Actino Medium No. 4 agar medium (Nippon Seiyaku). After 18 hours of culture at 30°C, this was covered with 2.5 mL of SNA (0.8% nutrient medium: Difco, 0.4% agar) comprising 2 mg/mL of ribostamycin. These were incubated at 30°C for 7 days to obtain ribostamycin-resistant pKU253-LZ-hyg2-PapsmAB-RX, pKU253-LZ-hyg2-PampsmAB-CLM-RX, pKU253-LZ-hyg2-PampsmAB-CLLM-RX, pKU253-LZ-hyg2-PampsmAB-CLFM-RX and pKU253-LZ-hyg2-PampsmAB-TY-RX transformants.

The resulting pKU253-LZ-hyg2-PapsmAB-RX, pKU253-LZ-hyg2-PampsmAB-CLM-RX, pKU253-LZ-hyg2-PampsmAB-CLLM-RX, pKU253-LZ-hyg2-PampsmAB-CLFM-RX and pKU253-LZ-hyg2-PampsmAB-TY-RX transformants were each inoculated into 10 mL of TSB medium containing no ribostamycin, and shaking cultured at 30°C for 24 hours. Each of pKU253-LZ-hyg2-PapsmAB-RX, pKU253-LZ-hyg2-PampsmAB-CLM-RX, pKU253-LZ-hyg2-PampsmAB-CLLM-RX, pKU253-LZ-hyg2-PampsmAB-CLFM-RX and pKU253-LZ-hyg2-PampsmAB-TY-RX transformant culture liquid was collected and, washed twice with 10 mL of sterilized water and suspended in 10 mL of sterilized water. Each suitably diluted suspension was applied to YMS agar medium (0.4% yeast extract, 1% wheat germ extract, 0.4% soluble starch, 2% agar, 10 mM calcium chloride) comprising 200 µg/mL hygromycin B, and incubated at 30°C for 4 days. Single colonies growing in YMS agar medium comprising hygromycin B were transplanted to YMS agar medium comprising 200 µg/mL of hygromycin B and YMS agar medium comprising 200 ug/mL of ribostamycin, and incubated at 30°C for 2 days. After the cultivation, hygromycin B-resistant, ribostamycin-sensitive strains were selected. The resulting strains were strains having the hygromycin B-resistance gene hyg2 and psmAB or variants thereof inserted downstream in the genome (between nucleotides 73362 and 73369 in the Sequence No. 1) from DNA participating in pladienolide biosynthesis, and were called Mer-11107-ZX::hyg2::PapsmAB, Mer-11107-ZX::hyg2::PampsmAB-CLM, Mer-11107-ZX::hyg2::PampsmAB-CLLM, Mer-11107-ZX::hyg2::PampsmAB-CLFM and Mer-11107-ZX::hyg2::PampsmAB-TY. Example 18: Pladienolide production by Mer-11107-ZX::hyg2::PapsmAB, Mer-11107-ZX::hyg2::PampsmAB-CLM, Mer-11107-ZX::hyg2::PampsmAB-CLLM, Mer-11107-ZX::hyg2::PampsmAB-CLFM and Mer-11107-ZX::hyg2::PampsmAB-TY

500 µL each of frozen seed of the Mer-11107-ZX::hyg2::PapsmAB, Mer-11107-ZX::hyg2::PampsmAB-CLM, Mer-11107-ZX::hyg2::PampsmAB-CLLM, Mer-11107-ZX::hyg2::PampsmAB-CLFM and Mer-11107-ZX::hyg2::PampsmAB-TY obtained in Example 17 was inoculated into 25 mL of seed medium (soluble starch 2%, soy bean meal (ESUSAN-MEAT manufactured by Ajinomoto Co. Ltd.) 2%, yeast extract 0.3%, K₂HPO₄ 0.1%, MgSO₄·7H₂O 0.25% CaCO₃ 0.3%, pH not adjusted) and incubated at 25°C for 3 days. 300 µL of the resulting seed culture broth was inoculated into 30 mL of main cultured medium (soluble starch 5%, glucose 1%, Pharmamedia 3%, SoyPro 1%, adekanol LG-126 0.05%, β-CD 2.0%, CaCO₃ 0.1%, pH 7.5), and incubated at 25°C for 6 days.

After the completion of the cultivation, the resulting culture broth was extracted by addition of 4 times the amount of acetonitrile. The amounts of pladienolide D, pladienolide H13 corresponded the 20-position hydroxyl pladienolide D, pladienolide C2 corresponded the 21-position carbonyl pladienolide D and pladienolide C3 corresponded the 20-position hydroxyl and 21-position carbonyl pladienolide D in the resulting extract were measured by HPLC. The measurement results are shown in Table 7. The HPLC measurement conditions were as follows.

### (HPLC analysis conditions)

Device: Shimadzu HPLC 10Avp
Column: Develosil ODS UG-3 (4.6 mm × 50 mm 3 µm)
Mobile phase: 20% to 35% acetonitrile (0 to 13 min)
   35% to 100% acetonitrile (13 to 17 min)
   20% acetonitrile (17 to 22 min)
Flow rate: 1.0 mL/min
Detection: UV 240 nm
Injection volume: 5 µL
Column temperature: 40°C
Analysis time: 22 minutes
Retention time: Pladienolide H13: 5.5 min
   Pladienolide C3: 8.3 min
   Pladienolide D: 9.0 min
   Pladienolide C2: 11.1 min

**Table 7**

| mg/L | Mer-11107-ZX::hyg2:: PapsmAB strain | Mer-11107-ZX::hyg2:: PampsmAB-CLM strain | Mer-11107-ZX::hyg2:: PampsmAB-CLLM strain | Mer-11107-ZX::hyg2:: PampsmAB-CLFM strain | Mer-11107-ZX::hyg2:: PampsmAB-TY strain |
|---|---|---|---|---|---|
| Pladienolide D | 1315.5 | 1412.8 | 1372.1 | 1371.3 | 755.6 |
| Pladienolide H13 | 163.3 | 28.2 | 24.3 | 16.3 | 43.9 |
| Pladienolide C3 | 93.8 | 17.5 | 50.4 | 28.5 | 10.6 |
| Pladienolide C2 | 180.2 | 45.4 | 28.6 | 68.7 | 45.6 |
| Pladienolide D degradation products (H13 + C3 + C2) | 437.3 | 91.1 | 103.3 | 113.4 | 100.1 |

As a result, large amounts of the pladienolide degradation products pladienolide H13, pladienolide C2 and pladienolide C3 were produced together with pladienolide D by Mer-11107-Zx:hyg2::PapsmAB having introduced PapsmAB, while with Mer-11107-ZX::hyg2::PampsmAB-CLM, Mer-11107-ZX::hyg2::PampsmAB-CLLM, Mer-11107-ZX::hyg2::PampsmAB-CLFM and Mer-11107-ZX::hyg2::PampsmAB-TY introduced with PampsmAB-CLM, PampsmAB-CLLM, PampsmAB-CLFM and PampsmAB-TY, pladienolide D was produced but production of the pladienolide degradation products pladienolide H13, pladienolide C2 and pladienolide C3 was reduced to approximately one quarter. These results suggested that by introducing variants of the pladienolide B 16-position hydroxylating enzyme gene psmAB into the pladienolide B-producing strain Mer-11107 it is possible to achieve direct production of pladienolide D while reducing production of pladienolide D degradation products.

## Claims

1. A recombinant microorganism, which is a microorganism having ability to produce 16-position hydroxyl macrolide compounds represented by the formula (I): 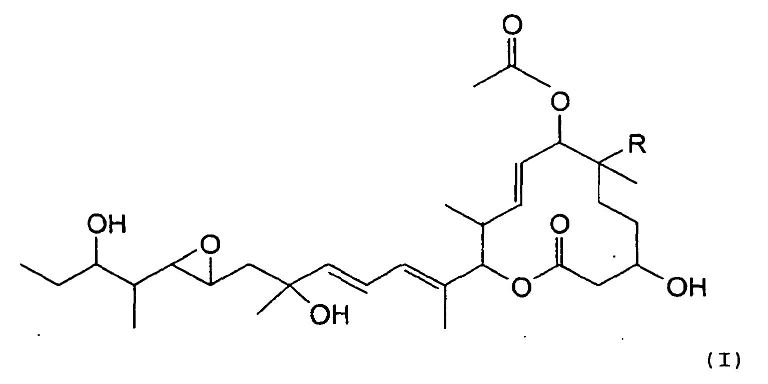 (wherein R represents a hydrogen atom or hydroxyl group),
and, which comprises:
(a) DNA encoding wholly or partially a polypeptide participating in biosynthesis of the macrolide compounds represented by the formula (II): 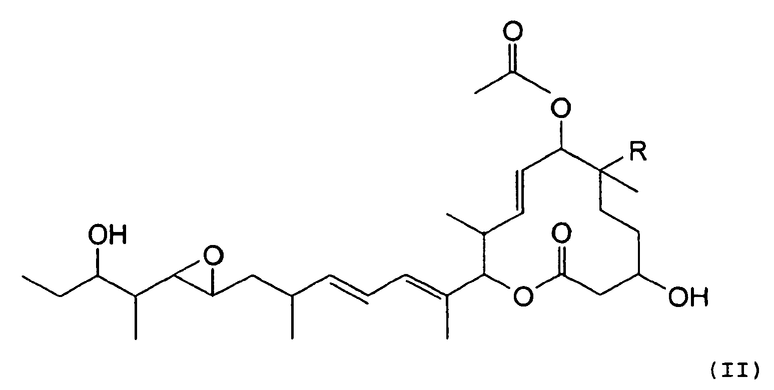 (wherein R represents a hydrogen atom or hydroxyl group),
wherein the DNA described in the above (a) is DNA encoding wholly or partially a polypeptide having polyketide synthetase activity, a polypeptide-having 7-position acetylase activity, a polypeptide having 18,19-position epoxidase activity and a polypeptide having transcriptional regulator factor activity, comprising:
(a-1) the continuous nucleotide sequence of nucleotides 8340 to 27935 in the Sequence No. 1;
(a-2) the continuous nucleotide sequence of nucleotides 28021 to 49098 in the Sequence No. 1;
(a-3) the continuous nucleotide sequence of nucleotides 49134 to 60269 in the Sequence No.1;
(a-4) the continuous nucleotide sequence of nucleotides 60269 to 65692 in the Sequence No. 1;
(a-5) the continuous nucleotide sequence of nucleotides 68160 to 66970 in the Sequence No. 1;
(a-6) the continuous nucleotide sequence of nucleotides 69568 to 68270 in the Sequence No. 1; and
(a-7) the continuous nucleotide sequence of nucleotides 72725 to 70020 in the Sequence No. 1; and
(b) DNA encoding wholly or partially a polypeptide having 16-position hydroxylating enzyme activity with respect to the macrolide compounds represented by the above formula (II), wherein the DNA is selected from the group consisting of:
(b-1) the continuous nucleotide sequence of nucleotides 1322 to 2548 in the Sequence No. 2;
(b-2) the continuous nucleotide sequence of nucleotides 420 to 1604 in the Sequence No. 3; and
(b-3) the continuous nucleotide sequence of nucleotides 172 to 1383 in the Sequence No. 4, or a variant thereof.

2. The recombinant microorganism according to Claim 1, wherein the DNA described under (a) above further includes DNA encoding wholly or partially a polypeptide having 6-position hydroxylating enzyme activity.

3. The recombinant microorganism according to Claim 2, wherein the DNA encoding wholly or partially a polypeptide having 6-position hydroxylating enzyme activity is DNA comprising the continuous nucleotide sequence of nucleotides 65707 to 66903 in the Sequence No. 1.

4. The recombinant microorganism according to any one of Claims 1 to 3, wherein the DNA represented by (b) above is a DNA comprising the continuous nucleotide sequence of nucleotides 1322 to 2548 in the Sequence No. 2 which has 1 or 2 or more modified sites selected from the group consisting of 1) to 6) below:
1) a modified site wherein the sequence cgc of nucleotides 1592 to 1594 is modified to a codon encoding an amino acid other than arginine;
2) a modified site wherein the sequence atg of nucleotides 1655 to 1657 is modified to a codon encoding an amino acid other than methionine;
3) a modified site wherein the sequence cgc of nucleotides 1904 to 1906 is modified to a codon encoding an amino acid other than arginine;
4) a modified site wherein the sequence cgc of nucleotides 2027 to 2029 is modified to a codon encoding an amino acid other than arginine;
5) a modified site wherein the sequence atc of nucleotides 2054 to 2056 is modified to a codon encoding an amino acid other than isoleucine; and
6) a modified site wherein the sequence ctg of nucleotides 2528 to 2530 is modified to a codon encoding an amino acid other than leucine.

5. The recombinant microorganism according to any one of Claims 1 to 3, wherein the DNA represented by (b) above is a DNA comprising the continuous nucleotide sequence of nucleotides 1322 to 2548 in the Sequence No. 2 which has 1 or 2 or more modified sites selected from the group consisting of 1) to 6) below:
1) a modified site wherein the sequence cgc of nucleotides 1592 to 1594 is modified to a codon encoding cysteine, methionine or proline;
2) a modified site wherein the sequence atg of nucleotides 1655 to 1657 is modified to a codon encoding threonine or serine;
3) a modified site wherein the sequence cgc of nucleotides 1904 to 1906 is modified to a codon encoding leucine, isoleucine, proline, tyrosine or phenylalanine;
4) a modified site wherein the sequence cgc of nucleotides 2027 to 2029 is modified to a codon encoding leucine, isoleucine or proline;
5) a modified site wherein the sequence atc of nucleotides 2054 to 2056 is modified to a codon encoding phenylalanine or valine; and
6) a modified site wherein the sequence ctg of nucleotides 2528 to 2530 is modified to a codon encoding methionine, cysteine or isoleucine.

6. The recombinant microorganism according to any one of Claims 1 to 3, wherein the DNA represented by (b) above is a DNA comprising the continuous nucleotide sequence of nucleotides 1322 to 2548 in the Sequence No. 2 which has 1 or 2 or more modified sites selected from the group consisting of 1) to 6) below:
1) a modified site wherein the sequence cgc of nucleotides 1592 to 1594 is modified to a codon encoding cysteine;
2) a modified site wherein the sequence atg of nucleotides 1655 to 1657 is modified to a codon encoding threonine;
3) a modified site wherein the sequence cgc of nucleotides 1904 to 1906 is modified to a codon encoding leucine or tyrosine;
4) a modified site wherein the sequence cgc of nucleotides 2027 to 2029 is modified to a codon encoding leucine;
5) a modified site wherein the sequence atc of nucleotides 2054 to 2056 is modified to a codon encoding phenylalanine; and
6) a modified site wherein the sequence ctg of nucleotides 2528 to 2530 is modified to a codon encoding methionine.

7. The recombinant microorganism according to Claim 1, comprising a host having the DNA described under (a) above into which has been incorporated the DNA described under (b) above derived from a microorganism of a different species.

8. The recombinant microorganism according to Claim 1, comprising a host having the DNA described under (b) above into which has been incorporated the DNA described under (a) above derived from a different species.

9. The recombinant microorganism according to Claim 1, comprising a host having none of the DNA described under (a) and (b) above into which has been incorporated the DNA described under both (a) and (b) above derived from different species.

10. The recombinant microorganism according to any of Claims 1 to 9, wherein the recombinant microorganism having the ability to produce the 16-position hydroxyl macrolide compound represented by the formula (I) is a strain belonging to Streptomyces genus.

11. A method for producing 16-position hydroxyl macrolide compounds represented by the formula (I) or pharmacologically acceptable salts thereof, or hydrates of them, wherein the recombinant microorganism according to any of Claims 1 to 10 is cultured in nutritious medium, and the 16-position hydroxyl macrolide compounds represented by the formula (I) are collected from the culture broth thereof.

12. The method according to Claim 11, wherein a cyclodextrin is present in the culture broth.

13. The method according to Claim 12, wherein the cyclodextrin is a cyclodextrin selected from the group consisting of β-cyclodextrin, -cyclodextrin, partially methylated β-cyclodextrin, dimethyl-β-cyclodextrin, trimethyl-β-cyclodextrin, glycosyl-β-cyclodextrin and hydroxypropyl-β-cyclodextrin.

## Patentansprüche

1. Rekombinanter Mikroorganismus, welcher ein Mikroorganismus mit der Fähigkeit ist, Position-16-Hydroxyl-Makrolidverbindungen herzustellen, die durch die Formel (I) dargestellt werden: 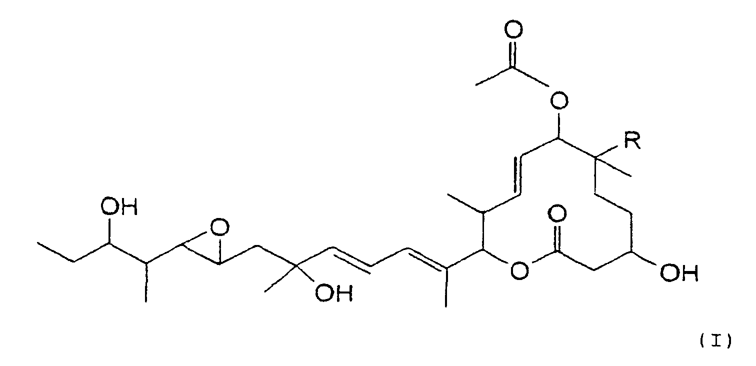 (wobei R ein Wasserstoffatom oder eine Hydroxylgruppe darstellt),
und welcher umfasst:
(a) DNA, die ganz oder partiell ein Polypeptid kodiert, das an der Biosynthese der Makrolidverbindungen beteiligt ist, die durch die Formel (II) dargestellt werden: 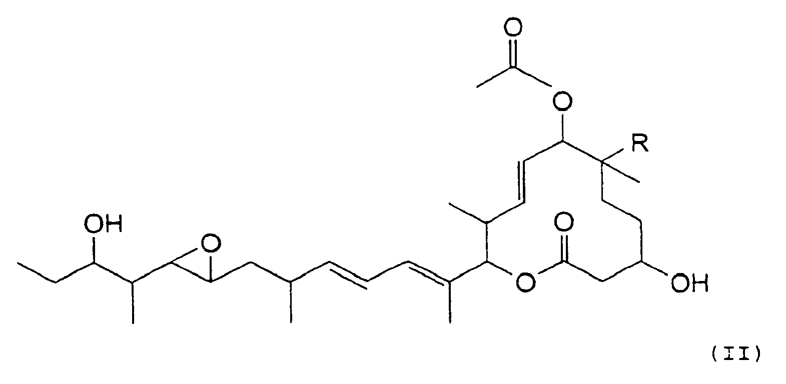 (wobei R ein Wasserstoffatom oder eine Hydroxylgruppe darstellt),
wobei die oben in (a) beschriebene DNA DNA ist, die ganz oder partiell ein Polypeptid mit Polyketid-Synthetaseaktivität, ein Polypeptid mit Position-7-Acetylaseaktivität, ein Polypeptid mit Position-18,19-Epoxidaseaktivität und ein Polypeptid mit Aktivität eines transkriptionell regulatorischen Faktors kodiert, die umfasst:
(a-1) die durchgehende Nukleotidsequenz der Nukleotide 8340 bis 27935 in der Sequenz Nr. 1;
(a-2) die durchgehende Nukleotidsequenz der Nukleotide 28021 bis 49098 in der Sequenz Nr. 1;
(a-3) die durchgehende Nukleotidsequenz der Nukleotide 49134 bis 60269 in der Sequenz Nr. 1;
(a-4) die durchgehende Nukleotidsequenz der Nukleotide 60269 bis 65692 in der Sequenz Nr. 1;
(a-5) die durchgehende Nukleotidsequenz der Nukleotide 68160 bis 66970 in der Sequenz Nr. 1;
(a-6) die durchgehende Nukleotidsequenz der Nukleotide 69568 bis 68270 in der Sequenz Nr. 1; und
(a-7) die durchgehende Nukleotidsequenz der Nukleotide 72725 bis 70020 in der Sequenz Nr. 1; und
(b) DNA, die ganz oder partiell ein Polypeptid kodiert mit Position-16-hydroxylierender Enzymaktivität in Bezug auf die in der obigen Formel (II) dargestellten Makrolidverbindungen, wobei die DNA ausgewählt ist aus der Gruppe bestehend aus:
(b-1) die durchgehende Nukleotidsequenz der Nukleotide 1322 bis 2548 in der Sequenz Nr. 2;
(b-2) die durchgehende Nukleotidsequenz der Nukleotide 420 bis 1604 in der Sequenz Nr. 3; und
(b-3) die durchgehende Nukleotidsequenz der Nukleotide 172 bis 1383 in der Sequenz Nr. 4, oder eine Variante davon.

2. Rekombinanter Mikroorganismus gemäß Anspruch 1, wobei die oben unter (a) beschriebene DNA ferner DNA beinhaltet, die ganz oder partiell ein Polypeptid mit Position-6-hydroxylierender Enzymaktivität kodiert.

3. Rekombinanter Mikroorganismus gemäß Anspruch 2, wobei die DNA, die ganz oder partiell ein Polypeptid mit Position-6-hydroxylierender Enzymaktivität kodiert, DNA ist, die die durchgehende Nukleotidsequenz der Nukleotide 65707 bis 66903 in der Sequenz Nr. 1 umfasst.

4. Rekombinanter Mikroorganismus gemäß einem der Ansprüche 1 bis 3, wobei die oben von (b) dargestellte DNA eine DNA ist, die die durchgehende Nukleotidsequenz der Nukleotide 1322 bis 2548 in der Sequenz Nr. 2 umfasst, die 1 oder 2 oder mehr modifizierte Stellen hat, die ausgewählt sind aus der Gruppe bestehend aus 1) bis 6) wie unten genannt:
1) eine modifizierte Stelle, wobei die Sequenz cgc der Nukleotide 1592 bis 1594 zu einem Kodon modifiziert ist, das eine andere Aminosäure als Arginin kodiert;
2) eine modifizierte Stelle, wobei die Sequenz atg der Nukleotide 1655 bis 1657 zu einem Kodon modifiziert ist, das eine andere Aminosäure als Methionin kodiert;
3) eine modifizierte Stelle, wobei die Sequenz cgc der Nukleotide 1904 bis 1906 zu einem Kodon modifiziert ist, das eine andere Aminosäure als Arginin kodiert;
4) eine modifizierte Stelle, wobei die Sequenz cgc der Nukleotide 2027 bis 2029 zu einem Kodon modifiziert ist, das eine andere Aminosäure als Arginin kodiert;
5) eine modifizierte Stelle, wobei die Sequenz atc der Nukleotide 2054 bis 2056 zu einem Kodon modifiziert ist, das eine andere Aminosäure als Isoleucin kodiert; und
6) eine modifizierte Stelle, wobei die Sequenz ctg der Nukleotide 2528 bis 2530 zu einem Kodon modifiziert ist, das eine andere Aminosäure als Leucin kodiert.

5. Rekombinanter Mikroorganismus gemäß einem der Ansprüche 1 bis 3, wobei die oben von (b) dargestellte DNA eine DNA ist, die die durchgehende Nukleotidsequenz der Nukleotide 1322 bis 2548 in der Sequenz Nr. 2 umfasst, die 1 oder 2 oder mehr modifizierte Stellen hat, die ausgewählt sind aus der Gruppe bestehend aus 1) bis 6) wie unten genannt:
1) eine modifizierte Stelle, wobei die Sequenz cgc der Nukleotide 1592 bis 1594 zu einem Kodon modifiziert ist, das Cystein, Methionin oder Prolin kodiert;
2) eine modifizierte Stelle, wobei die Sequenz atg der Nukleotide 1655 bis 1657 zu einem Kodon modifiziert ist, das Threonin oder Serin kodiert;
3) eine modifizierte Stelle, wobei die Sequenz cgc der Nukleotide 1904 bis 1906 zu einem Kodon modifiziert ist, das Leucin, Isoleucin, Prolin, Tyrosin oder Phenylalanin kodiert;
4) eine modifizierte Stelle, wobei die Sequenz cgc der Nukleotide 2027 bis 2029 zu einem Kodon modifiziert ist, das Leucin, Isoleucin oder Prolin kodiert;
5) eine modifizierte Stelle, wobei die Sequenz atc der Nukleotide 2054 bis 2056 zu einem Kodon modifiziert ist, das Phenylalanin oder Valin kodiert; und
6) eine modifizierte Stelle, wobei die Sequenz ctg der Nukleotide 2528 bis 2530 zu einem Kodon modifiziert ist, das Methionin, Cystein oder Isoleucin kodiert.

6. Rekombinanter Mikroorganismus gemäß einem der Ansprüche 1 bis 3, wobei die oben durch (b) dargestellte DNA eine DNA ist, die die durchgehende Nukleotidsequenz der Nukleotide 1322 bis 2548 in der Sequenz Nr. 2 umfasst, die eine oder zwei oder mehr modifizierte Stellen hat, die ausgewählt sind aus der Gruppe bestehend aus 1) bis 6) wie unten genannt:
1) eine modifizierte Stelle, wobei die Sequenz cgc der Nukleotide 1592 bis 1594 zu einem Kodon modifiziert ist, das Cystein kodiert;
2) eine modifizierte Stelle, wobei die Sequenz atg der Nukleotide 1655 bis 1657 zu einem Kodon modifiziert ist, das Threonin kodiert;
3) eine modifizierte Stelle, wobei die Sequenz cgc der Nukleotide 1904 bis 1906 zu einem Kodon modifiziert ist, das Leucin oder Tyrosin kodiert;
4) eine modifizierte Stelle, wobei die Sequenz cgc der Nukleotide 2027 bis 2029 zu einem Kodon modifiziert ist, das Leucin kodiert;
5) eine modifizierte Stelle, wobei die Sequenz atc der Nukleotide 2054 bis 2056 zu einem Kodon modifiziert ist, das Phenylalanin kodiert; und
6) eine modifizierte Stelle, wobei die Sequenz ctg der Nukleotide 2528 bis 2830 zu einem Kodon modifiziert ist, das Methionin kodiert.

7. Rekombinanter Mikroorganismus gemäß Anspruch 1, der einen Wirt umfasst mit der oben unter (a) beschriebenen DNA, wohinein die oben unter (b) beschriebene DNA inkorporiert wurde, die aus einem Mikroorganismus einer anderen Art abgeleitet wurde.

8. Rekombinanter Mikroorganismus gemäß Anspruch 1, der einen Wirt umfasst mit der oben unter (b) beschriebenen DNA, wohinein die oben unter (a) beschriebene DNA inkorporiert wurde, die von einer anderen Art abgeleitet wurde.

9. Rekombinanter Mikroorganismus gemäß Anspruch 1, der einen Wirt umfasst, der keine der unter (a) und (b) oben beschriebenen DNAs hat, und in den die oben unter sowohl (a) als auch (b) beschriebene DNA inkorporiert wurde, die von unterschiedlichen Arten abgeleitet ist.

10. Rekombinanter Mikroorganismus gemäß einem der Ansprüche 1 bis 9, wobei der rekombinanter Mikroorganismus, der die Fähigkeit hat, die von der Formel (I) dargestellte Position-16-Hydroxyl-Makrolidverbindung herzustellen, ein Stamm ist, der zu der Gattung Streptomyces gehört.

11. Verfahren zur Herstellung von Position-16-Hydroxyl-Makrolidverbindungen, die durch die Formel (I) dargestellt sind, oder pharmakologisch akzeptablen Salzen davon, oder Hydraten von diesen, wobei der rekombinante Mikroorganismus gemäß einem der Ansprüche 1 bis 10 in nährstoffreichem Medium kultiviert wird und die Position-16-Hydroxyl-Makrolidverbindungen, die durch die Formel (I) dargestellt werden, aus der Kulturbrühe davon gewonnen werden.

12. Verfahren gemäß Anspruch 11, wobei ein Cyclodextrin in der Kulturbrühe vorhanden ist.

13. Verfahren gemäß Anspruch 12, wobei das Cyclodextrin ein Cyclodextrin ist, das ausgewählt ist aus der Gruppe bestehend aus β-Cyclodextrin, -cyclodextrin, partiell methyliertes β-Cyclodextrin, Dimethyl-β-cyclodextrin, Trimethyl-β-cyclodextrin, Glycosyl-β-cyclodextrin und Hydroxypropyl-β-cyclodextrin.

## Revendications

1. Micro-organisme recombinant qui est un micro-organisme ayant la faculté de produire des composés macrolides hydroxylés en position 16 représentés par la formule (I) : 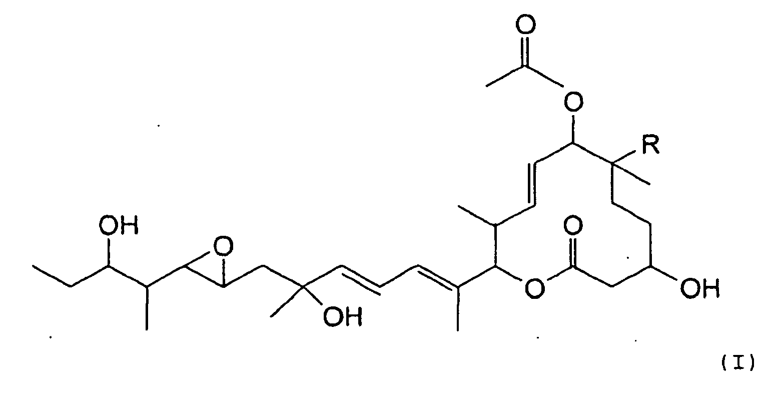 (où R représente un atome d'hydrogène ou un groupe hydroxyle),
et, qui comprend :
(a) de l'ADN codant totalement ou partiellement pour un polypeptide participant à la biosynthèse des composés macrolides représentés par la formule (II) : 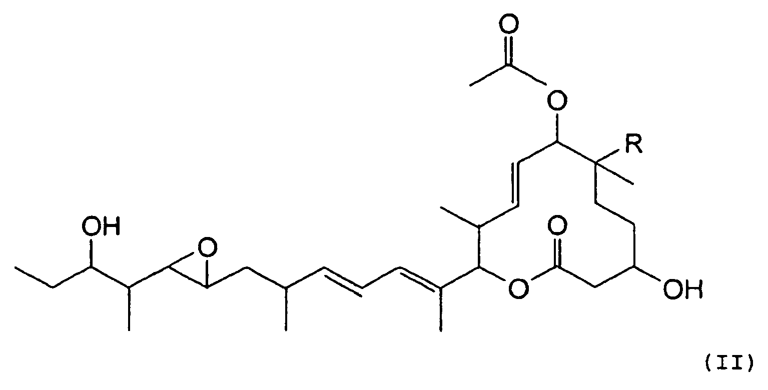 (où R représente un atome d'hydrogène ou un groupe hydroxyle),
où l'ADN décrit en (a) ci-dessus est un ADN codant totalement ou partiellement pour un polypeptide ayant une activité de polycétide synthétase, un polypeptide ayant une activité d'acétylase en position 7, un polypeptide ayant une activité d'époxydase en position 18, 19 et un polypeptide ayant une activité de facteur régulateur de transcription, comprenant :
(a-1) la séquence nucléotidique continue des nucléotides 8340 à 27935 dans la séquence No. 1;
(a-2) la séquence nucléotidique continue des nucléotides 28021 à 49098 dans la séquence No. 1;
(a-3) la séquence nucléotidique continue des nucléotides 49134 à 60269 dans la séquence No. 1;
(a-4) la séquence nucléotidique continue des nucléotides 60269 à 65692 dans la séquence No. 1;
(a-5) la séquence nucléotidique continue des nucléotides 68160 à 66970 dans la séquence No. 1;
(a-6) la séquence nucléotidique continue des nucléotides 69568 à 68270 dans la séquence No. 1; et
(a-7) la séquence nucléotidique continue des nucléotides 72725 à 70020 dans la séquence No. 1; et
(b) de l'ADN codant totalement ou partiellement pour un polypeptide ayant une activité enzymatique d'hydroxylation en position 16 par rapport aux composés macrolides représentés par la formule (II) ci-dessus, où l'ADN est choisi parmi le groupe consistant en :
(b-1) la séquence nucléotidique continue des nucléotides 1322 à 2548 dans la séquence No. 2;
(b-2) la séquence nucléotidique continue des nucléotides 420 à 1604 dans la séquence No. 3; et
(b-3) la séquence nucléotidique continue des nucléotides 172 à 1383 dans la séquence No. 4, ou une variante de celle-ci.

2. Micro-organisme recombinant selon la revendication 1, dans lequel l'ADN décrit en (a) ci-dessus inclut en outre de l'ADN codant totalement ou partiellement pour un polypeptide ayant une activité enzymatique d'hydroxylation en position 6.

3. Micro-organisme recombinant selon la revendication 2, dans lequel l'ADN codant totalement ou partiellement pour un polypeptide ayant une activité enzymatique d'hydroxylation en position 6 est de l'ADN comprenant la séquence nucléotidique continue des nucléotides 65707 à 66903 dans la séquence No. 1.

4. Micro-organisme recombinant selon l'une quelconque des revendications 1 à 3, dans lequel l'ADN représenté par (b) ci-dessus est un ADN comprenant la séquence nucléotidique continue des nucléotides 1322 à 2548 dans la séquence No. 2 qui possède 1 ou 2 ou plusieurs sites modifiés choisis parmi le groupe consistant en 1) à 6) ci-dessous :
1) un site modifié dans lequel la séquence cgc des nucléotides 1592 à 1594 est modifiée en un codon codant pour un acide aminé autre que l'arginine ;
2) un site modifié dans lequel la séquence atg des nucléotides 1655 à 1657 est modifiée en un codon codant pour un acide aminé autre que la méthionine ;
3) un site modifié dans lequel la séquence cgc des nucléotides 1904 à 1906 est modifiée en un codon codant pour un acide aminé autre que l'arginine ;
4) un site modifié dans lequel la séquence cgc des nucléotides 2027 à 2029 est modifiée en un codon codant pour un acide aminé autre que l'arginine ;
5) un site modifié dans lequel la séquence atc des nucléotides 2054 à 2056 est modifiée en un codon codant pour un acide aminé autre que l'isoleucine ; et
6) un site modifié dans lequel la séquence ctg des nucléotides 2528 à 2530 est modifiée en un codon codant pour un acide aminé autre que la leucine.

5. Micro-organisme recombinant selon l'une quelconque des revendications 1 à 3, dans lequel l'ADN représenté par (b) ci-dessus est un ADN comprenant la séquence nucléotidique continue des nucléotides 1322 à 2548 dans la séquence No. 2 qui possède 1 ou 2 ou plusieurs sites modifiés choisis parmi le groupe consistant en 1) à 6) ci-dessous :
1) un site modifié dans lequel la séquence cgc des nucléotides 1592 à 1594 est modifiée en un codon codant pour la cystéine, la méthionine ou la proline ;
2) un site modifié dans lequel la séquence atg des nucléotides 1655 à 1657 est modifiée en un codon codant pour la thréonine ou la sérine ;
3) un site modifié dans lequel la séquence cgc des nucléotides 1904 à 1906 est modifiée en un codon codant pour la leucine, l'isoleucine, la proline, la tyrosine ou la phénylalanine ;
4) un site modifié dans lequel la séquence cgc des nucléotides 2027 à 2029 est modifiée en un codon codant pour la leucine, l'isoleucine ou la proline ;
5) un site modifié dans lequel la séquence atc des nucléotides 2054 à 2056 est modifiée en un codon codant pour la phénylalanine ou la valine ; et
6) un site modifié dans lequel la séquence ctg des nucléotides 2528 à 2530 est modifiée en un codon codant pour la méthionine, la cystéine ou l'isoleucine.

6. Micro-organisme recombinant selon l'une quelconque des revendications 1 à 3, dans lequel l'ADN représenté par (b) ci-dessus est un ADN comprenant la séquence nucléotidique continue des nucléotides 1322 à 2548 dans la séquence No. 2 qui possède 1 ou 2 ou plusieurs sites modifiés choisis parmi le groupe consistant en 1) à 6) ci-dessous :
1) un site modifié dans lequel la séquence cgc des nucléotides 1592 à 1594 est modifiée en un codon codant pour la cystéine ;
2) un site modifié dans lequel la séquence atg des nucléotides 1655 à 1657 est modifiée en un codon codant pour la thréonine ;
3) un site modifié dans lequel la séquence cgc des nucléotides 1904 à 1906 est modifiée en un codon codant pour la leucine ou la tyrosine ;
4) un site modifié dans lequel la séquence cgc des nucléotides 2027 à 2029 est modifiée en un codon codant pour la leucine ;
5) un site modifié dans lequel la séquence atc des nucléotides 2054 à 2056 est modifiée en un codon codant pour la phénylalanine ; et
6) un site modifié dans lequel la séquence ctg des nucléotides 2528 à 2530 est modifiée en un codon codant pour la méthionine.

7. Micro-organisme recombinant selon la revendication 1, comprenant un hôte ayant l'ADN décrit en (a) ci-dessus dans lequel a été incorporé l'ADN décrit en (b) ci-dessus dérivé d'un micro-organisme d'une espèce différente.

8. Micro-organisme recombinant selon la revendication 1, comprenant un hôte ayant l'ADN décrit en (b) ci-dessus dans lequel a été incorporé l'ADN décrit en (a) ci-dessus dérivé d'une espèce différente.

9. Micro-organisme recombinant selon la revendication 1, comprenant un hôte n'ayant pas l'ADN décrit en (a) et (b) ci-dessus dans lequel a été incorporé l'ADN décrit en à la fois (a) et (b) ci-dessus dérivé d'espèces différentes.

10. Micro-organisme recombinant selon l'une quelconque des revendications 1 à 9, lequel micro-organisme recombinant ayant la faculté de produire le composé macrolide hydroxylé en position 16 représenté par la formule (I) est une souche appartenant au genre *Streptomyces.*

11. Procédé pour produire des composés macrolides hydroxylés en position 16 représentés par la formule (I) ou des sels pharmacologiquement acceptables de ces derniers, ou des hydrates de ces derniers, dans lequel le micro-organisme recombinant selon l'une quelconque des revendications 1 à 10 est mis en culture dans un milieu nutritif, et les composés macrolides hydroxylés en position 16 représentés par la formule (I) sont recueillis à partir du bouillon de culture de ce dernier.

12. Procédé selon la revendication 11, dans lequel une cyclodextrine est présente dans le bouillon de culture.

13. Procédé selon la revendication 12, dans lequel la cyclodextrine est une cyclodextrine choisie parmi le groupe consistant en β-cyclodextrine, -cyclodextrine, β-cyclodextrine partiellement méthylée, diméthyl-β-cyclodextrine, triméthyl-β-cyclodextrine, glycosyl-β-cyclodextrine et hydroxypropyl-β-cyclodextrine.


### SEQUENCE LISTING

<110> Eisai R&D MANAGEMENT C0. LTD.
<110> Mercian Corporation
<120> Recombinant microorganisms and methods for producing macrolide compounds using the microorganisms
<130> 06011PCT
<150> 2005-154114
   <151> 2005-05-26
<160> 56
<210> 1
   <211> 74342
   <212> DNA
   <213> Streptomyces sp.
<400> 1
<210> 2
   <211> 3793
   <212> DNA
   <213> Streptomyces sp.
<220>
   <221> CDS
   <222> (1322) . . (2548)
<220>
   <221> CDS
   <222> (2564).. (2761)
<400> 2
   ctgcagctcg acgtgcgggt cggacttcac gttgaagtac cagaccggat gcttgggcgc 60
   accgcccagc gaggcgaccg ccgcgtaact cccctcgtgc tcgacccgca tcagcggcgt 120
   cttgcggatc tttccgctgc gcgcgccccg ggtggtgagc acgatgaccg gcagcccggt    180
   gtcccgcagc gtggtgccct tggtgccccc ggaactctcg tacagctcga cctgctcgcg    240
   cacccactgc gtcgggctgg gctcgtactc gccctcaagt ggcaagggat ccgtctcctt    300
   cgtcggtccg gcggatggtg ctccggacgg tcccaactcc cgcggccgcc cggatcatcc    360
   gtaccgcatg ccccttcgcc cgagcgggtg atcaccgttc cggccatccg gtcgtccgca    420
   ccgcgagcac caggatcacg gcgctggaga gcagggccgt gaccagccgc ccccggtggc    480
   ccgtcagggc gcgacccagc agcgcgcccc cgcccgccag cagtagctgc cagctcgcgg    540
   acgcggcgaa ggccgccgcg gcgaacaccg cccgtttcag cggccgtgcc gcaccggcgg    600
   cgccgctgcc gagcaccagc gccacgaagt agaccaccgt catgggattg agcagggtga    660
   tcccgagaag gccgagataa gcccctgccg cgcctggaac cggccgttcc gggcgggtgg    720
   tgagccgatg ggcgcggtac tgccgcaggg cgagcagcgc cgcccgcagc gcgagcaccg    780
   cgaggaccag cgccgaggcc cagcgcagcg ggtccagcac cggccgcagc tgtgccgcga    840
   gggcggcgcc gcccacggtc gcgagcagcg cgtacagccc gtcggccgtg gcgacgccga    900
   gcgccgccga ggcgccggtg cgcagcgagg tgcgggcggt gagggagacc agataggtcc    960
   cgaccgcgcc gacgggcacc gcgatgccgt acccggcgag caggcccgcg agcagcgcgc   1020
   ccgtcacggg cgtgcgggac tggttcctcc ggggacggcg gggctgctgt cggcccggca   1080
   ccgcgggggc ggtggcagcg ggcgtcggca ggagggaggc tgtaggaggc atgggccgat   1140
   cctggggccg ccgcgcccgc accggcaaat gaattacggc gcgttccagc ccccggccgg   1200
   ctcgctcttc ggccacttca ccgcgtacgg cgatctggcc gaacttgctg tcgccccata   1260
   ggtgcctcgg gcatctaatg aagatcggca cgacgcacct cttcgtctgc gaggtctttc   1320
   c atg acg gaa ctg acg gac atc acc ggc ccg ggg acc ccg gcc gaa        1366
     Met Thr Glu Leu Thr Asp Ile Thr Gly Pro Gly Thr Pro Ala Glu
     1               5                   10                  15
   ccc gtc gca ttc ccc cag gac cgc acc tgc ccc tac cac ccc ccc acc      1414
   Pro Val Ala Phe Pro Gln Asp Arg Thr Cys Pro Tyr His Pro Pro Thr
                   20                  25                  30
   gga tac ggc ccg ctg cgc gac ggg cgc agc ctg tcc cgc gtc acc ctc      1462
   Gly Tyr Gly Pro Leu Arg Asp Gly Arg Ser Leu Ser Arg Val Thr Leu
               35                  40                  45
   ttc gac ggc cgt gag gtc tgg atg gtc acg ggc cac gcc acc gcc cgc      1510
   Phe Asp Gly Arg Glu Val Trp Met Val Thr Gly His Ala Thr Ala Arg
           50                  55                  60
   gcg ctg ctc gcg gac ccc cgg ctg tcc acc gac cgc acc ctc ccg ggc      1558
   Ala Leu Leu Ala Asp Pro Arg Leu Ser Thr Asp Arg Thr Leu Pro Gly
       65                  70                  75
   ttc ccc gtg tcc acg gcc cgc ttc gcg gcc gtc cgc gac cgg cgg gtg      1606
   Phe Pro Val Ser Thr Ala Arg Phe Ala Ala Val Arg Asp Arg Arg Val
   80                  85                  90                  95
   gcg ctg ctc ggc gtg gac gac ccg gtc cac cag acc cag cgg cgg atg      1654
   Ala Leu Leu Gly Val Asp Asp Pro Val His Gln Thr Gln Arg Arg Met
                   100                 105                 110
   atg atc ccg tcg ttc acc ctc aag cgc gcg gcc ggg ctg cgg ccc acc      1702
   Met Ile Pro Ser Phe Thr Leu Lys Arg Ala Ala Gly Leu Arg Pro Thr
               115                 120                 125
   atc cag cgg acc gtc gac ggg ctg ctg gac gcg atg atc gag aag ggg      1750
   Ile Gln Arg Thr Val Asp Gly Leu Leu Asp Ala Met Ile Glu Lys Gly
           130                 135                 140
   ccg ccg gcc gag ctg gtc tcc gcc ttc gcc ctg ccc gtg ccc tcg atg      1798
   Pro Pro Ala Glu Leu Val Ser Ala Phe Ala Leu Pro Val Pro Ser Met
       145                 150                 155
   gtc atc tgc ggc ctg ctc ggc gtg ccg tac gcc gac cac gag ttc ttc      1846
   Val Ile Cys Gly Leu Leu Gly Val Pro Tyr Ala Asp His Glu Phe Phe
   160                 165                 170                 175
   gag gaa cag tcc cgc acg ctg ctg cgc ggt ccc acg gcc gcc gac tcg      1894
   Glu Glu Gln Ser Arg Thr Leu Leu Arg Gly Pro Thr Ala Ala Asp Ser
                   180                 185                 190
   caa ggg gcg cgc gag cgg ctc gag gag tac ctc ggc ggg ctg atc gac      1942
   Gln Gly Ala Arg Glu Arg Leu Glu Glu Tyr Leu Gly Gly Leu Ile Asp
               195                 200                 205
   gac aag gag cgg cag gcc gaa ccc ggc gac ggc gtc ctg gac gac ctc      1990
   Asp Lys Glu Arg Gln Ala Glu Pro Gly Asp Gly Val Leu Asp Asp Leu
           210                 215                 220
   gtc cac cag cgg ctg cgc acc ggc gag ctg gac cga cgc gac gtg gtg      2038
   Val His Gln Arg Leu Arg Thr Gly Glu Leu Asp Arg Arg Asp Val Val
       225                     230                     235
   gcg ctg gcc gtc atc ctg ctc gtg gcc ggg cac gag acg acc gcc aac      2086
   Ala Leu Ala Val Ile Leu Leu Val Ala Gly His Glu Thr Thr Ala Asn
   240                 245                     250                 255
   atg atc tcc ctc ggc acc tac acg ctg ctg cgg cac ccc ggc cgg ctg      2134
   Met Ile Ser Leu Gly Thr Tyr Thr Leu Leu Arg His Pro Gly Arg Leu
                   260                     265                 270
   gcc gag ctg cgc gcc gac ccg gcg ctg ctg ccc gcc gcc gtg gag gag      2182
   Ala Glu Leu Arg Ala Asp Pro Ala Leu Leu Pro Ala Ala Val Glu Glu
               275                     280                 285
   ctg atg cgg atg ctc tcg atc gcg gac ggg ctg ctg cgc ctg gcc ctg      2230
   Leu Met Arg Met Leu Ser Ile Ala Asp Gly Leu Leu Arg Leu Ala Leu
           290                     295                 300
   gag gac atc gag atc gcc ggc gcc acg atc cgg gcc ggc gag ggc gtc      2278
   Glu Asp Ile Glu Ile Ala Gly Ala Thr Ile Arg Ala Gly Glu Gly Val
       305                     310                     315
   ctg ttc tcc acc tcg ctg atc aac cgc gac gag tcc gtg ttc gac gac      2326
   Leu Phe Ser Thr Ser Leu Ile Asn Arg Asp Glu Ser Val Phe Asp Asp
   320                 325                     330                 335
   ccc gac acc ctg gac ttc cac cgc tcc acc cgc cac cac gtg gcc ttc      2374
   Pro Asp Thr Leu Asp Phe His Arg Ser Thr Arg His His Val Ala Phe
                   340                     345                 350
   ggt ttc ggc atc cac cag tgc ctg ggc cag aac cta gcc cgc gcc gag      2422
   Gly Phe Gly Ile His Gln Cys Leu Gly Gln Asn Leu Ala Arg Ala Glu
               355                     360                 365
   ctg gag atc gcc ctg ggc acg ctc ctg gag cgg ctc ccc ggc ctc cgg      2470
   Leu Glu Ile Ala Leu Gly Thr Leu Leu Glu Arg Leu Pro Gly Leu Arg
           370                     375                 380
   ctg gcc gcg ccc gcc gag gag atc ccg ttc aaa ccc ggc gac acg atc      2518
   Leu Ala Ala Pro Ala Glu Glu Ile Pro Phe Lys Pro Gly Asp Thr Ile
       385                     390                     395
   cag ggg atg ctg gaa ctc ccc gtg acc tgg taa gaggctctgg tc atg cac    2569
   Gln Gly Met Leu Glu Leu Pro Val Thr Trp                   Met His
   400                 405                                   410
   atc gac atc gac aag gac cgc tgc atc ggc gcc ggc cag tgc gcg ctg      2617
   Ile Asp Ile Asp Lys Asp Arg Cys Ile Gly Ala Gly Gln Cys Ala Leu
               415                     420                 425
   gcc gcc ccg ggc gtg ttc acc cag gac gac gac ggc tac agc acc ctg      2665
   Ala Ala Pro Gly Val Phe Thr Gln Asp Asp Asp Gly Tyr Ser Thr Leu
           430                     435                 440
   ctc ccc ggc cgc gag gac ggc ggg ggc gac ccg atg gtc cgg gag gcg      2713
   Leu Pro Gly Arg Glu Asp Gly Gly Gly Asp Pro Met Val Arg Glu Ala
       445                     450                     455
   gcc cgc gcc tgc ccg gtg agc gcc atc cgg gtg acc gaa ccg gcc ggc      2761
   Ala Arg Ala Cys Pro Val Ser Ala Ile Arg Val Thr Glu Pro Ala Gly
   460                 465                     470                 475
   tga ggcggggccc ggcggccgcg gcccgctgcc gggaccgccg ttcccagttc agtagg    2820
   gtcgtgcgat gacctcacag gccgggaagc ccttcctcta cgtcgtcgtc tgcgcggccg    2880
   ggaccgccgc cggagtcacc acgctgatcg gcgccgccca ggcgcggggc tgggaggtgg    2940
   gggtcctggc cacgccggtg gcgatgggcg ggttcttcga cacggctgcg gtcgaggaga    3000
   tgacgggccg gcccatccgc tcggcctggc gctcgccggc cgatccgcgc ccgttcccgc    3060
   cgccgggcgc cgtggtggtg gcgcccgcca ccttcaacac cgtcaacaag tgggcggccg    3120
   gtctcgccga cacgctcgcc gtcggcacgc tctgcgaggc ggcgggcctc ggcgtgccga    3180
   tcgccgtcct gccctgcgtg gcggacgcgc tggccgccca ccccgcgtac cgggagggcc    3240
   ttctccggct gcgtgggatg ggcgtccgct tcggcgagcc gtacgccggc ccgccggggg    3300
   aggacggcga ggcggacggc gcacggcccg ggttcgcctg ggagaacgcc ctggacctgc    3360
   tggagcgggc ctgaacccgc tccccgaccc gtagggcctg tctgacactg tcagacaggc    3420
   cctaacggca ggtcagcgcc ggcccggcca gcatgccgcc ggtgtagagg tcctggcccc    3480
   gcggcagcca gtagcccagc ctggagacca ccgtggagca gtcaggcccg acggtgacgc    3540
   ggaccttcac cgtctcggga cggccgggct gcagcgcggt cagcgcgcag tccagggagt    3600
   acgcgagccg ggtcttcgag gtaccggccg accagcgggt tgacgcagcg ggcgtcgtcc    3660
   gtggcgatcc gcaccccggt gcccgccccg ccgatgagtc cgagccgggc gctgccgtcg    3720
   ccctcgtcgc tgtcccggcg gaccgtgtag gtcagcgtgg tggtggcgtc gcgccgcagg    3780
   gtgtccggtc gac                                                        3793
<210> 3
   <211> 2329
   <212> DNA
   <213> Streptomyces sp.
<220>
   <221> CDS
   <222> (420).. (1604)
<220>
   <221> CDS
   <222> (1643) . . (1834)
<400> 3
   ggatccacgg gtggccgccg cgctcgcccg ggtgaccgac cggcgtatcg gctatgtcgc       60
   cgcgctcttc gcggcgctgg gcttccccga gggcgaggcg cgggaccgcg gcctgctggc      120
   gtacaccgcc tacctcggcc acacccagct cggacatgcc gtccgacaga gcctgccggc      180
   cgaggcggca cacgaccgct atctggatgg cgtgatcgac accctcgtac ggccgcggga      240
   cggaggcgat gaagccgaac atgtcacaat ctgaacgagg ttggcggaac tgcgcgcaga      300
   acatgcccgg tatccgcggc atgaggtgag atcggcgcgg cgaaacacgg tgcgccacag      360
   cgttgccatc tcacacacga gcaactcgag ccacttgaga ctcgtacggg aggaaattc       419
   gtg acc gaa gcc atc ccc tac ttt cag aac cgc acc tgt ccc tac cac        467
   Val Thr Glu Ala Ile Pro Tyr Phe Gln Asn Arg Thr Cys Pro Tyr His
    1               5                  10                  15
   ccg ccc gcc gcc tat cag cca ctg cgc ggg gcc ggc ccg ctg agc cat        515
   Pro Pro Ala Ala Tyr Gln Pro Leu Arg Gly Ala Gly Pro Leu Ser His
               20                  25                  30
   gtc acg ttc tac gac ggc cgg aag gtg tgg gcg gtc acc ggc cac ccc        563
   Val Thr Phe Tyr Asp Gly Arg Lys Val Trp Ala Val Thr Gly His Pro
           35                  40                  45
   gag gca cgg gcg ctg ctg acc gac cag cga ctc tcc gcc gac cgg cag        611
   Glu Ala Arg Ala Leu Leu Thr Asp Gln Arg Leu Ser Ala Asp Arg Gln
       50                  55                  60
   aac ccg gcc ttc ccg gtc ccc ttc gaa cgc ttc gcg gcc atc cgc cgg        659
   Asn Pro Ala Phe Pro Val Pro Phe Glu Arg Phe Ala Ala Ile Arg Arg
   65                  70                  75                  80
   gtc cgg acg ccg ctg atc ggg gtc gac gac ccg gag cac aac acc cag        707
   Val Arg Thr Pro Leu Ile Gly Val Asp Asp Pro Glu His Asn Thr Gln
                   85                  90                  95
   cgc cgg atg ctg atc ccc agc ttc agc ctc aag cgg acc gcc gca ctg        755
   Arg Arg Met Leu Ile Pro Ser Phe Ser Leu Lys Arg Thr Ala Ala Leu
               100                 105                 110
   cgg ccg gag atc cag cgg atc gtc gac ggg ctg ctc gac cgg atg ctg        803
   Arg Pro Glu Ile Gln Arg Ile Val Asp Gly Leu Leu Asp Arg Met Leu
           115                 120                 125
   gat cag ggc ccg ccc acc gag ctg gtc tcc gcg ttc gcc ctg ccg gtc        851
   Asp Gln Gly Pro Pro Thr Glu Leu Val Ser Ala Phe Ala Leu Pro Val
       130                 135                 140
   ccg tcg atg gtg atc tgc gca ctg ctc gga gtc tca tac gcc gac cat        899
   Pro Ser Met Val Ile Cys Ala Leu Leu Gly Val Ser Tyr Ala Asp His
   145                 150                 155                 160
   gag ttc ttc gag gag gag tcc cgc cgc atc ctg cgc ggc cgg tcg gcc        947
   Glu Phe Phe Glu Glu Glu Ser Arg Arg Ile Leu Arg Gly Arg Ser Ala
                   165                 170                 175
   gag gag gcg gag gac gcc cgg ctg aag ctg gag gag tac ttc acc ggg        995
   Glu Glu Ala Glu Asp Ala Arg Leu Lys Leu Glu Glu Tyr Phe Thr Gly
               180                 185                 190
   ctg atc gcc gcc aag gag aag aac ccg ggc gac ggg ctg ctg gac gag       1043
   Leu Ile Ala Ala Lys Glu Lys Asn Pro Gly Asp Gly Leu Leu Asp Glu
           195                 200                 205
   ctg atc gag gac cgg ctg cgg acc ggc gcg ctc acc cgc gac gag ctg       1091
   Leu Ile Glu Asp Arg Leu Arg Thr Gly Ala Leu Thr Arg Asp Glu Leu
       210                 215                 220
   gtc cgg ctc gcc atg atc ctg ctg gtg gcc ggc cat gag acc acc gcc       1139
   Val Arg Leu Ala Met Ile Leu Leu Val Ala Gly His Glu Thr Thr Ala
   225                 230                 235                 240
   aac atg atc tcg ctc ggc acc ttc acc ctg ctg gac cac ccc gag cag       1187
   Asn Met Ile Ser Leu Gly Thr Phe Thr Leu Leu Asp His Pro Glu Gln
                   245                 250                 255
   ctg gcg cag ctc aag gcc gac gag ggc ctg atg ccg gcc gcc atc gag       1235
   Leu Ala Gln Leu Lys Ala Asp Glu Gly Leu Met Pro Ala Ala Ile Glu
               260                 265                 270
   gag ctg ctg cga ttc ctg tcc atc gcg gac ggc ctg ctg cgg gtg gcg       1283
   Glu Leu Leu Arg Phe Leu Ser Ile Ala Asp Gly Leu Leu Arg Val Ala
           275                 280                 285
   acg gag gac atc gag atc ggc ggt cag gtg atc cgg gcc gac gac gcg       1331
   Thr Glu Asp Ile Glu Ile Gly Gly Gln Val Ile Arg Ala Asp Asp Ala
       290                 295                 300
   gtc ctg ttc ccc gcc tca ctg atc aac cgg gac gag gcc gcc tat ccg       1379
   Val Leu Phe Pro Ala Ser Leu Ile Asn Arg Asp Glu Ala Ala Tyr Pro
   305                 310                 315                 320
   gca ccc gac gag ctg gac ctc ggc cgt tcg gcc cgc cat cac gtg gcg       1427
   Ala Pro Asp Glu Leu Asp Leu Gly Arg Ser Ala Arg His His Val Ala
                   325                 330                 335
   tcc ggc ttc ggg atc cac cag tgc ctg ggg cag aac ctc gcc cgc gcg       1475
   Ser Gly Phe Gly Ile His Gln Cys Leu Gly Gln Asn Leu Ala Arg Ala
               340                 345                 350
   gag atg gag atc gcg ctg cgc tca ctg ttc acc agg atc ccg cag ctg       1523
   Glu Met Glu Ile Ala Leu Arg Ser Leu Phe Thr Arg Ile Pro Gln Leu
           355                 360                 365
   cgg ctc gcc gtg ccg gcc gcc gag att ccg ttc aag gac gga gac acc       1571
   Arg Leu Ala Val Pro Ala Ala Glu Ile Pro Phe Lys Asp Gly Asp Thr
       370                 375                 380
   ctg caa ggc atg atc gaa ctg ccg ctg gcc tgg tag cagccaggac ggcaga     1623
   Leu Gln Gly Met Ile Glu Leu Pro Leu Ala Trp
   385                 390                 395
   ccaaagaaag gggtccgga atg cgg atc gcg atc gac acc gac cgc tgt atc     1675
                        Met Arg Ile Ala Ile Asp Thr Asp Arg Cys Ile
                                            400                 405
   ggc gcc ggc cag tgt gcc ctg acc gcg ccc ggg ggt ttc acc cag gat       1723
   Gly Ala Gly Gln Cys Ala Leu Thr Ala Pro Gly Gly Phe Thr Gln Asp
               410                 415                 420
   gac gac ggt ttc agt gca ctg ctg ccc ggc cgg gag gac ggc gcc ggc       1771
   Asp Asp Gly Phe Ser Ala Leu Leu Pro Gly Arg Glu Asp Gly Ala Gly
           425                 430                 435
   gac ccg ctg gtg cgg gaa gcc gcc cgc gcc tgc ccc gtg cag gcc att       1819
   Asp Pro Leu Val Arg Glu Ala Ala Arg Ala Cys Pro Val Gln Ala Ile
       440                 445                 450
   gcg gtc acc gac gat tag cagcaccccc gcggacgacc cggcagacgc gcgcggcc     1875
   Ala Val Thr Asp Asp
   455
   ccggctgaca cccggcgccc gaggcgcgcc cgagccgtcc gcccctccac ttgtccctac     1935
   ggcatccacc ccatccgcta ccgcaacacc ccttgggtga cgggcagttt cgaggacccc     1995
   ggtgtgcccg gggcgtactg gtgaccgtca ccggcttcac gccgcgattg cccacatagg     2055
   cgtcgtcgct cgcggcgatc acgaagcgcg gtcggtgccc cggctcgtaa cggtgcacga     2115
   tgcccggcag ttccacggtg aaccgccggg ccacatcggg cacccgggcc ggggccacca     2175
   acaggtgcac cagcgtcttc ctgccgttcg gcgcgacatc gtagagcttg gcgaacagca     2235
   ccagcttgtc cgccgcatcc gcggaccgct gcgcccgccc ggcctgcggc gaggcaacct     2295
   tcagcgtcac cctcggcgcg cccaccacgt cgac                                 2329
<210> 4
   <211> 1860
   <212> DNA
   <213> A-1560 strain
<220>
   <221> CDS
   <222> (172).. (1383)
<220>
   <221> CDS
   <222> (1399).. (1593)
<400> 4
   cggggatcgt acgccgtacc gtttcggggc aaccgaatta cgatgcggaa tggatggttc      60
   ccagccagat cccgcaggta gccgatctgg ccgaacttga tgtcgtgcac tggatgcctc     120
   gggcatctaa tgaagatcgg cacgacgcat ccttcgtctg cgaggtctcc c atg aca     177
                                                            Met Thr
                                                              1
   gac acg aca gac ctg acc gag ctg tca gat ccc gtc tcc ttc ccc cag      225
   Asp Thr Thr Asp Leu Thr Glu Leu Ser Asp Pro Val Ser Phe Pro Gln
             5                  10                  15
   gac cgg agc tgc ccc tac cac ccg ccc acc ggg tac gac ccg ctg cgc      273
   Asp Arg Ser Cys Pro Tyr His Pro Pro Thr Gly Tyr Asp Pro Leu Arg
        20                  25                  30
   acc gaa cgg ccg ccc gcc cgc atc cgg ctc tac gac ggc cgc ccc gcc      321
   Thr Glu Arg Pro Pro Ala Arg Ile Arg Leu Tyr Asp Gly Arg Pro Ala
    35                  40                  45                  50
   tgg ctc gtc acc ggc cac gcc gtc gcc cgt gac ctg ctg gtc gac ccc      369
   Trp Leu Val Thr Gly His Ala Val Ala Arg Asp Leu Leu Val Asp Pro
                    55                  60                  65
   cgc ctg tcc acg gac cgc acc cgc tcg ggc ttc ccg gcc aca act ccc      417
   Arg Leu Ser Thr Asp Arg Thr Arg Ser Gly Phe Pro Ala Thr Thr Pro
                70                  75                  80
   cgc ttc gcc gcg gtc cgc gac cgc aag ccg gcg ctc ctc ggc gtc gac      465
   Arg Phe Ala Ala Val Arg Asp Arg Lys Pro Ala Leu Leu Gly Val Asp
            85                  90                  95
   gac ccc aag cac cgc acc cag cgg tgg atg atg atc ccg agc ttc acc      513
   Asp Pro Lys His Arg Thr Gln Arg Trp Met Met Ile Pro Ser Phe Thr
       100                 105                 110
   ctc agg cgc gcc acc gag ctc agg ccg cgc atc cag gag atc gtc gac      561
   Leu Arg Arg Ala Thr Glu Leu Arg Pro Arg Ile Gln Glu Ile Val Asp
   115                 120                 125                 130
   gaa ctg ctg gac gtg atg atc gcc cag gga ccc ccg gcc gac ctg gtg      609
   Glu Leu Leu Asp Val Met Ile Ala Gln Gly Pro Pro Ala Asp Leu Val
                   135                 140                 145
   cgt tcc ttc gcg ctg ccg gtg ccg tcc atg gtg atc tgc gcc ctg ctc      657
   Arg Ser Phe Ala Leu Pro Val Pro Ser Met Val Ile Cys Ala Leu Leu
               150                 155                 160
   ggc gtg ccc tac gcc gac cac gag ttc ttc gag gac cag tcc agg cgg      705
   Gly Val Pro Tyr Ala Asp His Glu Phe Phe Glu Asp Gln Ser Arg Arg
           165                 170                 175
   ctg ctg cgc gga ccg gcg gcc gag gac acg cag gac gcc cgg gac cgg      753
   Leu Leu Arg Gly Pro Ala Ala Glu Asp Thr Gln Asp Ala Arg Asp Arg
       180                 185                 190
   ctc gcc gcg tac ctg gag gac ctg atc gac gag aag cgg cgc cgg ccc      801
   Leu Ala Ala Tyr Leu Glu Asp Leu Ile Asp Glu Lys Arg Arg Arg Pro
   195                 200                 205                 210
   ggt gac ggc ctg ctg gac gaa ctc gtc cag cag cgt ctg aac gaa ggc      849
   Gly Asp Gly Leu Leu Asp Glu Leu Val Gln Gln Arg Leu Asn Glu Gly
                   215                 220                 225
   gag ctc gac cgg gag gaa ctg acc gcg ctg gcg atg atc ctg ctg gtc      897
   Glu Leu Asp Arg Glu Glu Leu Thr Ala Leu Ala Met Ile Leu Leu Val
               230                 235                 240
   gcg ggc cac gag acc acc gcc aac atg atc tcc ctg ggc acc tac acg      945
   Ala Gly His Glu Thr Thr Ala Asn Met Ile Ser Leu Gly Thr Tyr Thr
           245                 250                 255
   ctc ctg ctg cac ccc gaa cgg ctg acc gag ctg cgc gcc gac ccc gcg      993
   Leu Leu Leu His Pro Glu Arg Leu Thr Glu Leu Arg Ala Asp Pro Ala
       260                 265                 270
   ctg ctg ccg gcc gcc gtc gag gaa ctg atg cgg atg ctg tcc atc gcg     1041
   Leu Leu Pro Ala Ala Val Glu Glu Leu Met Arg Met Leu Ser Ile Ala
   275                 280                 285                 290
   gac gga ctg ctg cgg cag gcc acc gag gac atc gag atc gcc ggg acc     1089
   Asp Gly Leu Leu Arg Gln Ala Thr Glu Asp Ile Glu Ile Ala Gly Thr
                   295                 300                 305
   acc atc agg gcc ggg gac ggc gtg gtc ttc tcc acc tct gtc atc aac     1137
   Thr Ile Arg Ala Gly Asp Gly Val Val Phe Ser Thr Ser Val Ile Asn
               310                 315                 320
   cgc gac gag gac gtc tac ccg gcc ccc gac acc ctc gac ttc cac cgc     1185
   Arg Asp Glu Asp Val Tyr Pro Ala Pro Asp Thr Leu Asp Phe His Arg
           325                 330                 335
   tcg acc cgc cac cac gtc gcc ttc ggt ttc gga atc cac cag tgc ctc     1233
   Ser Thr Arg His His Val Ala Phe Gly Phe Gly Ile His Gln Cys Leu
       340                 345                 350
   ggc cag aac ctc gcc cgc acc gaa ctg gag atc gcc ctg cgc acg ctc     1281
   Gly Gln Asn Leu Ala Arg Thr Glu Leu Glu Ile Ala Leu Arg Thr Leu
   355                 360                 365                 370
   ctc gaa cgg ctg ccc acg ctc cgg ctc gcc gcc cca ccg gag gaa atc     1329
   Leu Glu Arg Leu Pro Thr Leu Arg Leu Ala Ala Pro Pro Glu Glu Ile
                   375                 380                 385
   ccc ttc aaa ccc ggc gac acc atc cag ggg atg ctg gaa ctc ccc gtc     1377
   Pro Phe Lys Pro Gly Asp Thr Ile Gln Gly Met Leu Glu Leu Pro Val
               390                 395                 400
   agc tgg taa gaggctgccg tc atg cat atc gag atc gac aag gac cgc tgc   1428
   Ser Trp                   Met His Ile Glu Ile Asp Lys Asp Arg Cys
                             405                 410
   atc ggc gcc gga cag tgc gcc ctg acc gcc ccg ggt gtg ttc acc cag     1476
   Ile Gly Ala Gly Gln Cys Ala Leu Thr Ala Pro Gly Val Phe Thr Gln
                   420                 425                 430
   gac gac gac ggc ttc agt gac ctg ttg ccc ggc cgg gag gac ggc gcc     1524
   Asp Asp Asp Gly Phe Ser Asp Leu Leu Pro Gly Arg Glu Asp Gly Ala
   415             435                 440                 445
   ggc gac ccg atg gtc cgg gag gcc gcc agg gcc tgc ccc gtg agt gcc     1572
   Gly Asp Pro Met Val Arg Glu Ala Ala Arg Ala Cys Pro Val Ser Ala
               450                 455                 460
   atc acg ctg tcc gag gac ggg tag ggggccgagc cgcgccgccc gccggtccgc    1626
   Ile Thr Leu Ser Glu Asp Gly
           465
   tgccgcggcg ccgtgccgac gcggcggccg gccggcccgt ccggtgcccg tcgcgtcgcc   1686
   ccgtggcccc ggcggcggct gattgactag ggttcccggg tgagcgaaca ggcccagaag   1746
   ccctccgggg cgccgcccgc gaaagacacc gggacggcgc ccgggaaacc ccttcctcta   1806
   cgtcgtcgtc tgcgccgccg gcatcgccga aggcgtcagc aagctgatca ccgc         1860
<210> 5
   <211> 23
   <212> DNA
   <213> Artificial Sequence
<220>
   <223> STRANDNESS : single
<220>
   <223> TOPOLOGY : linear
<220>
   <223> Description of Artificial Sequence : KS-3F Primer
<400> 5
   gaccgcggct gggacgtgga ggg 23
<210> 6
   <211> 24
   <212> DNA
   <213> Artificial Sequence <220>

   <223> STRANDNESS : single <220>

   <223> TOPOLOGY : linear
<220>
   <223> Description of Artificial Sequence : KS-4R Primer
<400> 6
   gtgcccgatg ttggacttca acga 24
<210> 7
   <211> 27
   <212> DNA
   <213> Artificial Sequence
<220>
   <223> STRANDNESS : single
<220>
   <223> TOPOLOGY : linear
<220>
   <223> Description of Artificial Sequence : CB-1F Primer
<400> 7
   atgacagctt tgaatctgat ggatccc 27
<210> 8
   <211> 30
   <212> DNA
   <213> Artificial Sequence
<220>
   <223> STRANDNESS : single
<220>
   <223> TOPOLOGY : linear
<220>
   <223> Description of Artificial Sequence : CB-2R Primer
<400> 8
   tcagagacgg accggcagac tcttcagacg 30
<210> 9
   <211> 27
   <212> DNA
   <213> Artificial Sequence
<220>
   <223> STRANDNESS : single
<220>
   <223> TOPOLOGY : linear
<220>
   <223> Description of Artificial Sequence : PKC-1F Primer
<400> 9
   gtgcgccgta cccagcaggg aacgacc 27
<210> 10
   <211> 27
   <212> DNA
   <213> Artificial Sequence
<220>
   <223> STRANDNESS : single
<220>
   <223> TOPOLOGY : linear
<220>
   <223> Description of Artificial Sequence : PKC-2R Primer
<400> 10
   tcacgcgctc tccgcccgcc ccctgcc 27
<210> 11
   <211> 33
   <212> DNA
   <213> Artificial Sequence
<220>
   <223> STRANDNESS : single
<220>
   <223> TOPOLOGY : linear
<220>
   <223> Description of Artificial Sequence : PDL58-1F Primer
<400> 11
   gccccgcata tggatctgga aacccaactt ctc 33
<210> 12
   <211> 31
   <212> DNA
   <213> Artificial Sequence
<220>
   <223> STRANDNESS : single
<220>
   <223> TOPOLOGY : linear
<220>
   <223> Description of Artificial Sequence : PDL58-2R Primer
<400> 12
   gcactagtca gccgcgctcg acgaggaggt g 31
<210> 13
   <211> 33
   <212> DNA
   <213> Artificial Sequence
<220>
   <223> STRANDNESS : single
<220>
   <223> TOPOLOGY : linear
<220>
   <223> Description of Artificial Sequence : pldB-L-Bgl2F Primer
<400> 13
   gggagatcta gaggccggtt acctctacga gta 33
<210> 14
   <211> 30
   <212> DNA
   <213> Artificial Sequence
<220>
   <223> STRANDNESS : single
<220>
   <223> TOPOLOGY : linear
<220>
   <223> Description of Artificial Sequence : pldB-L-Hind3R Primer
<400> 14
   gggaagcttg cgatgagctg tgccagatag 30
<210> 15
   <211> 30
   <212> DNA
   <213> Artificial Sequence
<220>
   <223> STRANDNESS : single
<220>
   <223> TOPOLOGY : linear
<220>
   <223> Description of Artificial Sequence : pldB-R-Hind3F Primer
<400> 15
   gggaagcttg aactggcgcg acagtgtctt 30
<210> 16
   <211> 34
   <212> DNA
   <213> Artificial Sequence
<220>
   <223> STRANDNESS : single
<220>
   <223> TOPOLOGY : linear
<220>
   <223> Description of Artificial Sequence : pldB-R-Bgl2R Primer
<400> 16
   gggagatctg cagcggatcg tcttcgagac cctt 34
<210> 17
   <211> 29
   <212> DNA
   <213> Artificial Sequence
<220>
   <223> STRANDNESS : single
<220>
   <223> TOPOLOGY : linear
<220>
   <223> Description of Artificial Sequence : 5Dm-3F Primer
<400> 17
   ttcgcsctsc csgtcccstc satggtsat 29
<210> 18
   <211> 21
   <212> DNA
   <213> Artificial Sequence
<220>
   <223> STRANDNESS : single
<220>
   <223> TOPOLOGY : linear
<220>
   <223> Description of Artificial Sequence : 5Dm-3R Primer
<400> 18
   gttgatsays gasgtsgaga a 21
<210> 19
   <211> 30
   <212> DNA
   <213> Artificial Sequence
<220>
   <223> STRANDNESS : single
<220>
   <223> TOPOLOGY : linear
<220>
   <223> Description of Artificial Sequence : 6PIN-2F Primer
<400> 19
   gctgcgcctg gccctggagg acatcgagat 30
<210> 20
   <211> 30
   <212> DNA
   <213> Artificial Sequence
<220>
   <223> STRANDNESS : single
<220>
   <223> TOPOLOGY : linear
<220>
   <223> Description of Artificial Sequence : 6PIN-2R Primer
<400> 20
   ctgttcctcg aagaactcgt ggtcggcgta 30
<210> 21
   <211> 22
   <212> DNA
   <213> Artificial Sequence
<220>
   <223> STRANDNESS : single
<220>
   <223> TOPOLOGY : linear
<220>
   <223> Description of Artificial Sequence : 5D-1R Primer
<400> 21
   aggtgcccag cgagatcatg tt 22
<210> 22
   <211> 30
   <212> DNA
   <213> Artificial Sequence
<220>
   <223> STRANDNESS : single
<220>
   <223> TOPOLOGY : linear
<220>
   <223> Description of Artificial Sequence : 7PIN-2F Primer
<400> 22
   ccatgatcct gctggtggcc ggccatgaga 30
<210> 23
   <211> 21
   <212> DNA
   <213> Artificial Sequence
<220>
   <223> STRANDNESS : single
<220>
   <223> TOPOLOGY : linear
<220>
   <223> Description of Artificial Sequence : 5Dm-2R Primer
<400> 23
   ctggatsgtg tcsccsggyt t 21
<210> 24
   <211> 30
   <212> DNA
   <213> Artificial Sequence
<220>
   <223> STRANDNESS : single
<220>
   <223> TOPOLOGY : linear
<220>
   <223> Description of Artificial Sequence : 5PIN-2F Primer
<400> 24
   cggaatccac cagtgcctcg gccagaacct 30
<210> 25
   <211> 33
   <212> DNA
   <213> Artificial Sequence
<220>
   <223> STRANDNESS : single
<220>
   <223> TOPOLOGY : linear
<220>
   <223> Description of Artificial Sequence : pldApro-SpeNdeF Primer
<400> 25
   gggcatatga ctagtagccg tgtcctgccc gcc 33
<210> 26
   <211> 29
   <212> DNA
   <213> Artificial Sequence
<220>
   <223> STRANDNESS : single
<220>
   <223> TOPOLOGY : linear
<220>
   <223> Description of Artificial Sequence : pldApro-NdeR Primer
<400> 26
   gggcatatgt tcggacgtga attcattcg 29
<210> 27
   <211> 30
   <212> DNA
   <213> Artificial Sequence
<220>
   <223> STRANDNESS : single
<220>
   <223> TOPOLOGY : linear
<220>
   <223> Description of Artificial Sequence : eDM-SphF Primer
<400> 27
   tccccgcatg ccggaactga cggacatcac 30
<210> 28
   <211> 30
   <212> DNA
   <213> Artificial Sequence
<220>
   <223> STRANDNESS : single
<220>
   <223> TOPOLOGY : linear
<220>
   <223> Description of Artificial Sequence : eDM-SphR Primer
<400> 28
   cccgggcatg cagacgacga cgtagaggaa 30
<210> 29
   <211> 36
   <212> DNA
   <213> Artificial Sequence
<220>
   <223> STRANDNESS : single
<220>
   <223> TOPOLOGY : linear
<220>
   <223> Description of Artificial Sequence : PRR-1F Primer
<400> 29
   gccccccata tgaacgcaaa cgacaacgtg gtcatc 36
<210> 30
   <211> 35
   <212> DNA
   <213> Artificial Sequence
<220>
   <223> STRANDNESS : single
<220>
   <223> TOPOLOGY : linear
<220>
   <223> Description of Artificial Sequence : PRR-2R Primer
<400> 30
   gcggatcctc aggcactact cagttcagct ttggc 35
<210> 31
   <211> 27
   <212> DNA
   <213> Artificial Sequence
<220>
   <223> STRANDNESS : single
<220>
   <223> TOPOLOGY : linear
<220>
   <223> Description of Artificial Sequence : SP-1
<400> 31
   tatgcgtcac tagtcgggag tgcgtta 27
<210> 32
   <211> 27
   <212> DNA
   <213> Artificial Sequence
<220>
   <223> STRANDNESS : single
<220>
   <223> TOPOLOGY : linear
<220>
   <223> Description of Artificial Sequence : SP-2
<400> 32
   tataacgcac tcccgactag tgacgca 27
<210> 33
   <211> 30
   <212> DNA
   <213> Artificial Sequence
<220>
   <223> STRANDNESS : single
<220>
   <223> TOPOLOGY : linear
<220>
   <223> Description of Artificial Sequence : DM-NdeF
<400> 33
   gcccccatat gacggaactg acggacatca 30
<210> 34
   <211> 30
   <212> DNA
   <213> Artificial Sequence
<220>
   <223> STRANDNESS : single
<220>
   <223> TOPOLOGY : linear
<220>
   <223> Description of Artificial Sequence : DM-SpeR
<400> 34
   gggccactag tcagccggcc ggttcggtca 30
<210> 35
   <211> 30
   <212> DNA
   <213> Artificial Sequence
<220>
   <223> STRANDNESS : single
<220>
   <223> TOPOLOGY : linear
<220>
   <223> Description of Artificial Sequence : DM-BgIF
<400> 35
   cgcatagatc ttcacccgag cgggtgatca 30
<210> 36
   <211> 30
   <212> DNA
   <213> Artificial Sequence
<220>
   <223> STRANDNESS : single
<220>
   <223> TOPOLOGY : linear
<220>
   <223> Description of Artificial Sequence : DM-BgIR
<400> 36
   tcccgagatc ttgaaggtcc gcgtcaccgt 30
<210> 37
   <211> 27
   <212> DNA
   <213> Artificial sequence
<220>
   <223> R91C-F primer
<400> 37
   ttcgcggccg tctgcgaccg gcgggtg 27
<210> 38
   <211> 27
   <212> DNA
   <213> Artificial sequence
<220>
   <223> R91C-R primer
<400> 38 27
   cacccgccgg tcgcagacgg ccgcgaa 27
<210> 39
   <211> 27
   <212> DNA
   <213> Artificial sequence
<220>
   <223> R195L-F primer
<400> 39 27
   tcgcaagggg cgctcgagcg gctcgag 27
<210> 40
   <211> 27
   <212> DNA
   <213> Artificial sequence
<220>
   <223> R195L-R primer
<400> 40 27
   ctcgagccgc tcgagcgccc cttccga 27
<210> 41
   <211> 31
   <212> DNA
   <213> Artificial sequence
<220>
   <223> L403M-F primer
<400> 41
   acgatccagg ggatgatgga actccccgtg a 31
<210> 42
   <211> 30
   <212> DNA
   <213> Artificial sequence
<220>
   <223> L403M-R primer
<400> 42 30
   tcacggggag ttccatcatc ccctggatcg 30
<210> 43
   <211> 30
   <212> DNA
   <213> Artificial sequence
<220>
   <223> R236L-F primer
<400> 43
   agctggaccg actcgacgtg gtggcgctgg 30
<210> 44
   <211> 29
   <212> DNA
   <213> Artificial sequence
<220>
   <223> R236L-R primer
<400> 44
   agcgccacca cgtcgagtcg gtccagctc 29
<210> 45
   <211> 27
   <212> DNA
   <213> Artificial sequence
<220>
   <223> I244F-F primer
<400> 45
   tggcgctggc cgtcttcctg ctcgtgg 27
<210> 46
   <211> 30
   <212> DNA
   <213> Artificial sequence
<220>
   <223> I244F-R primer
<400> 46
   ccacgagcag gaagacggcc agcgccacca 30
<210> 47
   <211> 27
   <212> DNA
   <213> Artificial sequence
<220>
   <223> M112T-F primer
<400> 47
   cagcggcgga tgacgatccc gtcgttc 27
<210> 48
   <211> 27
   <212> DNA
   <213> Artificial sequence
<220>
   <223> M112T-R primer
<400> 48
   gaacgacggg atcgtcatcc gccgctg 27
<210> 49
   <211> 30
   <212> DNA
   <213> Artificial sequence
<220>
   <223> R195Y-F primer
<400> 49
   <400> aggggcgcgc gagtacctcg aggagtacct 30
<210> 50
   <211> 30
   <212> DNA
   <213> Artificial sequence
<220>
   <223> R195Y-R primer
<400> 50
   ggtactcctc gaggtactcg cgcgcccctt 30
<210> 51
   <211> 35
   <212> DNA
   <213> Artificial sequence
<220>
   <223> pldout-L-Bgl2F primer
<400> 51
   gggagatctg caggtcatcg gggggaagaa ccaca 35
<210> 52
   <211> 31
   <212> DNA
   <213> Artificial sequence
<220>
   <223> pldout-L-Sph1R primer
<400> 52
   tctccagccg catgcggtcc cgggtcaccg 31
<210> 53
   <211> 32
   <212> DNA
   <213> Artificial sequence
<220>
   <223> pldout-R-Sph1F primer
<400> 53
   cccgcatgcg gctggagatc atccagagcg ca 32
<210> 54
   <211> 34
   <212> DNA
   <213> Artificial sequence
<220>
   <223> pldout-R-Bgl2R primer
<400> 54
   gggagatcta gaacatgccg ggccagaggc tgac 34
<210> 55
   <211> 33
   <212> DNA
   <213> Artificial sequence
<220>
   <223> hyg-SpeSphF primer
<400> 55
   ggggcatgcg gactagtaca ccgtcgcctc ggt 33
<210> 56
   <211> 30
   <212> DNA
   <213> Artificial sequence
<220>
   <223> hyg-SphR primer
<400> 56
   gccgcatgcg tcaggcgccg ggggcggtgt 30